# EUROPEAN PATENT SPECIFICATION

(11) **EP 2 809 333 B1**
(45) Date of publication and mention of the grant of the patent: **16.08.2017**
(21) Application number: 13701801.6
(22) Date of filing: 31.01.2013
(51) Int. Cl.: A61K 38/00, A61L 27/00, A61K 35/12

(54) **DEVICE-BASED METHODS FOR LOCALISED DELIVERY OF CELL-FREE CARRIERS WITH STRESS-INDUCED CELLULAR FACTORS**
VERFAHREN UND FORMULIERUNGEN ZUR ÖRTLICH BEGRENZTEN ABGABE VON ZELLFREIEN TRÄGERN MIT STRESSINDUZIERTEN ZELLFAKTOREN
PROCÉDÉS ET FORMULATIONS POUR L'ADMINISTRATION LOCALISÉE DE SUPPORTS SANS CELLULES AVEC FACTEURS CELLULAIRES INDUITS PAR LE STRESS

(30) Priority: 03.02.2012 EP 12153769
(43) Date of publication of application: 10.12.2014
(73) Proprietor: Technische Universität München - Klinikum Rechts der Isar, 81675 München (DE)
(72) Inventor: SCHILLING, Arndt, 81377 München (DE); HADJIPANAYI, Ektoras, 80801 München (DE); MACHENS, Hans-Günther, 81675 München (DE)
(74) Representative: Vossius & Partner Patentanwälte Rechtsanwälte mbB
(86) International application number: PCT/EP2013/051910
(87) International publication number: WO 2013/113821

(56) References cited:
- WO-A2-2009/136173
- US-A1- 2010 210 530
- HADJIPANAYI E ET AL: "Controlling physiological angiogenesis by hypoxia-induced signaling", JOURNAL OF CONTROLLED RELEASE, ELSEVIER, AMSTERDAM, NL, vol. 146, no. 3, 15 September 2010 (2010-09-15), pages 309-317, XP027260361, ISSN: 0168-3659 [retrieved on 2010-06-09] cited in the application
- HADJIPANAYI E ET AL: "First implantable device for hypoxia-mediated angiogenic induction.", 10 August 2011 (2011-08-10), JOURNAL OF CONTROLLED RELEASE : OFFICIAL JOURNAL OF THE CONTROLLED RELEASE SOCIETY 10 AUG 2011 LNKD- PUBMED:21458514, VOL. 153, NR. 3, PAGE(S) 217 - 224, XP002679092, ISSN: 1873-4995 cited in the application figure 2
- CHEEMA U ET AL: "Spatially defined oxygen gradients and vascular endothelial growth factor expression in an engineered 3D cell model", CMLS CELLULAR AND MOLECULAR LIFE SCIENCES, BIRKHAUSER VERLAG, HEIDELBERG, DE, vol. 65, no. 1, 1 January 2008 (2008-01-01), pages 177-186, XP002552273, ISSN: 1420-682X, DOI: 10.1007/S00018-007-7356-8 cited in the application
- STEFANO DI SANTO ET AL: "Novel Cell-Free Strategy for Therapeutic Angiogenesis: In Vitro Generated Conditioned Medium Can Replace Progenitor Cell Transplantation", PLOS ONE, vol. 4, no. 5, 1 January 2009 (2009-01-01) , pages E5643-E5643, XP055025655, ISSN: 1932-6203, DOI: 10.1371/journal.pone.0005643 cited in the application
- E. HADJIPANAYI ET AL: "Injectable system for spatio-temporally controlled delivery of hypoxia-induced angiogenic signalling", JOURNAL OF CONTROLLED RELEASE, 22 May 2012 (2012-05-22), XP055031551, ISSN: 0168-3659, DOI: 10.1016/j.jconrel.2012.04.048

## Description

The present invention relates to an in vitro or ex vivo method of preparing a cell-free composition, said method comprising or consisting of (a) subjecting cells to stress; and (b) collecting factors produced by said cells when subjected to said stress, thereby obtaining said cell-free composition; wherein said cells are comprised in or form at least one first carrier and said collecting is effected by means of a second carrier which second carrier is cell-free and concomitantly present with and spatially distinct from said first carrier and is a scaffold composed of natural or synthetic polymer or comprises inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium; and said collecting is effected using a device comprising or consisting of (i) said first carrier(s) which first carrier(s) comprise(s) said cells or is/are suitable to hold said cells; (ii) said second carrier which is cell-free; and (iii) means of subjecting said cells in said first carrier to stress; wherein first carrier(s) and second carrier are positioned such that factors secreted by said cells when subjected to stress are collected in said second carrier, wherein at least one filter is positioned between said first and second carrier which prevents any cells and pathogens present in (any of) said first carrier(s) from entering into said second carrier(s).

*Role of hypoxic stress in angiogenesis.* Current strategies for therapeutic angiogenesis and vascularisation of implants/grafts, engineered constructs, wounds (e.g. diabetic ulcers, skin burns, incisions) and ischaemic tissues (e.g. myocardial, cerebral, peripheral tissues) largely rely on exogenous delivery of single or few angiogenic factors (e.g. recombinant factor proteins, gene transfer etc.). However, the limited success of such strategies in clinical trials has highlighted the concept that the spatio-temporal complexity of an angiogenic response is difficult to mimic, by isolating and delivering certain factors (e.g. VEGF) (Cao and Mooney 2007; Carmeliet 2000). A large area of research, for example, focuses on stabilisation of hypoxia-inducible factor 1 alpha (HIF-1a) (e.g. pharmacologically, by gene transfer etc.) in order to recapitulate hypoxia-induced signalling (Semenza 2007; Raghunath et al. 2009; Pajusola et al. 2005).

Utilisation of hypoxia as the primary angiogenic stimulus is a powerful tool since this approach overcomes the limitations of our incomplete understanding of the complexity of multi-factorial angiogenic cascades and harnesses the innate biological mechanism that naturally generates angiogenesis in the body, in physiological (e.g. during embryogenesis), as well as pathological states (e.g. cancer). Exposing cells to hypoxia to induce production of angiogenic factors has widely been investigated (Cheema et al. 2008; Namiki et al. 1995; Di et al. 2009). Conditioned media from hypoxic cell cultures have previously been described to induce angiogenesis (Di et al. 2009). Furthermore, pre-conditioning cells (e.g. bone marrow stem cells) to hypoxia has been shown to increase their survival and angiogenic potency upon transplantation (Kubo et al. 2009).

While chronically ischaemic tissues are constantly exposed to hypoxia, they have a limited capacity to appropriately respond to hypoxic stress. The proposed mechanism for the inadequate amount of compensatory angiogenesis seen in many chronic ischemic conditions involves a blunting of the ability of cells to induce angiogenic factors (e.g.VEGF) in response to prolonged/repeated hypoxic episodes (Levy 1999), but this effect might be further complicated by the fact that the spatial and temporal distribution patterns of endogenously produced angiogenic growth factors in ischaemic tissues are, to a great extent, influenced by inflammation (Luo et al. 2002).

As well as controlling the onset of an angiogenic response, it is also important to spatio-temporally regulate it (i.e. control where it is induced, for how long and in what direction). For example, control of the directionality of angiogenesis can be achieved by localised delivery of angiogenic signalling at an area of interest (e.g. infracted area, implant site etc), while allowing diffusion of these factors to generate a spatial factor gradient that chemo-attracts host endothelial cells towards the factor source (Barkefors et al. 2008). Cellular hypoxia-induced signaling could induce directional angiogenesis *in vitro* and *in vivo* (Hadjipanayi et al. 2010b). Importantly, the *in vivo* angiogenic response was both rapid (within 1 week), as well as functional, as shown by improvement in deep implant oxygenation compared to acellular constructs. Utilisation of implantable hypoxic cell depots, as factory units that constantly produce angiogenic factors *in vivo,* could prove a useful future strategy for improving vascularisation of engineered constructs and ischaemic tissues. However, the reliability on implanting living cells could be an immediate setback for its direct application in the clinic, due to safety and ethical concerns, as well as cell source limitations. In addition, since it is difficult to characterise the *in vivo* behaviour of such cells (e.g. levels/duration of angiogenic factor production, cell survival), process control in living cell implants still remains problematic. In an effort to overcome such limitations, a novel angiogenic therapy was recently developed that aims to deliver hypoxia-induced signaling without living cells (Hadjipanayi et al. 2011; WO/2009/136173). Here, dermal fibroblast-seeded collagen matrices were cultured under cell-mediated hypoxia for 10 days to upregulate production of angiogenic proteins (e.g. VEGF), before snap-freezing the matrices to kill all cells. Subcutaneous implantation of such pre-conditioned, non-viable depots *in vivo* (in rabbits) induced a directional angiogenic response within 1 week, through release of trapped angiogenic factor proteins. While this study provides an example of how hypoxia-induced signalling can be delivered *in vivo,* on-demand, without relying on ongoing production of factors by living cells, it still faces certain limitations; 1) since the strategy is based on implanting an angiogenic depot, it is highly invasive and not only frustrating for patients, but also accompanied by the common complications of surgery (e.g. bleeding, infection, thrombosis). 2) since it has been shown that stabilisation of a newly formed vascular network requires long-term release of angiogenic factors (note; exogenous VEGF has a short half-life (∼50 minutes) *in vivo*) (Cao and Mooney 2007), it is likely that any therapeutic approach will have to rely on multiple applications, which is difficult to be carried out with an implantable device. 3) It is known that physiological angiogenesis critically depends on tight temporal regulation of angiogenic factor release through differential gene expression at different time points (Ozduman et al. 2010; Ray et al. 2000; Komatsu and Hadjiargyrou 2004), which could not be recapitulated/replicated with an 'one time-off' implantable therapy. 4) frozen implants used in this therapy contained dead cells (in addition to the produced factors), which raises concerns about possible immunogenic reaction to allogeneic cells (which would be the likely cell source in an off-the-shelf therapy).

*Role of mechanical stress in muscle growth and regeneration.* Muscle is highly responsive to changes in functional demands. Overload leads to hypertrophy, whilst decreased load force generation and immobilization with the muscle in the shortened position leads to atrophy. As far as increase in mass is concerned, IGF-1 that is produced by active muscle appears to be a factor that controls local tissue repair, maintenance and remodelling. This growth factor has been called mechano-growth factor (MGF) to distinguish it from the liver IGFs which have a systemic mode of action. Although the liver is usually thought of as the source of circulating IGF-1, it has recently been shown that during exercise skeletal muscle not only produces much of the circulating IGF-1 but active musculature also utilizes most of the IGF-I produced. Furthermore, research has indicated the presence of an autocrine and endocrine IGF-1, both of which are upregulated in cardiac as well as skeletal muscle when subjected to overload. Following resistance exercise, MGF "kick starts" muscle hypertrophy and is important in local tissue repair (Goldspink 2005). IGF-I promotes myoblast proliferation, differentiation, and protein accretion in muscle through multiple signaling mechanisms (e.g. PI3-kinase, MAP kinase, calcineurin and Akt/mammalian target of rapamycin pathways (Tidball 2005; Favier et al. 2008). Exercise and injury induce increases in IGF-I, IGF-I receptors and IGF-I-activated signaling pathways. Expression of IGF-1 increases in the early stage of myoblast stretching, followed by a decrease in the late stage. Thus mechanical stimulation results in an immediate increase in IGF-1 expression, followed by a decrease as cells adapt to mechanical stress (Iwanuma et al. 2008).

Muscle aging is characterized by a decline in functional performance and restriction of adaptability, due to progressive loss of muscle tissue coupled with a decrease in strength and force output. Together with selective activation of apoptotic pathways, a hallmark of age-related muscle loss is the progressive incapacity of regeneration machinery to replace damaged muscle. These characteristics are shared by pathologies involving muscle wasting, such as muscular dystrophies or amyotrophic lateral sclerosis, cancer and AIDS, all characterized by alterations in metabolic and physiological parameters, progressive weakness in specific muscle groups. Muscle degeneration and persistent protein degradation through activation of proteolytic systems (e.g. calpain, ubiquitin-proteasome and caspase) occurs, while additional decrements in muscle growth factors compromise skeletal muscle growth, differentiation, survival and regeneration. Sarcopenia, i.e. the decline of muscle mass and strength with age, leads to significant impairment in the ability to carry out normal daily function and thus there is a great need for interventions that will lead to muscle regeneration and repair in the aging population. Age-related sarcopenia in humans, characterized by loss of type I and type II muscle fibers and a decrease in fiber cross-sectional area primarily in type II fibers, can be attenuated by mechanical load on the muscle, which increases cross-sectional area of the remaining fibers. Aging may be associated with attenuation of the ability of exercise to induce an isoform of IGF-I that promotes satellite cell proliferation. Considerable evidence also implicates age-related declines in muscle insulin-like growth factor action in sarcopenia; aging muscle may be resistant to IGF-I, an effect that is reversed by exercise. However, it is clear that overexpression of IGF-I in muscle can protect against age-related sarcopenia (Adamo and Farrar 2006).It has also been shown that, in contrast to normal muscle, MGF is not detectable in dystrophic muscles even when subjected to stretch and stretch combined with electrical stimulation. This is true for muscular dystrophies, where the necessary systemic as well as autocrine IGF-1 growth factors required for local repair are not produced and the ensuing cell death results in progressive loss of muscle mass (Goldspink 1999).

Skeletal muscle hypertrophy is a result of increased load, such as functional and stretch-overload (e.g. strength training) and involves activation of satellite cells and proliferation, differentiation and fusion of myoblasts. On the contrary, a dramatic loss of skeletal muscle mass determines atrophy settings. Muscle atrophy and wasting is a serious problem that occurs in patients with prolonged debilitating illness, burn injury, spinal injury, as well as with space flight. Current treatment for such atrophy, which often relies on nutritional supplementation and physical therapy, is of limited value in preventing the muscle wasting that occurs. To date, there is no accepted treatment to improve muscle size and strength. Several molecules, including Magic-F1, myostatin inhibitor, IGF, glucocorticoids and microRNAs are currently investigated to interfere positively in the blueprint of skeletal muscle growth and regeneration (Cassano et al. 2009). Considerable recent attention has focused on the use of anabolic growth factors such as insulin-like growth factor (IGF-1) in preventing muscle atrophy during limb disuse or with various catabolic conditions. However, potential side effects such as hypoglycemia appear to be limiting factors in the usefulness of IGF-1 for clinical treatment of muscle wasting conditions. Furthermore, due to the ability of IGF-I to stimulate cell division and its association with several forms of cancer, controversy exists concerning the advisability of treating cachexia or age-associated muscle wasting with IGF-I. A formulation of IGF-1 that is already bound to its endogenous-binding protein (BP3) and, as a result, has a greater specificity of action, had significantly less hypoglycemic effect (Zdanowicz and Teichberg 2003).

Importantly, it has been shown that the IGF-I gene is spliced in response to mechanical signals producing forms of IGF-I which have different actions. IGF-IEa, which initiates the fusion of myoblasts to form myotubes, is constitutively expressed in myoblasts and myotubes held under endogenous tension, and its expression can be upregulated by a single ramp stretch but reduced by repeated cyclical stretch. In contrast, mechano-growth factor (MGF), which is involved in the proliferation of mononucleated myoblasts, that are required for secondary myotube formation and to establish the muscle satellite (stem) cell pool, shows no significant constitutive expression in static cultures, but can be upregulated by a single ramp stretch and by cycling loading (Cheema et al. 2005).

Interleukin-15 (IL-15) is another skeletal muscle anabolic factor. IL-15 overexpression induces a hypertrophic myotube morphology. In contrast to IGF-I, the hypertrophic action of IL-15 on skeletal myogenic cells does not involve stimulation of skeletal myoblast proliferation or differentiation. IL-15 induces myotube hypertrophy at both low and high IGF-I concentrations, indicating that IL-15 action on skeletal myogenic cells is distinct from that of IGF-I (Quinn et al. 2002). The results of these studies suggest that stimulation of physiological muscle growth is likely to require complex, multiple growth factor mixtures rather than simple application of any one of these factors (or their different splice forms) in isolation.

*Role of mechanical stress in bone healing.* A major unmet need in the medical field today is the availability of suitable treatments for the ever-increasing incidence of osteoporosis and the treatment of bone deficit conditions. Osteoporosis is a major public health burden and is associated with increased fracture risk. Furthermore, fracture healing in osteoporosis is delayed, with reduced callus formation and impaired biomechanical properties of newly formed bone leading to high risk of fixation failure. Although therapies exist which prevent bone loss, the options are extremely limited for patients once a substantial loss of skeletal bone mass has occurred. The intricate process of bone formation is co-ordinated by the action of many different bone growth factors, some stored in bone matrix and others released into the bone micro-environment from surrounding cells. Although all these factors play important roles, the bone morphogenetic proteins (BMPs) clearly play a central role in bone formation and repair (Garrett 2007). BMPs form a unique group of proteins within the Transforming Growth Factor beta (TGF-beta) superfamily of genes and have pivotal roles in the regulation of bone induction, maintenance and repair. They act through an autocrine or paracrine mechanism by binding to cell surface receptors and initiating a sequence of downstream events that have effects on various cell types; BMPs and Wnt induce the differentiation of mesenchymal stem cells toward osteoblasts, while IGF-I enhances the function of mature osteoblasts.

Numerous unfavorable factors that disturb the balance between resorption and bone formation affect bone mechanical strength. Also mechanical loading (body mass and muscle tension) affects bone strength. Loading applied to the bone may condition its structure, and physical therapy affects bone mechanical strength by stimulating bone cells. This is possible thanks to the cellular adaptation mechanisms of bone modelling and remodelling. While mechanical loading is critical for the maintenance of bone mass, weightlessness, as with reduced physical activity in old age, bed rest, or space flight, invariably leads to bone loss.

Osteoblasts are key components of the bone multicellular unit and have a key role in bone remodeling, The coordinated function of skeletal cells is regulated by several hormones, growth factors and mechanical cues that act via interconnected signaling networks, resulting in the activation of specific transcription factors and, in turn, their target genes (Papachroni et al. 2009). Diminished mechanical forces eliminate signals that maintain osteocyte viability, thereby leading to apoptosis. Dying osteocytes in turn signal osteoclast recruitment to the vicinity and the resulting increase in bone resorption and bone loss (Aguirre et al. 2006). Mechanical stimulation (e.g. stretching) activates the ERKs, which in turn are responsible for the attenuation of osteocyte apoptosis (Plotkin et al. 2005). It has been suggested that altered responsiveness of osteoblasts in osteoporotic bone to mechanical stimuli may contribute to osteoporotic bone involution. Indeed, osteoporotic osteoblasts fail to increase proliferation and TGF beta release under 1% cyclic strain that stimulates both parameters in normal osteoblasts (Neidlinger-Wilke et al. 1995), indicating that these cells adapt to a micro-environement of reduced mechanical stress.

Mechanical cues affect production and secretion dynamics of growth factors (GFs) involved in osteogenesis. For example, compressive loading of bone marrow explants for 28 days sustained the production level of vascular endothelial growth factor, insulin-like growth factor-1 and transforming growth factor-β1 after day 21, while the levels of GFs produced positively correlated with ossification volume (Gurkan et al. 2011). It has also been shown that mechanical stimulation (20% compression, 1 Hz over 3 days) of bone fracture haematomas increases VEGF production (Groothuis et al. 2010), while exposure of osteoblasts to 10% compressive strain (11.81+/-0.42 kPa) results in a rapid induction of bone morphogenic protein-2 (BMP-2), runt-related transcription factor 2 (Runx2), and MAD homolog 5 (Smad5) (Rath et al. 2008). In addition to these factors, cytokines of the interleukin (IL)-11 subfamily participate in the regulation of bone cell proliferation and differentiation, possibly by enhancing BMP actions in bone. Indeed, mechanical unloading suppresses and reloading enhances Interleukin (IL)-11 gene expression in the hindlimb of mice in vivo (Kido et al. 2009), while overexpression of human IL-11 gene in transgenic mice stimulates bone formation and prevents cortical bone loss with advancing age (Takeuchi et al. 2002). While it has been accepted that mechanical signals must be large in order to be anabolic to bone tissue, recent evidence indicates that extremely low-magnitude (<10 microstrain) mechanical signals readily stimulate bone formation if induced at a high frequency (Rubin et al. 2001). For example, brief exposure of animals or humans to low magnitude, high frequency (LMHF) mechanical loading has been shown to normalize and prevent bone loss. Furthermore, LMHF mechanical loading (0.1-0.4 g at 30 Hz for 10-60 min/day) of pre-osteoblasts prevented disuse-induced decrease in mineralization, while mineralization induced by LMHF mechanical loading was blocked by the BMP antagonist noggin, suggesting a role for BMPs in this response (Patel et al. 2009).

Current approaches to anabolic therapies for osteoporosis include the use of neutralizing antibodies to Wnt antagonists, the enhancement of BMP signaling by proteasome inhibitors, adenoviral gene transfer of bone morphogenetic proteins (e.g. BMP-2 (Egermann et al. 2006), local (Li et al. 2010a) or systemic (Simic et al. 2006) delivery of recombinant BMPs, or the use of activin soluble receptors, IGF-I, or PTH analogs. Also, PDGF-BB, and to a lesser extent PDGF-AA, may have potential therapeutic use in the treatment of osteoporosis and bone healing (Graham et al. 2009). However, most current approaches are based on delivering single growth factors in isolation, even though it is also important that an anabolic growth factor needs to be targeted specifically to the skeleton to avoid unwanted non-skeletal effects (e.g. non-specific bone formation in areas not affected by injury or disease) and ensure safety and effectiveness (Canalis 2010).

*Role of mechanical stress in cartilage repair.* Articular cartilage (AC) provides a resilient and compliant articulating surface to the bones in joints. It protects the joint by distributing loads applied to it, so preventing potentially damaging stress concentrations on the bone, while at the same time it provides a low-friction-bearing surface to enable free movement of the joint. AC has no or very low ability of self-repair, and untreated lesions may lead to the development of osteoarthritis. Osteoarthritis (OA) is a debilitating, progressive disease of diarthrodial joints associated with the aging process. With the exception of anti-inflammatory corticosteroids and non-steroidal anti-inflammatory drugs which inhibit cyclo-oxygenase-2, no specific therapy based on fundamental intracellular pathways of chondrocytes and synoviocytes exists for the medical management of OA. At the molecular level, OA is characterized by an imbalance between chondrocyte anabolism and catabolism. Disruption of chondrocyte homeostasis primarily affects the cartilage extracellular matrix (ECM), which is responsible for the biomechanical properties of the tissue. Recent evidence has implicated cytokines, among which interleukin (IL)-1, tumor necrosis factor-alpha, IL-6, and IL-17 seem most involved in the OA process of cartilage destruction. The primary role of these cytokines is to modulate the expression of matrix metalloproteinases and cartilage ECM proteins. Cartilage repair that could restore the functional integrity of the joint is also impaired because chondrocytes in osteoarthritic cartilage appear unable to respond to insulin-like growth factor-1 or respond abnormally to transforming growth factor-beta (Malemud 2004).

Cartilage is exposed to mechanical forces during joint loading and chondrocytes cultured in vitro respond to mechanical stress by upregulating a range of growth factors. For example, application of dynamic compression and surface shear 1 h daily enhanced chondrogenesis in human bone marrow mesenchymal stem cells within fibrin-polyurethane composite scaffolds compared with the non-loaded control samples. Higher load frequency and higher compression amplitude induced transforming growth factor beta1 (TGFB1) and TGFB3 gene expression, while the chondrogenesis level of hMSCs was positively related to the TGFB1 and TGFB3 gene expression level (Li et al. 2010b). It has also been shown that cyclic compressive loading (10% strain at 1 Hz for 4 hours/day) can promote the chondrogenesis of rabbit bone-marrow mesenchymal stem cells by inducing the synthesis of TGF-beta1, which can stimulate the BM-MSCs to differentiate into chondrocytes (Huang et al. 2004).

The use of bioactive growth factors is under consideration as a potential therapy to enhance healing of chondral injuries and modify the arthritic disease process. By modulating the local microenvironment, e.g. recruitment of chondrogenic cells, stimulation of chondrogenic cell proliferation and enhancement of cartilage matrix synthesis, the anabolic and anticatabolic effects of a variety of growth factors have demonstrated potential in both in vitro and animal studies of cartilage injury and repair. Members of the transforming growth factor-β superfamily, fibroblast growth factor family, insulin-like growth factor-I, and platelet-derived growth factor have all been investigated as possible treatment in the management of chondral injuries and early arthritis (Fortier et al. 2011). It has been shown for example that controlled local delivery of TGF-β3 is essential to neocartilage formation by mesenchymal stem cells (Bian et al. 2011), while implantation of fibrin sealant incorporating FGF-2 successfully induced healing of the surface with hyaline cartilage and concomitant repair of the subchondral bone at eight weeks in femoral defects (Ishii et al. 2007).

Co-overexpression of IGF-I and FGF-2 by transplanted articular chondrocytes significantly enhances the early repair of cartilage defects in vivo and protects the neighboring cartilage from degeneration (Orth et al. 2011). Furthermore, different chondrogenic factors appear to exhibit differential behaviour regarding their effect in cartilage repair, e.g. recombinant BMP-2 shows better restoration of subchondral bone in contrast to the superior efficiency of recombinant BMP-4 for hyaline cartilage repair (Lopiz-Morales et al. 2010). Alginate has been successfully used for the controlled delivery of TGF-beta into osteochondral defects in vivo (Mierisch et al. 2002).

*Role of mechanical stress in tendon healing.* Mechanical stretch increases the secretion pattern of growth factors in tendon fibroblasts. Increased production of TGF-beta, bFGF and PDGF after cyclical mechanical stretching may have a positive influence on tendon and ligament healing through stimulation of cell proliferation, differentiation and matrix formation (Skutek et al. 2001; Yang et al. 2004). Factor delivery, as a means to improve tendon repair has already been investigated. For example, TGF-β3 delivered at a sustained rate enhanced tendon-to-bone healing in a rat model (Manning et al. 2011), while recombinant PDGF-BB-coated sutures enhanced histologic scores of sheep rotator cuff repairs (Uggen et al. 2010). As for other tissue types, the majority of studies have concentrated on delivering single growth factors. It has recently been shown that combined administration of TGF-b1 and IGF1 results in a significant increase in force at failure, ultimate stress and stiffness, in a patellar tendon defect model in rabbits (Lyras et al. 2010). The effects of growth factor combinations are mediated by quantifiable changes in cellular behaviour. In an in vitro study, for example, delivery of bFGF and PDGF-BB stimulated tendon fibroblast proliferation and promoted changes in the expression of matrix genes related to tendon gliding, strength, and remodelling (Thomopoulos et al. 2010).

*Role of electrical stimulation in nerve regeneration.* To date, a number of different growth factors (e.g. nerve growth factor, brain-derived neurotrophic factor, neurotrophin-3, ciliary neurotrophic factor, and fibroblast growth factor) have been shown to act as neurotrophic agents on distinct neuronal populations within the peripheral and central nervous system. Neurons upregulate neurotrophic factors after axotomy. The upregulation is normally slow, beginning after 7 days and occurring in association with a protracted period of axonal regeneration in which axons grow out from the proximal nerve stump across a suture site (e.g. over a period of 1 month in rodents). It has been shown that this staggered axon regeneration across the suture site is accelerated by a 1-hour period of low-frequency electrical stimulation that accelerates the expression of brain-derived neurotrophic factor (BDNF) in the neurons (Gordon 2009). Brief electrical stimulation (ES) has widely been shown to be effective in promoting neuronal regeneration following peripheral nerve injury. These effects are thought to be mediated largely by the upregulation of the expression of BDNF in spinal cord neurons, through activation of Ca²⁺- and Erk-dependent signaling pathways (Wenjin et al. 2011). ES (1 Hz) is also capable of enhancing nerve growth factor (NGF) release from cultured Schwann cells, a process that is also calcium dependent(Huang et al. 2010). In vivo studies have also confirmed the effectiveness of ES in nerve regeneration. Application of low intensity electrical stimulation (20 Hz) for 30-60 minutes immediately following crush injury is effective to promote sciatic nerve regeneration in a rat model, and this effect is associated with elevated expression of BDNF (Alrashdan et al. 2010; Wan et al. 2010). Transcorneal electrical stimulation (TES) promotes both axonal regeneration and survival of retinal ganglion cells after optic nerve crush, a response that is mediated by insulin-like growth factor 1 (IGF-1) upregulation (Tagami et al. 2009). These studies show the effectiveness of ES in improving nerve regeneration, but also indicate that this effect is mediated through multiple growth factor release. Furthermore, studies showing that exogenous BDNF and GDNF do not increase the number of neurons that regenerate their axons in freshly cut and repaired rat nerves, but do increase the numbers significantly after chronic axotomy (i.e. when injured motoneurons remain without peripheral targets for prolonged periods), indicating that while there is sufficient endogenous neurotrophic factor supply in axotomized motoneurons to support nerve regeneration, the reduced supply must be supplemented when target reinnervation is delayed (Gordon 2010). Importantly, it has also been shown that while stimulation of cut and repaired femoral nerves for 1 h leads to rapid mRNA upregulation of BDNF and its receptor, trkB, within the first 8 h, this stimulation effect peakes at 2 days and thereafter the levels of BDNF and trkB mRNA expression decline, suggesting that stimulation-mediated factor release is a regulated, adaptive process (Al-Majed et al. 2000).

In the central and peripheral nervous system, there are many instances where the delivery of neurotrophins has great potential in tissue repair (e.g. treatment of spinal cord injury). Degradable hydrogels have proved useful vehicles for the delivery of growth factors to promote the regeneration of diseased or damaged tissue. For example, Ciliary-neurotrophic factor (CNTF) released from a degradable hydrogel based on poly(ethylene glycol), above an explanted retina was able to stimulate outgrowth of a significantly higher number of neurites than controls without CNTF (Burdick et al. 2006). Other studies have shown that basic fibroblast growth factor (bFGF) does not work well when exogenously applied because of its short acting time. Topical application, however, of bFGF through a hydrogel facilitates faster healing from traumatic facial paralysis than one-shot application of bFGF, due to continuous release of bFGF (Komobuchi et al. 2010). Furthermore, NGF released from fibrin gel exhibited significantly higher degrees of PC12 cell viability and differentiation than NGF added in a free form daily into the culture medium, indicating that fibrin gel can release NGF in a sustained, controlled manner and in a bioactive form (Bhang et al. 2007). Hydrogel scaffolds (e.g. agarose gel) have also been shown to have great potential in serving as growth permissive 3D scaffolds, as well as having the ability to present neurotrophic factors to the injury site and enhance regeneration after spinal cord injury(Jain et al. 2006). The release kinetics of these factors can be controlled by changes in the gel network crosslinking density, which influences neurotrophin diffusion and subsequent release from the gels with total release times ranging from weeks to several months. In addition to cross-linking, other chemical modifications can be applied to control release kinetics. For example, fibrin gels containing a heparin-based delivery system that is used to immobilize neurotrophin-3 (NT-3) within the gel, thus slowing diffusion-based release of NT-3, showed increased neural fiber density in spinal cord lesions versus unmodified fibrin at 9 days in vivo(Taylor et al. 2004). Importantly, it has been shown that delivering combinations of factors, instead of single growth factors, can result in improved nerve repair/regeneration. For example, synaptotrophic effects are larger when both BDNF and NT-3 neurotrophins are delivered to the stump of severed axons of abducens motoneurons than when delivering each neurotrophic factor in isolation (Davis-Lopez de Carrizosa MA et al. 2009).

*Cross-over effects of stress stimuli in tissue repair*/*regeneration*. Angiogenesis is essential for the physiological adaptation of tissues to hypoxic stress, such as seen in the adaptation of ischaemic tissues, but also occurs in response to mechanical stress, for example due to elevated capillary shear stress and cell stretch, e.g. during adaptation of skeletal muscle to exercise. Increased production of VEGF is a characteristic of endothelial cells undergoing either stretch- or shear-stress-induced angiogenesis, while skeletal muscle microvascular endothelial cells exposed to 10% stretch in vitro show an elevation in HIF-1alpha and HIF-2alpha mRNA (Milkiewicz et al. 2007). Similarly, cyclic uniaxial stretching of tendon fibroblasts with a frequency of 1 Hz over 24h results in an increased expression of VEGF and HIF-1alpha (Petersen et al. 2004), and cyclical mechanical stretch activates the gene expression of HIF-1alpha in cultured vascular smooth muscle cells (Chang et al. 2003). It is also evident that both hypoxia and mechanical (e.g. stretch) factors serve as powerful stimuli for the up-regulation of growth factors (e.g. VEGF) which facilitate angiogenesis and arteriogenesis in the developing heart and in the adult heart undergoing adaptive responses (e.g. when the heart is challenged by enhanced loading conditions or during hypoxia or ischemia) (Tomanek et al. 2004). These findings support the idea of an inter-dependence of stress stimuli (e.g. hypoxic stress, mechanical stress), and that tissues adapt to stress by responding to multiple stress stimuli, through upregulation of multiple growth factors.

Complementary to the idea that the same growth factor can be upregulated in response to different stress stimuli (e.g. upregulation of VEGF by hypoxia and mechanical stress), it is now evident that a growth factor that is upregulated in one type of tissue in response to stress can mediate repair/regeneration in other tissue types. This adaptive mechanism can be seen in various processes, e.g. during the loss of normal weight-bearing activity (which occurs during bed rest, limb immobilization, and spaceflight) which stimulates a catabolic response within the entire musculoskeletal system, resulting in a loss of skeletal muscle mass, as well as bone mineral. Here, muscle adaptation to stress can also prevent loss of bone mass. For example, it has been shown that electroporation and ectopic expression of IGF-I within the gastrocnemius/soleus muscle significantly stimulates muscle fiber hypertrophy, attenuates the loss of muscle fiber area and muscle mass that normally occurs during disuse muscle atrophy, but also inhibits parameters of osteopenia within the tibia and fibula associated with hindlimb unloading (Alzghoul et al. 2004). Furthermore, other studies have shown that recombinant human BMP-2, a key factor in bone formation and repair, improves facial nerve regeneration by inducing more neurons to return to embryonic patterns of gene expression (Wu et al. 2010). Several recent animal studies have also demonstrated the potential of BMPs to enhance spinal fusion, repair critical-size defects, accelerate fracture union, and heal articular cartilage lesions (Issack and DiCesare 2003).

*Vehicles for localised factor delivery.* Typically, aqueous solutions of hydrogels used in biomedical applications are liquid at ambient temperature and gel at physiological temperature (37°C). Factors can also be locally delivered through conventional gels, i.e. the delivery vehicle is in this case is in a semi-solid state before injection. For example, alginate gel has been investigated as vehicle for recombinant VEGF delivery (Silva and Mooney 2007).

In view of the prior art as described above, the technical problem underlying the present invention can be seen in the provision of alternative or improved means and methods for tissue repair. The technical problem is solved by the subject-matter of the enclosed claims.

The present invention in a first aspect provides an in vitro or ex vivo method of preparing a cell-free composition, said method comprising or consisting of (a) subjecting cells to stress; and (b) collecting factors produced by said cells when subjected to said stress, thereby obtaining said cell-free composition; wherein said cells are comprised in or form at least one first carrier and said collecting is effected by means of a second carrier which second carrier is cell-free and concomitantly present with and spatially distinct from said first carrier and is a scaffold composed of natural or synthetic polymer or comprises inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium; and said collecting is effected using a device comprising or consisting of (i) said first carrier(s) which first carrier(s) comprise(s) said cells or is/are suitable to hold said cells; (ii) said second carrier which is cell-free; and (iii) means of subjecting said cells in said first carrier to stress; wherein first carrier(s) and second carrier are positioned such that factors secreted by said cells when subjected to stress are collected in said second carrier, wherein at least one filter is positioned between said first and second carrier which prevents any cells and pathogens present in (any of) said first carrier(s) from entering into said second carrier(s).

The term "stress" as used herein has the meaning as established in the art in relation to living cells. Preferably, it relates to any condition that deviates from the conditions a given cell is subjected to when present in its natural environment within a healthy human or animal body. Typically, stress may be triggered by subjecting cells to a stimulus. The term "stress" may also be defined in terms of its result as regards the cells, which result is the raising of a cellular response, more specifically a change in the expression pattern of peptidic and/or proteinaceous factors. This change in expression pattern which occurs in response to a stimulus may change over time; see the enclosed examples. The change of expression pattern over time which is observed in vitro generally parallels that observed in vivo. Preferred forms of stress are disclosed further below.

As a consequence of being subjected to stress, cells provide a response. Said response includes the production, preferably the secretion of factors. In addition to secreted factors, the term "produced factors" includes factors which are produced inside the cell and leave the cell by means of passive diffusion as opposed to active secretion. It is furthermore contemplated that certain conditions of stress, such as electric stimulation using certain parameter settings such as 1 to 20 Hz for 30 to 60 min or mechanical stimulation may cause cells to be become leaky, which leakiness may entail that factors produced by the cells are set free. The term "factor" as used herein, in its broadest sense, refers to any chemical species produced by a cell when subjected to stress. Preferred factors are biomolecules, preferably selected from peptides, proteins, nucleic acids, lipids and carbohydrates. Particularly preferred are peptides and proteins, wherein it is understood that the term "protein" includes polypeptides. Peptides are polycondensates of amino acids containing up to 30 amino acids, whereas polypeptides typically contain 31 or more amino acids. Proteins, in addition to polypeptides, may further comprise other molecules, such as prosthetic groups. Moreover, one protein may comprise more than one polypeptide chain, as is the case with oligomeric such as dimeric, trimeric or tetrameric proteins. Preferred factors are described in more detail below. The identity of certain factors produced by cells when subjected to stress is also apparent from the examples enclosed herewith.

A requirement that the identity of one or more factors would be known, however, does not arise for the purposes of the present invention. Instead, and without wishing to be bound by a specific theory, it is considered that the method of the present invention permits to capture complex factor mixtures which may be difficult or even impossible to prepare by other means.

The method according to the first aspect of the present invention provides for the composition to be obtained to be cell-free, despite the fact that what is subjected to stress according to the invention are necessarily cells. This is achieved by the means specified in the first aspect, namely by confining the cells to a first carrier while using a second carrier for the purpose of collecting said factors. Said second carrier is cell-free at the beginning and remains cell-free owing to its spatial distinctness from the first carrier. Additional means in accordance with the invention of ensuring that the second carrier remains cell-free are described in more detail further below. It is furthermore important that said first and second carriers are concomitantly present at least during step (b) and preferably during both steps (a) and (b). Such setup provides for a one-step procedure of preparing said cell-free composition, preferably without any need for further steps such as separation, filtration, centrifugation or purification. Such cumbersome steps are avoided by the present invention as are any losses, contaminations or biases which may be introduced by such steps.

Said cells to be subjected to stress are either comprised in the first carrier, or they form the first carrier. It is understood that in the latter case, the first carrier actually consists of cells, the cells being held together by endogenously produced molecules. As a consequence, in these cases the first carrier is entirely free of any exogenously added substances. There is no requirement, though, that the first carrier be free of any exogenously added or prepared material.

Step (a) and/or step (b) may be repeated two or more times. In other words, said stress may be continuously applied for a defined amount of time, preferably from day(s) to week(s), such as 1, 2, 3, 4, 5, 6 or 7 days or 1, 2, 3, 4, 5, 6, 7, 8, 9 or 10 weeks, or the stress may be applied repeatedly, each repetition characterized by a given time period which may always be the same or different, said periods where stress is applied being separated by periods of time where no stress is applied. This intermittent pattern (regularly or irregularly spaced intervals) of stress application is preferred in that it may be used to prevent development of cellular adaptation/habituation to the stress applied in vitro or ex vivo, similar to that observed in vivo under chronic stress exposure, and therefore prevent/diminish the adaptive downregulation/reduction of cellular factor expression/production. The repetition of step (b) is of particular interest, and this will be discussed in more detail below, because in response to stress, cells do not continuously produce the same factors in the same relative amounts, but instead the response to stress may be divided into various phases, such as early, medium and late phase, the various phases to be distinguished from each other in terms of the factors being produced and/or secreted and/or their relative amounts.

Given that said cells are subjected to stress, and that the method provides for collecting a plurality of factors, the obtained cell-free composition is inherently suitable for tissue regeneration. To explain further, tissue regeneration and repair of tissue damage as it naturally occurs in the human or animal body at the site of, for example, a wound in a number of instances does not proceed normally. Instead, for example, the wound never heals completely. This happens despite the fact that the cells present at the site of a wound experience stress (e.g. hypoxia) which under ideal circumstances should trigger the secretion of factors sufficient for wound healing. A conceivable reason for such deficiency is that the cellular production of factors for wound healing, including growth factors, or the upregulation of these factors, undergoes an adaptive downregulation over time, the consequence being that tissues exposed to stress, in particular tissues chronically exposed to stress have a reduced limited or no ability to generate or to generate in sufficient amounts those factors that mediate repair or regeneration. Providing damaged tissue with one or more cell-free compositions according to the present invention is a suitable means to replace the missing factors at the site of tissue damage such that healing eventually proceeds further and is successfully completed. Effectively, and without wishing to be bound by a specific theory, administration of a cell-free composition according to the present invention provides for reversing the above-mentioned adaptive response of cells or tissues to constant, repeating or chronic stress and thereby restarts or fosters the healing process.

One advantage of the present invention is that complex factor mixtures may be obtained and used without any requirement of knowing the composition. Given that the obtained compositions are cell-free and in a preferred embodiment may be obtained from autologous cells, possible immunoreactivity is not an issue. Also from that perspective, a requirement to characterize the mixture of factors as comprised in the cell-free compositions according to the invention does not arise.

One means of ensuring that the composition obtained by the method is indeed cell-free is the use of a first and a second carrier as specified in the first aspect. While the first carrier contains the cells subjected to stress, the second carrier is a means of collecting those factors which, for example, by means of diffusion, leave the first carrier. It is understood that the materials of the first carrier and/or of the second carrier are chosen such that cells do not leave the first carrier and/or are not capable of entering to the second carrier. Further means of preventing entry of cells into the second carrier are disclosed below. An option for the first carrier is a matrix wherein the cells are embedded and prevented from escaping.

A further advantage of the method according to the first aspect is that it can be repeated, either as a whole or independently step (a), step (b) or step (a) and (b). This allows for the collection of several factor mixtures, wherein each collected factor mixture is representative of the state of said cells at the given point in time or period of time, the given point in time or beginning of said period of time being separated by defined time span from the beginning of the exposal to stress.

By using more than one second carrier it is possible to separate the total factor load generated by the cells in said first carrier into a plurality of smaller doses. For this specific purpose, also more than one first carrier is to be used. A plurality of first carriers together with a plurality of second carriers may be implemented in one device according to the invention. One operation of the device will deliver a plurality of (equally factor-loaded) cell-free compositions which are suitable for the concomitant treatment of a plurality of treatment locations such as wounds or burns.

Also, it is envisaged to use a plurality of second carriers in order to produce different cell-free compositions, in this case preferably in conjunction with one first carrier. The obtained cell-free compositions may differ with regard to factor composition and/or factor concentration. This may be achieved, for example, by exposing each second carrier for different periods, for example, four, eight or twelve days. To give a specific example, in a medical emergency situation, cell-free compositions in accordance with the present invention might need to be delivered acutely. In such a case, in a first stage of treatment a second carrier may be used which has been concomitantly present with said first carrier for a short period of time. Said treatment may be followed with (a) second (or further) second carrier(s) which has/have been conditioned, i.e. kept concomitantly present with the first carrier for a longer period of time. Second carriers may also be replaced, i.e. in a first step a particular second carrier may be concomitantly present with said first carrier for a given period of time, followed by removal of the second carrier. Subsequently another second carrier may be inserted into the device, followed by a period of time wherein the new second carrier is concomitantly present with said first carrier.

In a preferred embodiment of the first aspect, the subjecting to stress is effected by exposing said cells to (i) hypoxia, preferably by establishing 0 to 10% (v/v) O₂ and/or the addition of a chemical inducer of hypoxia; (ii) mechanical stress, preferably compressive, tensile, shear and/or hydrostatic stress; and/or (iii) electric stimulation; and/or wherein said cells are autologous, allogeneic or xenogeneic (iv) fibroblasts, preferably dermal or cardiac fibroblasts; bone-marrow derived stromal cells; skeletal, smooth or cardiac muscle cells; epithelial cells such as endothelial cells; adult stem cells, preferably obtained from bone marrow or adipose tissue; blood cells, preferably leukocytes; or adipose tissue-derived stromal cells; and wherein said subjecting to stress is preferably effected by exposing said cells to hypoxia; (v) myoblasts; osteocytes; osteoblasts; osteoclasts; chondrocytes; skeletal, smooth or cardiac muscle cells; dermal, tendon or cardiac fibroblasts; endothelial cells; or adult stem cells, preferably obtained from bone marrow or adipose tissue; and wherein said subjecting to stress is preferably effected by exposing said cells to mechanical stress; and/or (vi) nerve cells; glial cells; skeletal, smooth or cardiac muscle cells; fibroblasts; osteocytes; osteoblasts; osteoclasts; chondrocytes; or adult stem cells including neural stem cells; and wherein said subjecting to stress is preferably effected by exposing said cells to electrical stimulation.

The term "hypoxia" has a meaning as established in the art and refers to conditions where the partial pressure of oxygen is lower as compared to the partial pressure prevailing in normal air (about 21% (v/v) O₂; (v/v) indicating volume percentage). Lower partial pressures - or equivalently concentrations - may be established in closed compartments, where oxygen is gradually being consumed, and/or by any active measures, such as the use of incubators with defined oxygen concentrations or the addition of a chemical inducer of hypoxia, CoCl₂ being a preferred chemical inducer of hypoxia. In particular if no active measures are to be used, the size of the closed compartment and the amount of sample, in particular the number of cells comprised in the sample may be chosen such that, owing to the oxygen consumption by the cells, a defined partial oxygen pressure is eventually established.

As regards mechanical stress, basically all types of mechanical stress are conceivable for the purposes of the present invention. Preferred and exemplary forms of mechanical stress are recited above and further illustrated in the figures enclosed herewith. Means for exerting mechanical stress are described in the examples and furthermore at the skilled person's disposal.

Electric stimulation can be effected with various parameter settings, preferably at a frequency of 1 to 20Hz and for a period of time of 30 to 60mins. Preferably, the electric field strength is between 1 mV/mm and 1 kV/mm.

Generally speaking, the cells which may be subjected to stress are not particularly limited. Preferred cells are listed in the preferred embodiment recited above. In principle, any cell may be subjected to any type of stress. Preferably, however, it is considered to be particularly useful if specific types of cells are subjected to, for example, hypoxia, whereas other types of cells are preferably subjected to mechanical stress or electric stimulation. Preferred pairing of cell types with types of stress are also indicated above.

In a further preferred embodiment of the first aspect of the invention, all produced factors, preferably all peptidic and/or proteinaceous factors, are collected; and/or said factors are peptidic and/or proteinaceous factors, preferably (i) angiogenic factors; (ii) bone-specific, muscle-specific, tendon-specific and/or cartilage-specific growth factors; and/or (iii) neural growth factors.

In other words, it is preferred that a comprehensive collection of factors produced or secreted by the cells is effected. Suitable means for such comprehensive collection, in particular in terms of the composition of such means, are at the skilled person's disposal. In principle, any material with sufficient surface for adsorption is suitable. When preparing such second carrier, preferably consideration is given to the average pore width within the carrier, such that both peptidic as well as proteinaceous factors are collected. Preferred are furthermore carriers having a large surface, more specifically a large interior surface, such carriers being also referred to as "matrices" herein.

The collected factors may be further classified in terms of their activity; see items (i) to (iii) of this particular preferred embodiment.

In this regard, it is understood that the three preferred types of stress, namely hypoxia, mechanical stress and electric stimulation, the three groups of cells according to items (iv), (v) and (vi), and the three groups of factors to be produced are preferably associated with each other. More specifically, if, for example, hypoxia is applied to blood cells, the produced or secreted factors will be enriched in or be exclusively angiogenic factors. Such association, while being subject of a preferred embodiment, is, however, not to be construed as being limiting.

In a further preferred embodiment, in case step (b) is repeated two or more times, two or more cell-free compositions are obtained.

In a particular preferred embodiment, step (a) is performed once and step (b) is performed two or more times at defined points in time, said defined points in time preferably being measured (i) in days to weeks and/or (ii) from the beginning of said subjecting cells to stress.

The above preferred embodiments provide for drawing multiple samples, i.e. the obtaining of several cell-free compositions containing factors during one performance of the in vitro or ex vivo method according to the first aspect. Since nature and amount of factors vary as a function of time, time being counted from the beginning of the exposure to said stress, a plurality of cell-free compositions drawn at different points in time will usually differ as regards the comprised factors. This is particularly advantageous given that, for example, in non-healing wounds, the cells of the human or animal body may be deficient in different aspects, which different types of deficiency are preferably treated with different cell-free compositions, the compositions differing with regard to their time of collecting. Moreover, preference is given to matching compositions. The term "matching" when used herein to characterize cell-free compositions has one or more of the following meanings: (i) the cell type(s) in the first carrier correspond to the cell type(s) of the target tissue to be treated; and/or (ii) the age - or equivalently the passage number - of cells in the first carrier correspond to the age of the cells in a patient, subject or animal to whom the cell-free composition is to be administered.

In a second aspect, the present invention provides a device comprising or consisting of (a) one or more first carrier(s) which first carrier(s) comprise(s) cells or is/are suitable to hold cells; (b) a second carrier which is cell-free and is a scaffold composed of natural or synthetic polymer or comprises inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium; and (c) means of subjecting said cells in said first carrier to stress; wherein first carrier(s) and second carrier are positioned such that factors secreted by said cells when subjected to stress are collected in said second carrier; wherein at least one filter is positioned between said first and second carrier which prevent any cells and pathogens present in (any of) said first carrier(s) from entering into said second carrier.

Such device may be employed when performing the method according to the first aspect.

As specified above, the recited first carrier may either comprise the cells to be subjected to stress, or may be suitable to hold cells, but be still cell-free, in particular prior to performing the method of the invention. In the latter case, the device as a whole may be cell-free, but is capable of taking up and holding cells within the first carrier comprised in said device.

Said cells as they may be comprised in a first carrier may be either of one type or may be mixtures of cells of different types, preferably mixtures of cells derived from one tissue type. In case more than one first carrier is employed, each of said first carriers may contain different cell types or different sets of cell types.

As already discussed above, the second carrier is cell-free, when performing the methods according to the first aspect, and remains cell-free. The second carrier is a means of collecting the factors and is accordingly positioned such with respect to the first carrier that said factors may leave the first carrier and enter into the second carrier.

Further details regarding the first carrier can be found further below in the section entitled "system cellular component". Further details regarding the second carrier can also be found further below, namely in the section entitled "system acellular component". It goes without saying, and it is apparent from the detailed description of said first and second carrier, that these terms do not extend to a liquid phase such as a culture supernatant.

Means of subjecting said cells in said first carrier to stress are described in more detail below; see the section entitled "types of cellular stress".

In accordance with the invention, means are positioned between said first and second carrier which prevent any cells and/or pathogens present in (any of) said first carrier(s) from entering into said second carrier, said means being at least one filter, preferably a nano-porous filter, preferably with a pore width of 1 to 1000 nm, preferably 0.22 µm, wherein in case of more than one filter an array of filters, preferably with decreasing pore size may be used, the filter with largest pore size facing said first carrier(s), wherein said filter or at least one of said filters may comprise at least one agent binding at least one of the factors produced by said cells when subjected to stress, such agent preferably being selected from a receptor specific for such factor, e.g. a growth factor receptor, an antibody specific for such factor, an enzyme and an inhibitor of an enzyme.

These means of further ensure that cells or pathogens as they may be present within the first carrier do not enter the second carrier. Such means include unspecific filters which remove cells and/or pathogens simply by their size, or may be specific binding agents. Specific binding and/or inhibitory agents may be employed in case the composition of factors as it is delivered from the first carrier upon applying said stress is to be further modified. For example, the removal and/or inhibition of certain factors, such as inflammatory cytokines, matrix metalloproteinases and/or cellular metabolic products, may be desirable. This could be achieved for example with cytokine neutralizing antibodies (e.g. anti-TNFa monoclonal antibody), cytokine receptors (e.g. TNF-binding receptor; Etanercept) or enzyme inhibitors, in particular protease inhibitors (e.g. Tissue Inhibitors of Metalloproteinases; TIMP1, TIMP2, TIMP3, TIMP4). On the other hand, specific binding agents might also be used in order to prevent cells and/or pathogens from entering into the second carrier. For this latter embodiment, e.g. anti-integrin antibodies may be used.

In a further preferred embodiment of the device according to the second aspect, (a) one or both carriers comprise one or more of (i) proteins, preferred proteins being collagen, fibrin, elastin and fibronectin; (ii) polysaccharides, preferred polysaccharides being chitin, alginate and cellulose; (iii) glycosaminoglycans, preferred glycosaminoglycans being chondroitin sulfate, dermatan sulfate, keratan sulfate, heparin sulfate, heparan sulfate and hyaluronic acid; (iv) synthetic polymers, preferred synthetic polymers being polyglycolic acid, polylactic co-glycolic acid, polylactic acid and polycaprolactone, polystyrene and polyethylene terephtalate; (v) de-cellularised native tissue, preferred native tissues being dermis, small intestinal submucosa and bladder submucosa; and (vi) inorganic material, preferably calcium phosphate ceramics, alumina ceramics, zirconia ceramics, nitride ceramics, carbon nanotubes and metals selected from or alloys comprising titanium, tantalum, iron, magnesium, cobalt and chromium; (b) one or both carriers are selected from gel; sponge; mesh; microparticles; nanoparticles; microfibers; nanofibers; preferably electrospun microfibers or nanofibers; and nanotubes; (c) said first carrier or at least one of said first carrier(s) furthermore comprises an electric conductor; (d) said first carrier or (at least one of) said first carrier(s) is/are selected from cell sheet(s) and split-thickness or full-thickness skin graft(s), said graft(s) and cell sheet(s) preferably being autologous; and/or (e) said second carrier is selected from cream, emollient or ointment.

Calcium phosphate ceramics include hydroxyapatite.

This preferred embodiment provides various options as regards (a) the constituents of first and/or second carriers, (b) the structure or texture of the carriers, and (c) further constituents of the first carrier for special applications, said special applications including electric stimulation. Finally, and as already mentioned above, the first carrier may exclusively consist of cells, in that case typically embedded within endogenously produced matrix. Such first carriers may also be referred to a cell sheet. Cell sheets can be prepared, for example, by using the guidance provided in L'Heureux et al. 1998 or Kushida et al. 2000. Split-thickness or full-thickness skin grafts, preferably autologous, can also be used as the first carrier and represent another example where the first carrier consists of cells, the cells being held together by endogenously produced molecules. As a consequence, in these cases the first carrier is entirely free of any exogenously added substances.

Further preferred synthetic polymers include Poly(2-hydroxy ethyl methacrylate), Poly(N-vinyl pyrrolidone), Poly(methyl methacrylate), Poly(vinyl alcohol), Poly(acrylic acid), Polyacrylamide, Poly(ethylene-co-vinyl acetate), Poly(ethylene glycol), Poly(methacrylic acid), Polyanhydrides, and Polyorthoesters.

In a preferred embodiment of the second aspect, the present invention provides a device comprising (a) a first chamber comprising (i) at least one inlet; (ii) a first carrier which is capable of adsorbing, absorbing or trapping blood cells; and (iii) at least one filter which prevent any cells or pathogens from entering into a second chamber while permitting entry of peptidic or proteinaceous factors into said second chamber; (b) said second chamber comprising a second carrier which second carrier is cell-free, said second carrier being selected from (i) a nano- or microporous matrix capable of specifically binding, adsorbing, absorbing or trapping said peptidic or proteinaceous factors; and (ii) a nano- or microfiber mesh or nano- or microparticle network which mesh or network can be rendered injectable by mechanical agitation; and (c) in case (b)(ii), said second chamber has at least one port for a syringe, a needle and/or a third chamber connected to said second chamber.

Related thereto, described is a device comprising (a) a first chamber comprising (i) at least one inlet which inlet(s) preferably is/are (a) port(s) for a blood-drawing syringe; (ii) a first carrier which is capable of adsorbing, absorbing and/or trapping blood cells; (iii) means which prevent any cells and/or pathogens from entering into a second chamber while permitting entry of peptidic and/or proteinaceous factors into said second chamber, wherein said means are preferably positioned such that they are not in contact with said first carrier; (iv) preferably an anti-coagulant and/or an inducer of hypoxia; and (v) optionally a port preferably distinct from the inlet according to (i), said port being configured for the addition of medium to said first chamber; (b) said second chamber, said second chamber comprising a second carrier which second carrier is cell-free, said second carrier preferably being selected from (i) a nano- or microporous matrix capable of specifically binding, adsorbing, absorbing and/or trapping said peptidic and/or proteinaceous factors; and (ii) a nano- or microfiber mesh or nano- or microparticle network which mesh or network can be rendered injectable by mechanical agitation; wherein said second chamber preferably is connected to said first chamber in a detachable manner and/or can be opened; (c) in case (b)(ii), said second chamber has at least one port for a syringe and/or a third chamber connected to said second chamber, preferably in a detachable manner; and (d) said first and/or second chamber(s) has/have at least one inlet which is sealable, preferably in an air-tight manner or a manner which controls oxygen exchange wherein optionally (e) said first and second chamber (s) is/are under vacuum; (f) said first and/or second chamber(s) is/are divided in two or more sub-compartments, each sub-compartment of first chamber having at least one inlet which inlet(s) preferably is/are (a) port(s) for a blood-drawing syringe; (g) said second chamber is positioned above or around said first chamber; (h) said inducer of hypoxia is a chemical inducer of hypoxia; (i) said first carrier is loaded with blood cells; and/or (j) said third chamber comprises a gel or a sol-gel, which gel or sol-gel preferably comprises collagen, fibrin, and/or alginate.

Said port in accordance with item (a)(v) provides for the addition of medium, such as nutrient medium, preferably serum-free medium when operating said device.

Said first carrier may also be collageneous.

A preferred anti-coagulant is heparin or EDTA.

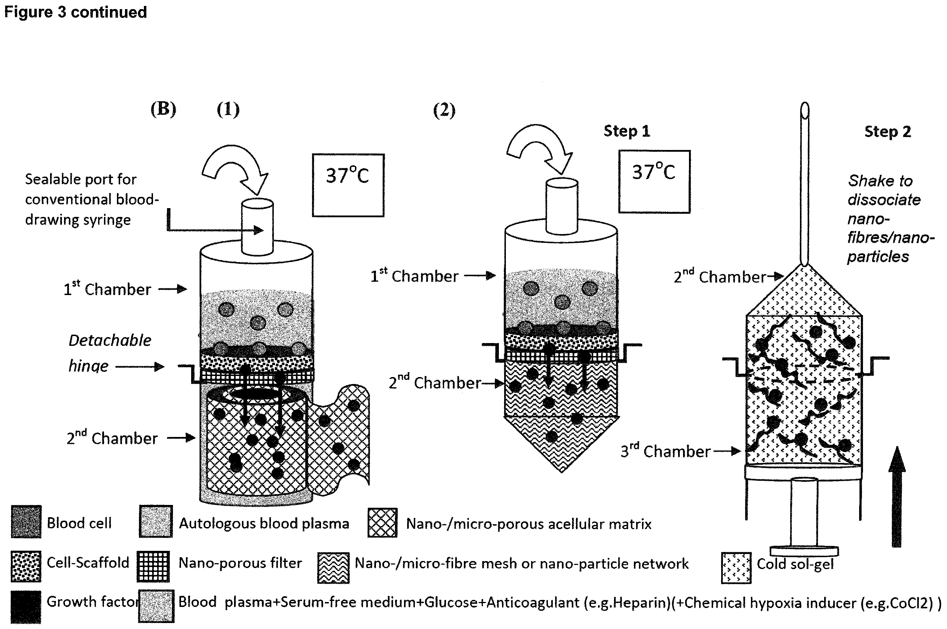

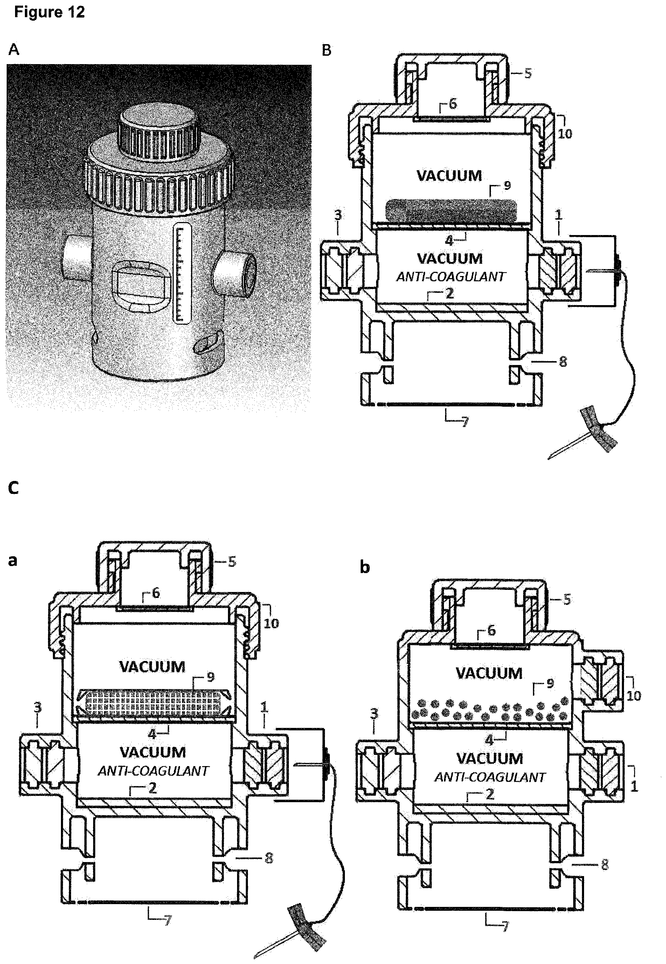

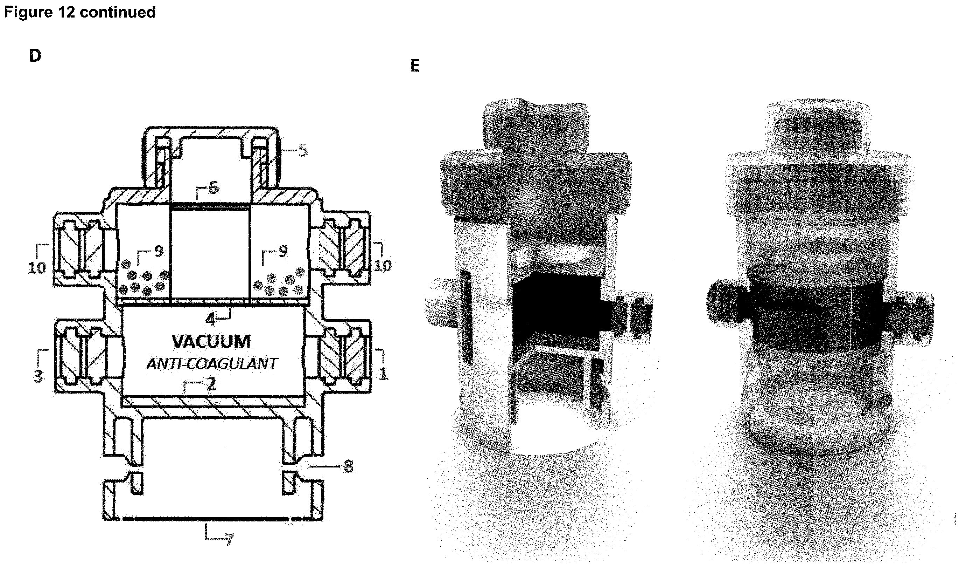

An exemplary implementation of the device is depicted in Figure 3B, wherein parts (1) and (2) of Figure 3B correspond to options (i) and (ii) of part (b) of the third aspect. For a further detailed description of exemplary implementations of the device according to the third aspect, said further description not being limiting, can be found in Example 3 as enclosed herewith. Figure 12 illustrates a further preferred device of the invention.

The first chamber is designed such that blood taken from a patient can conveniently be introduced to that first chamber.

Within said first chamber hypoxic conditions may be established, preferably either by the mere fact that the first chamber is sealable, the blood cells will consume oxygen, and hypoxic conditions will establish in situ, or by the addition of an inducer of hypoxia, a conceivable inducer of hypoxia being CoCl₂. In a further alternative, the device according to the invention may be manufactured such that said first chamber is under vacuum. In such a case, blood taken from a patient may be aspired into said first chamber.

Said port may be implemented as shown in Figure 12, i.e. as port that is designed to be compatible with standard blood-drawing equipment such as the BD Vacutainer system. Said port may be equipped with a double-rubber membrane. The port may be designed such that it extends from the wall of the first chamber which design preferably allows for mounting of the protective barrel that sheaths the piercing needle of the blood-drawing equipment; see Figure 12 for an exemplary implementation.

The optional anti-coagulant according to (a)(iv) may also be omitted. In that case, a blood sample drawn from a subject, upon transferring it into said first chamber, is allowed to clot, which clotting is a (further) means of securing that the cells remain in the first chamber and do not enter the second carrier. For esthetic reasons and/or for convenience of the patient, the first chamber or the device as a whole can be manufactured from non-transparent material.

Said first chamber may be equipped with a second port, the second port preferably being implemented as a double-rubber membrane, said second port allowing for adding a serum-free medium; see reference No. (3) in Figure 12.

The second chamber comprises a second carrier, which second carrier takes up factors produced by the cells comprised in the first carrier when subjected to stress, more specifically, to hypoxia. Depending on the particular design of the second carrier - and this is further illustrated in an exemplary manner in Figure 3B - the second carrier, once loaded with factors, may be directly used as, for example, a wound dressing; or when designed such that mechanical agitation causes dissociation of the second carrier loaded with factors, so that it is converted into an injectable cell-free composition according to the present invention. Dissociation refers to a loss of or a reduction of interactions, in particular noncovalent interactions between the molecules of the second carrier.

Said first chamber and said second chamber are preferably adjacent to each other, for example such as shown in Figure 12. Means which prevent any cells and/or pathogens from entering into the second chamber in accordance with item (a)(iii) are positioned such that they separate the first and second chamber. A preferred means is a nano-porous filter.

The second chamber is preferably equipped with a vented cap, said vented cap preferably communicating with a nano-porous hydrophobic membrane for gas exchange. In terms of operation, said vented cap is opened when the first chamber is filled with blood and/or replenished with serum-free medium.

Described is that (a) said inducer of hypoxia is a chemical inducer of hypoxia; (b) said first carrier is loaded with blood cells; and/or (c) said third chamber comprises a gel or a sol-gel, which gel or sol-gel preferably comprises collagen, fibrin, and/or alginate.

Said third chamber comprises a gel or a sol-gel, preferably in cooled form, which gel or sol-gel is to be mixed with the second carrier, thereby obtaining a further type of cell-free composition according to the present invention. Gels and sol-gels are a preferred subject-matter in relation to various aspects as will be apparent further below. A gel is a composition comprising liquid, preferably water, which composition is solid or semi-solid and furthermore stays in place and does not significantly change its shape once deployed. A sol-gel according to the present invention is a composition of matter which may change between a liquid sol state and the gel state as defined above. Such change from a sol to gel state preferably occurs upon administration to the subject.

The device may be designed such that multiple devices can be stacked on top of each other, for example for the purpose of incubation; see Figure 12.

In a further preferred embodiment of the method according to the first aspect, said collecting is effected by using the device of the invention. In other words, any of the above disclosed devices is preferably used when performing the in vitro or ex vivo method according to the invention.

Described is an in vitro or ex vivo method of preparing a cell-free composition, said method comprising or consisting of (a) subjecting cells to stress; and (b) collecting factors produced, preferably secreted by said cells when subjected to said stress, thereby obtaining said cell-free composition; wherein said cells are comprised in or form at least one first carrier and said collecting is effected by means of a second carrier which second carrier is cell-free and concomitantly present with and spatially distinct from said first carrier; and said collecting is effected using a device comprising or consisting of (i) said first carrier(s) which first carrier(s) comprise(s) said cells or is/are suitable to hold said cells; (ii) said second carrier which is cell-free; and (iii) means of subjecting said cells in said first carrier to stress; wherein first carrier(s) and second carrier are positioned such that factors secreted by said cells when subjected to stress are collected in said second carrier, wherein means are positioned between said first and second carrier which prevent any cells and/or pathogens present in (any of) said first carrier(s) from entering into said second carrier.

In a fourth aspect, the present invention provides one or more cell-free compositions obtained by the method of the invention for use in a method of treating or preventing tissue damage, wherein said composition comprises or consists of at least one second carrier as defined in the first aspect.

Described is also (a) cell-free composition(s) obtained by the method according to the first aspect, wherein said composition comprises or consists of at least one second carrier and preferably is selected from (a) dressings, patches, meshes, nerve conduits, threads and sutures and biomedical implants; or (b) gels, sol-gels, sprays, powders, meshes, threads, sutures, nerve conduits, biomedical implants; injectable compositions and/or compositions suitable for delivery by endoscopic means, which injectable compositions or compositions deliverable by endoscopic means preferably gel upon administration; wherein gels and sol-gels preferably comprise collagen, fibrin and/or alginate; wherein preferably said composition is storable for at least two weeks to six months at temperatures between -80°C and -20°C, and preferably comprises a stabilizer such as albumin.

The above aspect of the present invention provides cell-free compositions obtainable or obtained by the method according to the first aspect of the present invention. The above disclosed methods provide for compositions comprising the collected factors, wherein said composition may be a second carrier, the second carrier being loaded with said factors. On the other hand, said second carrier - or any other composition comprising factors and obtained by the methods according to the invention - may be further processed in order to obtain cell-free compositions according to the present invention. To the extent the second carrier as such is a cell-free composition according to the present invention, the cell-free composition, in preferred embodiments, may take various shapes which are specified in part (a) of this aspect of the invention.

Alternatively, the second carrier - or any other cell-free composition obtained by the method according to the present invention - may be further processed such that any of the compositions according to item (b) of the fourth aspect is obtained. Such further processing may comprise the method of manufacturing a cell-free composition according to the present invention as defined further below and/or the addition of further constituents which further constituents ensure that a composition according to item (b) of the fourth aspect is formed. Particular preference is given to gels and sol-gels. In this regard, it is understood that in the course of further processing the second carrier, constituents capable of forming gels or sol-gels are added.

Generally speaking, cell-free compositions according to the present invention are solid, semi-solid, or to the extent they are liquid, they exhibit high viscosity. High viscosity in this context refers to such viscosities which, upon administration of said cell-free composition, ensure that said cell-free composition remains at the site of administration. Viscosity is preferably higher than that of water under the same conditions. Given that endoscopes generally have a wider bore than needles for injection, preference will be given to compositions deliverable by endoscopic means if particularly highly viscous compositions are to be used.

Owing to the state of matter, the cell-free compositions according to the present invention allow for topical administration. Preferably, and in many instances related to said topical administration, the compositions according to the present invention provide for spatially or spatio-temporally controlled administration.

Given that said compositions are cell-free and as a result of the method of obtaining them, and furthermore, where applicable, owing to the cell constituents added (see discussion of item (b)), the cell-free compositions are storable for extended periods of time at temperatures between -80°C and -20°C. In order to further stabilize the compositions for storage over extended periods of time, stabilizers may be added. A preferred stabilizer is albumin. Alternatively or in addition, any stabilizer known in the art may be used. It turns out that cell-free compositions according to the present invention are stable in the mentioned temperature range for at least two weeks, four, weeks, two months, three months, four months, five months or six months.

Further details regarding compositions according to the present invention as well as their preferred constituents can be found in the section entitled "delivery vehicles" further below.

Described is a method of manufacturing a cell-free composition as defined in item (b) of the fourth aspect, said method comprising the step of fragmenting, preferably mechanically fragmenting, at least one factor-loaded second carrier, the second carrier(s) being obtained by the method according to the first aspect, and optionally a step of adding said second carrier(s) to a gel or a sol-gel.

This relates to a method of manufacturing those cell-free compositions according to the invention where the second carrier is subjected to one or more further processing steps in order to obtain a cell-free composition. In this regard, it is envisaged to alter the physical properties of the second carrier, more specifically from a solid, semi-solid or highly viscous liquid state to a liquid state of lower viscosity, and/or by changing the size of its constituent units (such as fibers and particles) to one of smaller size (preferably in the µm to nm range), preferably such that an injectable composition or a composition deliverable by endoscopic means is obtained. Accordingly, the method according to the fifth aspect comprises a step of fragmenting, wherein it is understood that the term "fragmenting" refers to constituents of the second carrier and not the factors as comprised in the second carrier after the methods according to the present invention have been effected. Particular preference is given to mechanical fragmenting. This provides for a particularly good protection of the factors as present in the second carrier in that preferentially or exclusively the second carrier is fragmented, but not said factors. In an alternative or additional approach, enzymes may be used for the purpose of fragmenting the second carrier. In this regard, it is understood that those enzymes and those constituents of second carriers are preferred which permit an enzymatic degradation specifically of the second carrier without the factors being affected.

As indicated above, the latter method preferably comprises a further step or consists of the above recited step and said further step, the further step being the addition of constituents providing gel or sol-gel properties or adding said second carrier(s) to a gel or sol-gel. To the extent the method of manufacturing a cell-free composition further comprises such a second step, it is particularly preferred that said method consists of (i) the step of fragmenting as defined above and (ii) said second step.

The cell-free compositions according to the present invention, owing to the presence of stress-induced factors, are capable of mimicking the appropriate physiological tissue response to stress. This is particularly useful in the treatment or prevention of tissue damage, noting that tissue damage is typically characterized in that the affected cells are adapted to stress and, instead of a normal healing process, the damaged state of the tissue persists. In such conditions, compositions according to the present invention provide for normal tissue regeneration, e.g. wound healing, to resume and proceed to completion.

In this regard, it is preferred that the compositions(s) is/are topically administered. Topical administration can be achieved by any of the means disclosed herein above, namely by using solid, semi-solid or viscous liquid compositions which all provide for the respective compositions to stay in place upon administration. Furthermore, it is preferred that autologous material is used, i.e. the cells which are subjected to stress according to the method of the first aspect of the invention are obtained from the subject or patient to whom subsequently said compositions are to be administered.

In a preferred embodiment of the one or more cell-free composition(s) for use, said tissue damage is related to conditions selected from wounds, including wounds caused by radiation, steroid wounds and venous stasis wounds; ulcers, in particular decubitus ulcers and diabetic ulcers; incisions; lacerations; abrasions; burns; grafts, in particular skin grafts, local flaps and free flaps; and ischemic tissues such as post-wounding or post-grafting ischemic tissues; and/or disorders selected from vascular occlusive disease; peripheral artery disease; atherosclerosis; myointimal hyperplasia; thromboangiitis obliterans; thrombotic disorders; mesenteric ischemia; limb ischemia; peripheral artery stenosis; vasculitis; infarctions including cerebral and myocardial infarctions; diabetes; traumatic injuries; bone fractures; osteoporosis; arthritis including osteoarthritis; rheumatism; cartilage rupture or detachment; torn or ruptured tendons; spinal injuries; muscular dystrophies; amyothrophic lateral sclerosis; cancer; AIDS; and central or peripheral nerve injury or degeneration.

Further information on diseases or tissues to be preferably treated can be found further below in the section entitled "target tissue sites".

Described herein is also a method of treating or preventing tissue damage, said method comprising or consisting of (a) optionally the method according to the first aspect, wherein said cells are preferably obtained from the subject or patient defined in step (b) below; and (b) the step(s) of topically administering one or more cell-free composition(s) according to the invention, one or more composition(s) obtained by the method of the first aspect, and/or the composition(s) obtained in step (a), to a subject at risk of developing tissue damage or a patient suffering from tissue damage.

In a preferred embodiment of the composition(s) for use or the method of treating, said compositions are (to be) topically administered at defined points in time which defined points in time preferably match the defined points in time as defined above, and wherein said compositions are (to be) topically administered preferably in the order of said collecting. Said "defined points in time as defined above" are those disclosed in conjunction with a preferred embodiment of the method according to the first aspect, said defined points in time preferably being measured (i) in days to weeks and/or (ii) from the beginning of the subjecting of cells to stress in accordance with the first aspect of the present invention.

### System cellular component ("first carrier")

In the description of the first carrier which follows below, said first carrier is also referred to as a system cellular component. While preference is given to first carriers comprising cells, it is at the same time understood, and apparent from the general description provided further above, that the first carrier in certain aspects does not comprise cells. In such cases, the first carrier may be loaded with cells prior to performing methods according to the first aspect.

In either case, and as apparent from the capability to secrete said factors, the cells comprised in said first carrier are or comprise living cells.

Different cell types can be exposed to specific stress stimuli to upregulate cell type-specific and/or stimulus-specific growth factor mixtures, which may vary in terms of factor composition/concentration. Preferred cells that can be pre-conditioned to hypoxic stress to produce angiogenic factors are mammalian cells, including stromal cells (e.g. fibroblasts, bone marrow-derived stromal cells, smooth/skeletal/cardiac muscle cells), epithelial cells (e.g. endothelial cells), peripheral blood cells and stem cells (e.g. corneal stem cells; skin epidermal stem cells; gut stem cells; orogenital stem cells; adipose-derived stem cells; epithelial stem cells; bone marrow stromal stem cells; and growth plate stem cells). Preferred stem cells are those which, when practicing the present invention, do not require the use or handling of human embryos. Particularly preferred are accordingly established cell lines which are at the skilled person's disposal and non-human stem cells. A further preferred type of stem cells are adult stem cells as they may be obtained or purified from adult tissue including bone marrow and adipose tissue. Preferred cells that can be pre-conditioned to mechanical stress for upregulating bone-, muscle and cartilage-specific growth factors are mammalian cells, including osteoblasts, myoblasts, smooth/skeletal/cardiac muscle cells, chondrocytes, stem cells (e.g. bone marrow stromal stem cells). Preferred cells that can be electrically simulated for upregulation of nerve-specific growth factors include nerve cells, glial cells (e.g. astrocytes, oligodendrocytes, Schwann cells), neural stem cells, skeletal muscle cells, cardiomyocytes. Suitable cells include waste human cells (e.g. human foreskin fibroblasts), allogeneic GMP produced cells (e.g. allogeneic human neonatal fibroblasts), allogeneic or autologous blood cells (which can be selectively obtained from a patient before an operative procedure), or allogeneic or autologous stromal stem/progenitor cells from bone marrow, adipose tissue or other sources, all of which are available for clinical use at GMP grade. Cell type (e.g. as source of angiogenic factors) may be selected based on the target tissue (e.g. cardiac fibroblasts when treating myocardial infarcts; dermal fibroblasts for skin wounds, skin ulcers and vascularisation of skin grafts; skeletal muscle cells for vascularisation of ischaemic skeletal muscle etc.). Xenogeneic cells could also be used, as these provide a larger cell source.

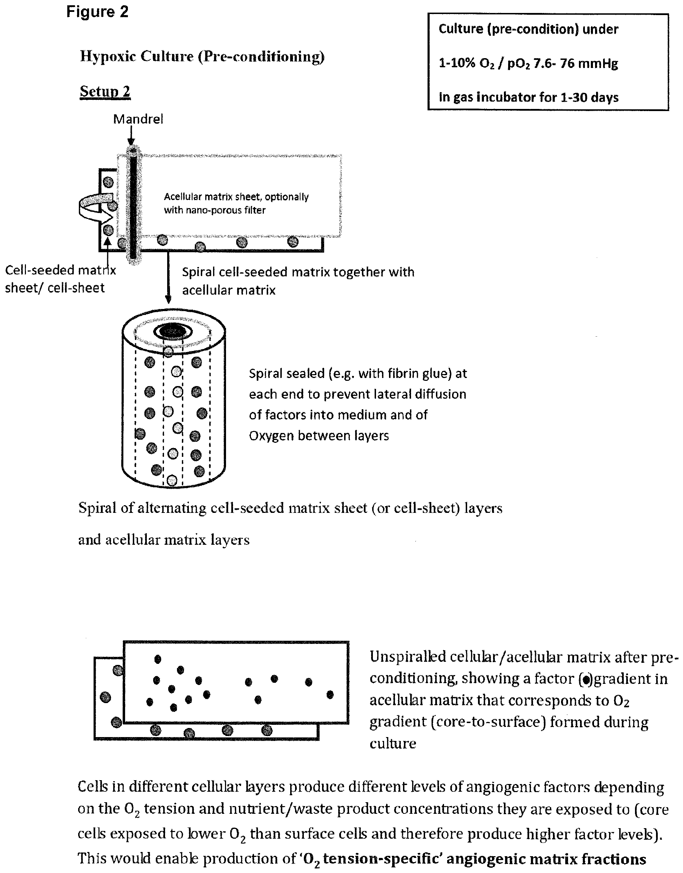

Cell density within the cellular compartment (e.g. cell-seeded matrix) can be anything greater than about 1×10⁴ cells/ml, preferably in the range of 1×10⁵ to 10×10⁶ cells/ml. Cell density will directly influence the amount of factors produced at a given time point (i.e. the higher the cell density the greater the amount, as long as cell viability is not significantly affected). Cell density will also determine O₂ consumption, and therefore the O₂ gradient (rate, level) established (i.e. the greater the number of cells, the faster an O₂ gradient is achieved and the steeper it is) (Fig. 2), which will also affect hypoxia-induced growth factor expression by cells (i.e. the steeper the O₂ gradient from the construct surface to the core, the greater the proportion of cells exposed to lower O₂ tension and the greater the level of factor expression). The cellular metabolic activity (which depends on cell type, as well as cell number) will also determine the minimum oxygen tension reached (i.e. cells with high metabolic activity, such as smooth muscle cells, will generate lower oxygen tension than cells with low metabolic activity, such as dermal fibroblasts, at the same density (Cheema et al. 2009)), which will subsequently influence cell viability. Cell culture (pre-conditioning) can be carried out in serum-supplemented or serum-free medium.

Preferably, a minimum partial oxygen pressure is used for culturing/pre-conditioning which is non-pathological i.e. insufficient to significantly reduce cell viability or induce cell death. A non-pathological minimum partial oxygen pressure may be greater than 7.6 mmHg (greater than 1.1 kPa or greater than 1% oxygen).

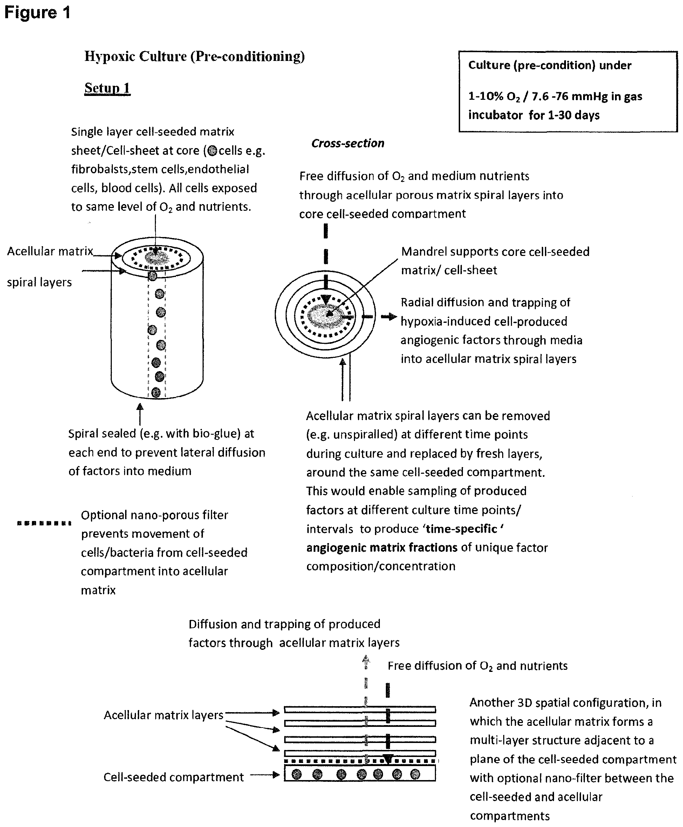

For fabrication of the cell-seeded compartment, cells can be seeded within a biomaterial matrix scaffold (e.g. in the form of sheet/layer) composed of natural biopolymer (e.g. collagen, fibrin, elastin, fibronectin), polysaccharides (e.g. chitin, or cellulose), glycosaminoglycans (GAGs) (e.g. chondroitin sulfate, dermatan sulfate, keratan sulfate, heparin/ heparan sulfate, hyaluronic acid) or synthetic polymer (e.g. polyglycolic acid (Deng et al. 2009), polycaprolactone, polylactone, polyglycone, polylactic co-glycolic acid etc.). Examples of natural biopolymer matrix scaffolds include collagen, fibrin (or other biopolymeric) hydrogels. These hydrogels can be optionally subjected to plastic compression, in which excess interstitial fluid is irreversibly removed from the hydrogel through fixed mechanical loading over a porous support, to produce a sheet of controlled cell/matrix density (depending on the level of compression/fluid removal) (Brown et al. 2005; Hadjipanayi et al. 2009b; Hadjipanayi et al. 2010a). Other scaffolds that can be used in engineering the cellular compartment include biopolymeric (e,g, collagen) sponges, decellularised extracellular matrices from various tissues (e.g. dermis, small intestinal submucosa (SIS), urinary bladder, amniotic membrane) (Badylak et al. 2009), electrospun nano-fibre (e.g. collagen, elastin, fibrinogen, glycosaminoglycans) scaffolds (Smith et al. 2008), synthetic polymers (e.g. PLGA, PEG). The scaffold material may also be a composite/hybrid material comprising two (or more) different types of biomaterials (e.g. collagen/elastin, collagen/fibrin, collagen/fibronectin, collagen/glycosaminoglycan). While it is preferred that the cellular compartment comprises a single-cell layer (cell-seeded matrix layer or cell-sheet layer), thus preventing formation of steep oxygen- and nutrient-consumption gradients and ensuring that all cells are exposed to the same partial oxygen pressure,(Fig.1), it can be also optionally produced as a multi-layer structure, with each matrix layer having a thickness of one or more cell diameters (i.e. as multiple cell layers within the same matrix layer and /or multiple single-cell matrix layers). It has previously been shown that cell-seeded multi-layer biopolymeric (e.g. collagen) matrices undergo interface integration in vitro within days (Hadjipanayi et al. 2009a), a response mediated partly by cell migration and collagen deposition at the interface. This is preferred, especially in the case where the cell-seeded compartment is exposed to mechanical stress, as it will ensure that stresses are uniformly applied to cells.

The biomaterial matrix within cells are seeded ideally has sufficiently strong mechanical properties which would allow it to be attached (e.g. tethered, sutured) to a loading bioreactor, for application of mechanical stimuli. For example, a biomaterial matrix with an elastic modulus of more than about 20 kPa, preferably 1-2 MPa, may be chosen. For application of electrical stimulation, cells can be seeded within a electroactive / electro-conductive polymeric scaffold (e.g polylactide and aniline pentamer copolymer (Huang et al. 2008)) or electrically active nano-material scaffolds such as composite materials with nanoscale, piezoelectric zinc oxide particles embedded into a polymer matrix (zinc oxide, when mechanically deformed through ultrasound, for example, can provide an electrical stimulus (Seil and Webster 2010)).

In addition to utilisation of cell-seeded matrix sheets for development of the cellular compartment, cell-sheets can also be used, without any exogenous biomaterial scaffold (L'Heureux et al. 1998). Cell-sheets can be obtained for example by non-enzymatic harvest of cells grown to confluency on a thermo-responsive culture plate (Kushida et al. 2000). These are sparse in cell-produced matrix, but have sufficient mechanical strength for handling (e.g. spiralling/folding). In some setups, a combination of biomaterial cell-seeded matrix and cell-sheets can also be used.

Autologous split-thickness or full-thickness skin grafts, obtained from a patient at a prior time point, can also be used as the first carrier and represent another example where the first carrier consists of cells (e.g. including or consisting of fibroblasts and/or keratinocytes), the cells being held together by endogenously produced extracellular matrix. As a consequence, in these cases the first carrier is entirely free of any exogenously added substances.

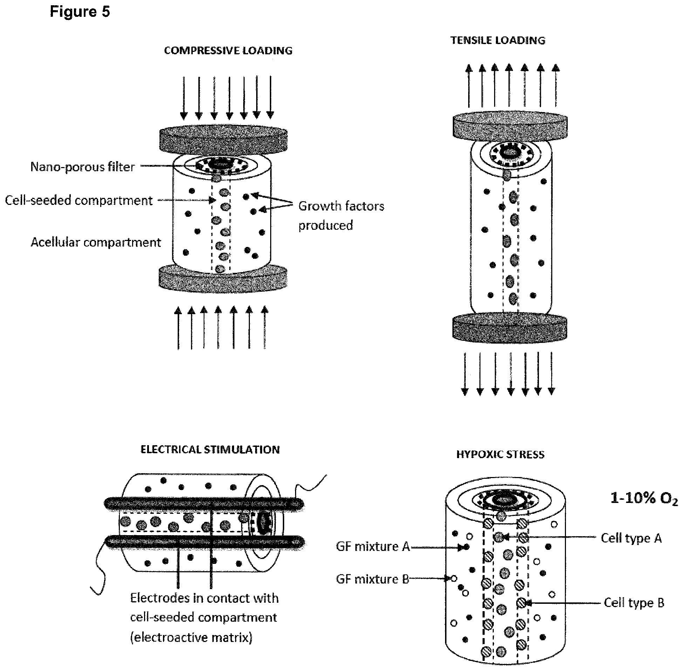

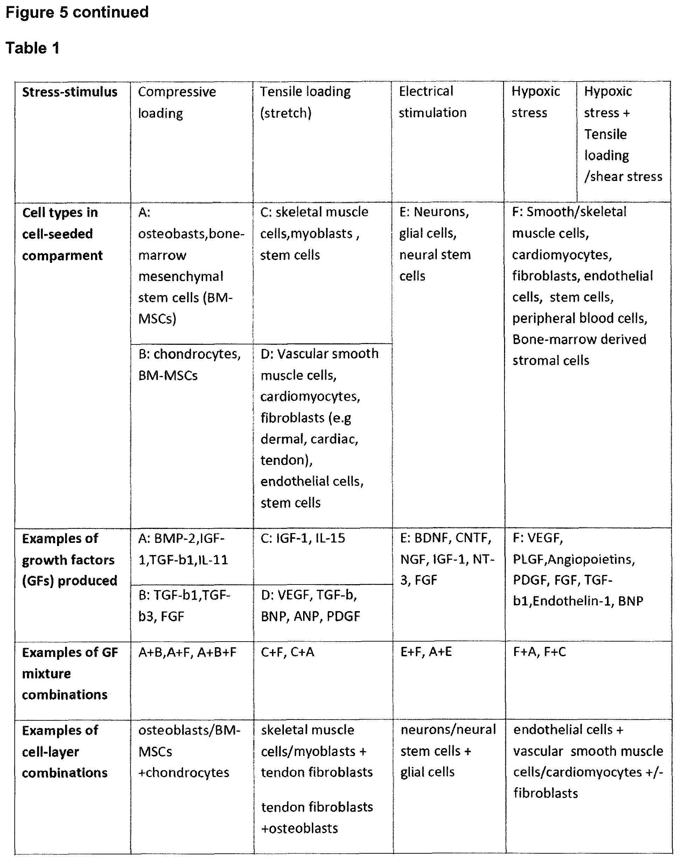

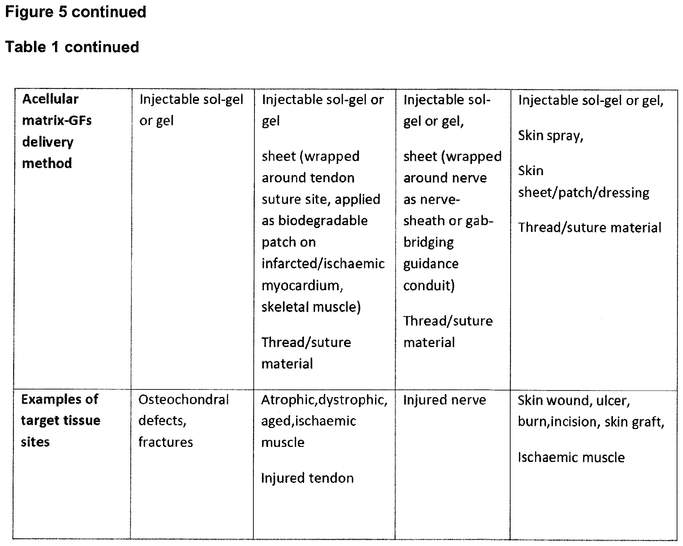

The cell-seeded compartment could comprise of one or more cell-seeded matrix and/or cell-sheet layers. For example, it could comprise of an inner endothelial cell layer, a middle vascular smooth muscle cell layer, and an outer fibroblast layer, where the cells can be simultaneously exposed to tensile loading and hypoxic stress, thus micmicking the natural stresses within a vascular wall under inschaemic conditions (Fig. 5). Other possible combinations of cell-type layers in the cell-seeded compartment include osteoblasts/chondrocytes (mimicking an osteo-chondral structure), neurons/glial cells (mimicking a nerve), myoblasts/tendon fibroblasts (mimicking a muscle-tendon interface), osteoblasts/tendon fibroblasts (mimicking a bone-tendon interface), cardiomyocytes/cardiac fibroblasts (mimicking cardiac tissue) (Fig. 5; Table 1).

### System acellular component ("second carrier")

Owing to the design of the means and methods of the invention, it is evident that said second carrier, also referred to as system acellular component, remains cell-free during the entire performance of any method of the invention.

The above mentioned types of matrices (natural or synthetic polymers) i.e. in general all those materials which are described in relation to the first carrier, can also be used (e.g. in sheet/layer form) to construct the acellular matrix compartment that is positioned adjacent to (e.g. wrapped or spiralled around) the cell-seeded compartment in order to capture the growth factors produced by the cells. A suitable matrix could be one with a nano-/micro-scale interconnected porous structure that allows free diffusion of oxygen and nutrients (e.g. glucose) into the cellular compartment (this will ensure maintenance of high cell viability for prolonged periods, i.e. weeks) and outward diffusion of waste metabolites (e.g. CO₂, lactate, malate, ammonia), but (partly) traps macro-molecules (e.g. angiogenic factor proteins) produced by the cells. A suitable matrix may have an oxygen diffusion coefficient of 7×10⁻⁶ to 4×10⁻⁵ cm²/s, more preferably in the range of 2 - 4×10⁻⁵ cm²/s, which is in the range of oxygen diffusion coefficient of small intestinal submucosa (SIS) and human dermis (Androjna et al. 2008). The interconnected porous structure also provides a large surface area for factor binding and capturing. A suitable acellular matrix could therefore be a nano-/micro-fibre based material such as a hydrogel or mesh. It could also comprise a network of nano-/micro-particles. The acellular compartment may be composed of a single material or be a composite material comprising two or more different types of fibre. For example, the scaffold may comprise fibronectin and collagen; collagen and polylactide; fibrin and collagen; collagen fibres and carbon- nanotubes; fibrin, collagen and fibronectin; collagen and hydroxyapatite.

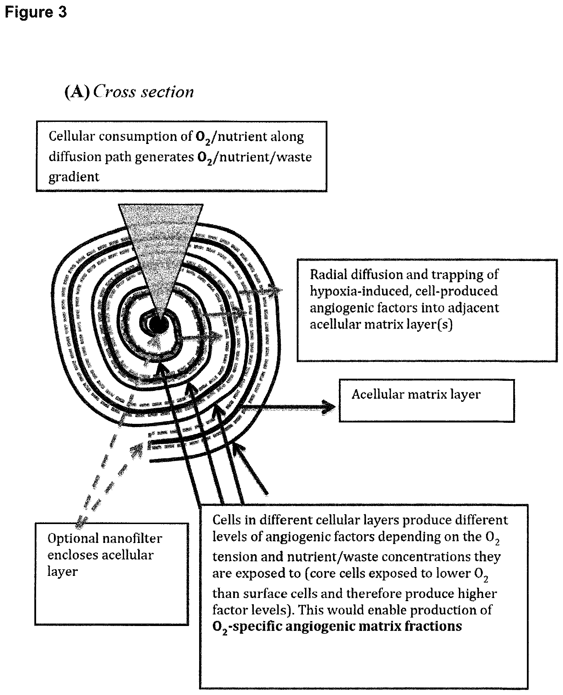

A nano-porous filter can be optionally positioned (e.g. in the form of a single layer sheet) between the cell-seeded compartment and the acellular compartment, or incorporated into the construct as a sheath enclosing the cellular or acellular compartment (Fig.1, 3). The pore size is preferably in the range of 1-1000 nm. This would prevent lateral movement (e.g. diffusion, migration) of micro-meter scale particles (e.g. cells, bacteria) from the cell-seeded compartment into the acellular compartment, while allowing free diffusion of nutrients (e.g. oxygen, glucose) from the media into the cell-seeded compartment, and outward diffusion of metabolic products (e.g. lactate) into media, and cellular growth factors from the cell-seeded compartment into the acellular compartment. Such a filter could be especially useful in cases where xenogeneic cells are used in order to ensure prevention of disease transmission (e.g. viruses, bacteria). The filter could comprise one or more layers of progressively decreasing pore size. This would ensure that the filter maintains its functionality even if pores become blocked in individual layers.

The concentration of angiogenic (e.g.VEGF) and other growth factors may be regulated to provide appropriate signalling to drive angiogenesis and other tissue repair/regeneration processes. The concentration of delivered exogenous growth factors will affect the number of binding events that happen on cell surface receptors, and the extent of subsequent downstream intracellular signaling that dictates the proliferation, migration and differentiation of cells (Cao and Mooney 2007). Factor concentration needs to reach a threshold to generate adequate intracellular signaling. For example, insufficient VEGF expression leads to embryonic lethality. On the other hand, excessive factor concentration may saturate the available receptors and downregulate receptor expression (Cao and Mooney 2007). The concentration of trapped factors in the acellular compartment will be determined by their ability to diffuse through the acellular matrix, which itself depends on the factor molecular size (note that different factors have different molecular weights, e.g. VEGF = 45 kDa, FGF = 18-22 kDa, Angiopoietin = 70 kDa, PDGF-B = 22kDa), the factor binding affinity to the matrix and the acellular matrix density/pore size/porosity (Cao and Mooney 2007). Therefore, use of native or biomimetic matrix, such as decellularised native matrix or collagen (and other biopolymeric e.g. fibrin, GAG) hydrogels (these can be optionally subjected to plastic compression), respectively, for the acellular matrix compartment are means of trapping of factors at physiological concentrations and ratios. This is preferred, not only because the optimal (i.e. physiological) concentration of several growth factors remains unknown, but also because additional, yet to be identified factors may potentially be involved in tissue repair and regeneration processes; capturing stress-induced factors within a native or biomimetic acellular matrix while they are being produced by cells, overcomes these limitations, which would not be possible by trying to reconstitute complex growth factor mixtures (either stress-induced factor or recombinant factors) at a later stage into media or any other form of vehicle.

Loading of acellular matrix with stress-induced cellular factors during culture occurs by diffusion of such factors from the cell-seeded compartment, through media, towards the acellular matrix compartment, and simultaneous trapping of such factors as they diffuse through the matrix. While it is preferred that this process occurs simultaneously while factors are being produced by cells, it is also possible to carry out the loading of factors from pre-conditioned media onto acellular matrix at a later stage (i.e. at the end of cellular stress stimulation), either by passive diffusion (e.g incubation of acellular matrix in pre-conditioned media) or active loading (e.g. flushing pre-conditioned media through a nano-/micro-porous acellular matrix using a pump).

One or more layers of acellular matrix can be used to trap cell-produced growth factors, depending on the matrix layer thickness and the matrix density/porosity/pore size (i.e. nano-/micro-scale structure). While the cell-seeded compartment and the acellular matrix are ideally shaped into a tubular/spiral form, to allow radial (i.e. 360°) trapping of factors produced, the acellular matrix can also be positioned as a (multi-) layer adjacent to any given plane of the cell-seeded compartment (Fig. 1). Depending on the diffusion rate of different factors through the matrix, spatial factor gradients form along the acellular matrix layers (i.e. higher factor concentration in layers closer to the cell-seeded compartment) (Fig. 1). Smaller factors, which diffuse faster through the acellular matrix, are found in higher concentration in layers further away from the cell-seeded compartment, in comparison to larger factors. In this way, the second carrier, preferably embodied as a spiral acellular matrix, acts as a filter to separate stress-induced factors based on their molecular size, which enables formation of 'factor-specific' matrix fractions (obtained by fragmenting the acellular matrix across the spiral layers; see, e.g. the different layers in Figure 3A). Since different factors act at different sites and at different time points during tissue repair/regeneration and angiogenic induction (for example, during angiogenesis VEGF initiates assembly of endothelial cells, followed by PDGF-BB-mediated recruitment of pericytes and smooth muscle cells, whereas angiopoietin-1 and TGF-b1 stabilize the nascent vessel (Carmeliet 2000)), such 'factor-specific' matrix fractions can be injected at specific tissue sites and at different time points, preferably to mimic the natural spatio-temporal sequence in which factors act *in vivo.* The density/porosity of the acellular matrix can be further controlled by any process that alters the fibrillar matrix structure (e.g. plastic compression, chemical-/photo-cross-linking, alignment of fibres by tensile loading, fibre alignment by application of magnetic fields (Cheema U et al. 2007; Dubey et al. 1999; Freeman and Silver 2005)).

In order to selectively load the acellular matrix with specific/preferred growth factors, it is possible to incorporate within the filter layer(s) enclosing it, one or more types of growth factor receptors (e.g. recombinant receptors for VEGF, FGF, PLGF, IGF etc.) or antibodies against growth factors. Receptors or antibodies within the filter can selectively capture specific factors from the produced growth factor mixture, thus excluding them from the factors reaching the acellular matrix. By excluding selected factors it could be possible to selectively tune the mixture factor composition towards desired factors. In addition, neutralizing antibodies (e.g. cytokine neutralizing antibodies) and/or receptors (e.g. cytokine receptors) and/or enzyme inhibitors (e.g. Tissue Inhibitors of Metalloproteinases) can be incorporated within the filter layer(s) in order to block/inhibit certain factors (e.g. inflammatory cytokines, Metalloproteinases), in order to avoid unwanted effects (e.g. inflammatory response, matrix degradation) upon administration.

The concentration of stress-induced cellular factors within the acellular compartment typically correlates with the length of exposure of cells to stress stimulation. For example, the duration of hypoxic culture/pre-conditioning determines the total amount of angiogenic factors produced by cells. Moreover, the expression profile of different angiogenic factors during angiogenesis *in vivo* varies with time (i.e. different factors have different expression profiles and are maximally upregulated at different time points) (Ozduman et al. 2010) (Komatsu and Hadjiargyrou 2004), and different factors produced by cells in response to hypoxia (e.g. angiopoietin 1 and 2) show temporal variation in their levels depending on the duration of hypoxic exposure (Ray et al. 2000). Therefore, by varying the duration of cell exposure to hypoxic stress, 'time-specific' angiogenic matrix fractions can be produced with unique angiogenic factor composition/concentration. In particular, the acellular matrix (e.g. spiral) layer(s) can be removed/detached (e.g. unspiralled) from the cell-seeded compartment at different time points during culture and replaced by fresh layer(s) to enable continuous sampling of produced angiogenic factors at different time intervals (e.g. every 5 days) (Fig. 1). Such 'time-specific' angiogenic matrix fractions can be injected at pre-determined, suitable time intervals (i.e. matching the temporal profile of factor expression in vivo), in order to temporally reconstruct/replicate a physiological angiogenic cascade. For example, a preferred injection regime could be injecting 0-5 day, 5-10 day and 10-15 day pre-conditioned matrix fractions at day 0, 5 and 10, respectively (i.e. the injection time interval corresponds to the time interval (5 days) between pre-conditioned matrix samples).

Fragmentation of acellular matrix containing cell-produced angiogenic factors can be any form of mechanical (i.e. non-enzymatic) fragmentation/sectioning (e.g. with a micro-dismembrator) that is able to break up the matrix into nano-/micro-meter scale fractions. While enzymatic degradation of the acellular matrix is envisaged, it is not preferred since; 1) added enzymes (e.g. collagenase) may not only break down the matrix protein(s), but also result in breakdown/deactivation of trapped factor proteins, and 2) injection of enzymes *in vivo* may result in unwanted tissue breakdown/degradation (with subsequent inflammatory reaction).

Factor-loaded matrix fractions can be preserved by freezing / freeze-drying at low temperature (typically -20 to -80 °C) for a period of months (e.g. up to 6 months) and can be added to a low temperature (1-5 °C) thermo-responsive solution (e.g. neutralised collagen solution) on demand. The thermo-responsive solution containing factor-loaded matrix fractions can be used immediately, or itself be stored at low temperature (1-5 °C) or frozen at -20 to -80 °C for a period that doesn't significantly reduce the stability and bioactivity of factors (e.g. 1-12 weeks).

Factor-loaded matrix fractions can also be prepared (e.g. by freeze-drying/lyophilisation) into a powder that can be applied in the form of a spray. For example, acellular matrix fractions containing hypoxia-induced angiogenic factors can be sprayed or sprinkled onto the skin (e.g. wounds, ulcers, incisions, abrasions, burns, grafts).

In accordance with item (a) of the fourth aspect of the invention, acellular matrix loaded with stress-induced (e.g. hypoxia-, mechanical stress-, electrical stimulation-induced) growth factors can be used without fragmentation in the form of a sheet or patch that can be attached/tethered/fixed (e.g. glued, sutured) on the surface of a tissue (e.g. bone, cartilage, muscle) or wrapped around a tissue such as nerve (the sheet can be folded into a tube to function as nerve conduit), tendon or bone during an operative procedure, such that localised factor release would aid tissue repair/regeneration. Acellular matrix containing hypoxia-induced angiogenic factors can also be used without fragmentation, in the form of a skin dressing, sheet or patch. This can be applied to the skin (e.g. wounds, ulcers, incisions, abrasions, burns, grafts) or internal organ (e.g. heart tissue, oesophagus, stomach, liver, viscera) surfaces by suturing or any other form of tethering. Release and diffusion of trapped angiogenic factors from the matrix into adjacent tissue will result in formation of angiogenic factor spatial gradients that will chemo-attract host endothelial cells into a wound/ulcer, graft or ischaemic tissue to aid its vascularisation, and therefore healing or take/survival (in the case of grafts). The functionality of an angiogenic matrix sheet/patch is not dependent on its integration with underlying tissue (although this is not precluded), for example when applied to skin wounds/ulcers it can function as a temporary angiogenic dressing that aids wound coverage and accelerates healing. Alternatively, it can function as a biodegradable patch that delivers factors to an internal organ surface.

Acellular matrix loaded with stress-induced growth factors can be prepared (e.g. pulled/shredded) in the form of threads/fine fibers/strips that can be used as suture material.

### Types of cellular stress

Exposure of cells to physiological hypoxia (0 to 10%, preferably 1-10 % (v/v) O₂), either through a controlled gas incubator (Fig.1) or through cellular O₂ consumption (Fig.2), is intended to produce up-regulation of a physiological angiogenic factor cascade. Cells that can be pre-conditioned to hypoxic stress to produce angiogenic factors are mammalian cells, including stromal cells (e.g. fibroblasts, bone marrow-derived stromal cells, smooth/skeletal/cardiac muscle cells), epithelial cells (e.g. endothelial cells) and stem cells (e.g. corneal stem cells; skin epidermal stem cells; gut stem cells; orogenital stem cells; epithelial stem cells; bone marrow stromal stem cells; adipose-derived stem cells; and growth plate stem cells). Factors produced may include, but are not limited to, vascular endothelial growth factor (VEGF), placental growth factor (PLGF), basic and acidic fibroblast growth factor (FGF), platelet-derived growth factor (PDGF), Angiopoietin-1 and -2, transforming growth factor alpha and beta (TGFa, TGFb), tumor necrosis factor (TNF), interleukin 8 (IL-8), insulin-like growth factor (IGF), hepatocyte growth factor (HGF), epidermal growth factor (EGF), nitric oxide synthase (NOS), angiogenin.

Mechanical stress stimuli that can be applied to cells, in order to replicate physiological mechanical loading, include compression, tension/stretch, shear, hydrostatic pressure (which acts equally in every direction), torsion and combinations of the above. Mechanical loading can be applied in various patterns (e.g. cyclical loading, step ramp loading) and frequencies (preferentially 1-100 Hz) depending on the cell type used and/or types of growth factors to be upregulated. For example, compression can be applied to osteoblasts and bone marrow stem cells for upregulation of bone-related growth factors (e.g. BMPs, IGF-I, IL-11,TGF-beta, PDGF-BB) and to chondrocytes for upregulation of cartilage-related growth factors (e.g. FGF, TGF-beta, BMPs, IGF-I, PDGF). Stretch can be applied to skeletal/smooth/cardiac muscle cells for upregulation of muscle-related growth factors (e.g. IGF-I, IL-15, TGF-beta) and tendon fibroblasts for upregulation of factors involved in tendon healing (e.g. TGF-beta, bFGF, PDGF).

Electrical stimulation represents another form of stress stimulus, that can be applied to nerve cells, glial cells (e.g. astrocytes, Schwann cells), neural stem cells, in order to upregulate factors that promote nerve repair/regeneration (e.g. nerve growth factor, brain-derived neurotrophic factor, neurotrophin-3, ciliary neurotrophic factor, and fibroblast growth factor, insulin growth factor-1). Skeletal muscle cells and cardiomyocytes can also be subjected to electrical stimulation. Brief (e.g. 30-60 min) low frequency (e.g. 1-50 Hz) electrical stimulation can be applied. However, higher frequencies (e.g. 50-100Hz) or periods of stimulation (e.g. 6-24 h) can also be used. Electrical stimulation can be applied to cells seeded within a electroactive / electro-conductive polymeric scaffold (e.g polylactide and aniline pentamer copolymer (Huang et al. 2008) or electrically active nano-material scaffolds such as composite materials with nanoscale, piezoelectric zinc oxide particles embedded into a polymer matrix (zinc oxide, when mechanically deformed through ultrasound, for example, can provide an electrical stimulus) (Seil and Webster 2010).

A combination of stress stimuli can be applied to cells within a bioreactor. This is particularly relevant for upregulating angiogenic factors, along with other tissue-specific factors involved in tissue repair/regeneration, since angiogenesis is a common pre-requisite for optimal regeneration and repair. For example, cells (e.g. skeletal muscle cells/myoblasts, bone marrow stem cells, cardiomyocytes) can be mechanically stimulated, while exposed to hypoxia within a controlled oxygen bioreactor; in addition to tissue-specific growth factors, such mechano-responsive cells would upregulate angiogenic factors (e.g. VEGF (Germani et al. 2003; Wang et al. 2007), secretoneurin (Egger et al. 2007), erythropoietin, angiopoietin-1 (Hu et al. 2008), PLGF (Torry et al. 2009). Such complex growth factor mixtures (e.g. angiogenic and bone-related growth factors; angiogenic and muscle-related growth factors) can be simultaneously captured, as they are being produced by cells, within the same acellular matrix and delivered to stimulate a bi-functional response (e.g. muscle regeneration and angiogenesis, bone regeneration and angiogenesis).

Acellular matrix fractions loaded with factors induced by specific stress stimuli can be delivered in isolation or in combination. For example, acellular matrix fractions loaded with hypoxia-induced angiogenic factors can be co-delivered with matrix fractions loaded with mechanically-induced bone growth factors and/or cartilage growth factors and/or muscle growth factors, in order to stimulate supportive angiogenesis along with tissue-specific repair/regeneration (Fig. 5; Table 1). Acellular matrix fractions with hypoxia-induced angiogenic factors can also be co-delivered with electrically-stimulated neurotrophic factors, since there is evidence that angiogenic factors (e.g. VEGF) play a neuroprotective role in nerve injury and repair (Ara et al. 2011; Rosenstein et al. 2010). Other possible growth factor mixture combinations may include bone-related and neurotrophic factors, muscle-related and bone-related factors, bone-related and cartilage-related factors (Fig. 5; Table 1).

### Delivery vehicles

The ability to locally deliver factors, as opposed to systemically administer them or deliver them in liquid media (that rapidly and widely leak into the tissue), is key for controlling the directionality of a tissue regenerative process. For example, natural diffusion of locally delivered matrix fractions with angiogenic factors into host tissue will predictably result in formation of physiological spatial gradients, which will chemo-attract host endothelial cells towards the injection site (i.e. towards the target tissue) (Barkefors et al. 2008; Cao and Mooney 2007), to aid its vascularisation and repair. The ability of hypoxia-induced angiogenic factors (e.g. VEGF₁₆₅), trapped within a biomimetic matrix, to diffuse and form a spatial gradient over time through a collagen hydrogel has already been tested and confirmed *in vitro* (Hadjipanayi et al. 2011). Spatial gradients of neurotrophic factors will also directionally guide axonal growth and nerve regeneration (Bonner et al. 2010).

An injectable sol-gel containing factor-loaded matrix fractions preferably undergoes a solution-to-gel phase transition at body temperature, and therefore it remains (together with the contained matrix fractions) localised at the injection site. The gel then effectively acts as a reservoir that sequesters the factors at the injection site, while releasing them in a sustained/controlled manner (i.e. avoiding spikes in factor concentration and short-lived effects). In the case of delivering growth factor carriers for bone/cartilage repair, the gel permits containment, as well as structural conformation of the carriers to the defect shape. Thermo-reversible gels (e.g. collagen, agarose gel) also have the benefit of serving as growth permissive 3D scaffolds, as well as having the ability to present factors (e.g. neurotrophic factors) to the injury site, which is specifically relevant for nerve regeneration after injury, i.e. the gel acts as a nerve conduit.

Injection of acellular factor carriers (e.g. factor-loaded matrix fractions) through a sol-gel/conventional gel represents a minimally invasive form of administration, which allows repeated delivery of factors, which is important for temporal control of the response. In particular, 'time-specific' matrix fractions can be injected at pre-determined, suitable time intervals (i.e. matching the temporal profile of factor expression *in vivo*), in order to temporally reconstruct/replicate a physiological growth factor cascade. For example, a preferred injection regime could be injecting 0-5 day, 5-10 day and 10-15 day pre-conditioned matrix fractions at day 0, 5 and 10, respectively (i.e. the injection time interval corresponds to the time interval (5 days) between pre-conditioning groups).

Localised delivery of growth factors through an injectable sol-gel or conventional gel can help prevent unwanted systemic side effects (e.g. ectopic angiogenesis, vascular leakage, mitogenic effects etc) that are common with systemic factor delivery or injection of liquid media that leak into tissue. It also ensures that an adequate factor dose/concentration is achieved at the delivery site, which is a pre-requisite for a successful response.

Collagen hydrogels are preferable candidate delivery vehicles as they are thermo-responsive, biocompatible (i.e. non-immunogenic, FDA approved) and biomimetic (e.g. they undergo natural cellular remodelling). Collagen tissue is tissue obtained from a natural source which contains collagen. Preferably, the collagen tissue is mammalian, marine or avian collagen tissue. Examples of collagen containing tissues include bone, skin, tendon and ligament. Preferably, the collagen tissue is ligament or tendon, which has been found to have low levels of contaminants.

Neutralised collagen solution for injection is obtained by neutralising acid-soluble collagen (e.g. collagen dissolved in acetic acid, hydrochloric acid etc.) with an alkali (e.g. NaOH). For the collagen solution, native fibril-forming collagen types can be used including collagen types I, II, III, V, VI, IX and XI and combinations of these (e.g. I, III V or II, IX, XI). Preferably, collagen type I is used. Collagen can be used in its monomeric and/or polymeric form.

Collagen concentration in the collagen solution should be greater than about 1 mg/ml, preferably between 1-10mg/ml. Collagen concentration will determine the rate of sol-gel transition within the body (faster gelling at higher concentrations, see results). Neutralised collagen solution containing matrix fractions can be briefly (e.g. 1-30 min) pre-warmed (e.g. at 10-37°C) before injection in order to speed up the gelling process after injection. Faster gelling would ensure less leakage of injected solution and better localisation of factor-loaded matrix fractions at the injection site. Gels are formed by the coalescence and elongation of fibrils, as the fibrils form a continuous network around the aqueous interstitial liquid which originally held the monomers. For example, triple helical collagen monomers may be induced to polymerize (aggregate) to fibrils (e.g. at 37°C and neutral pH). As the fibrils polymerise, there is a phase transition and the solid network of fibrils supports the remaining interstitial liquid in approximately the same volume and shape - i.e. it gels. Phase transition from soluble monomer to solid polymer is characteristic of a gel. FDA approved collagen (e.g. bovine collagen, marine collagen) can be used to produce both the matrix component and the collagen solution for injection.

While collagen is the preferred canditate for preparation of the thermo-responsive solution which is used to locally deliver the angiogenic matrix fractions, other biodegradable temperature-sensitive polymers including polyesters, polyphosphazenes, polypeptides, and chitosan, and pH/temperature-sensitive polymers such as sulfamethazine-, poly(beta-amino ester)-, poly(amino urethane)-, and poly(amidoamine)-based polymers can also be used (Nguyen and Lee 2010). Thermo-responsive hydrogels can be based on natural polymers, N-isopropylacrylamide polymers, poly(ethylene oxide)-b-poly(propylene oxide)-b-poly(ethylene oxide) polymers, as well as poly(ethylene glycol) / poly(propylene fumarate-co-ethylene glycol)-biodegradable polyester copolymers (Klouda and Mikos 2008) (Fisher et al. 2004). Recently developed block copolymer hydrogels that respond to temperature, pH or both stimuli, are discussed by He et al., and include poly(N-substituted acrylamide)-based block copolymers, poloxamers and their derivatives, poly(ethylene glycol)-polyester block copolymers, polyelectrolyte-based block copolymers and the polyelectrolyte-modified thermo-sensitive block copolymers (He et al. 2008).

In addition to an injectable thermo-responsive solution, a delivery vehicle for carriers of stress-induced cellular factors can be a semi-solid material, such as a conventional gel of an appropriate viscosity that allows it to be injected (e.g. alginate gel (Mierisch et al. 2002; Silva and Mooney 2007), fibrin gel (Ishii et al. 2007).

The *in vivo* biodegradation rate, mechanical properties and the density/pore size/porosity (i.e. inner three dimensional network) of the resulting gel can be controlled by cross-linking (Potta et al. 2010), which will determine the growth factor release kinetics (factor release kinetics will additionally be determined by the nano-/micro-scale structure and biodegradability of the matrix in which they are trapped).

Delivery of sol-gel/gel into a target tissue by injection can be through conventional needles or endoscopes.

In addition to delivery by injection, factor-loaded matrix fractions can be prepared in the form of a powder that can be sprayed or sprinkled onto a superficial skin area, e.g. one that requires vascularisation (e.g. wound, ulcer, burn, abrasion, incision, skin graft) to aid healing.

An acellular matrix sheet loaded with a stress-induced factor mixture (or combination of factor mixtures) can also be used without fragmentation in the form of a sheet/dressing/patch (single layer or multi-layer). For example, an acellular matrix sheet loaded with angiogenic factors can be applied as (temporary) dressing (e.g. sutured, glued, simple fixation) onto a skin area that requires vascularisation (e.g. wound, ulcer, skin graft, burn, abrasion, incision). Similarly, an angiogenic matrix sheet can be applied as a biodegradable patch on an infarcted or ischaemic area (e.g. myocardium, skeletal muscle) during surgery to aid its vascularisation. Natural diffusion of locally delivered factors into the tissue will result in development of spatial gradients that will directionally chemo-attract endothelial cells towards the target site to promote angiogenesis and accelerate healing. Also, an acellular matrix sheet loaded with neurotrophic factors can be wrapped around lesioned nerves as a factor-delivery sheath, or can act directly as a gap-bridging guidance conduit that delivers factors, to aid nerve repair/regeneration. A similar concept can be employed in injured tendons, where a factor-loaded acellular matrix sheet can be wrapped around the tendon suture site, tendon-bone or muscle-tendon interface to aid tendon repair/healing (Fig. 5; Table 1). Acellular matrix loaded with stress-induced growth factors can also be prepared (e.g. pulled/shredded) in the form of threads/fine fibers/strips that can be used as suture material.

### Target tissue sites

Target sites for delivering acellular carriers with angiogenic factor mixtures can include tissues where vascular supply is impaired (e.g. infarcted cardiac/cerebral/peripheral tissue, wounds, decubitus ulcers, diabetic ulcers, incisions/lacerations, radiation wounds, steroid wounds, venous stasis wounds, burns). Such tissues are encountered in various conditions including vascular occlusive disease; peripheral arterial disease; atherosclerosis; myointimal hyperplasia; thromboangiitis obliterans; thrombotic disorders; mesenteric or limb ischemia; stenosis; vasculitis, myocardial and cerebral infarctions or other vascular death, diabetes, traumatic injuries and (non-union) fractures (e.g. bone). In addition, angiogenic factor mixtures can be applied at implant sites (e.g. skin grafts, flaps, cosmetic implants) or other transplanted tissue (native or engineered) to improve implant vascularisation and graft take.

Target sites for delivering carriers with bone-related growth factor mixtures include osteoporotic bone, osteoporotic-related fractures, osteoarthritic and rheumatic joints, (non-union) fractures that arise as a result of injury/trauma.

Target sites for delivering carriers with cartilage-related growth factor mixtures include arthritic joints and sites of traumatic cartilage rupture or detachment (e.g. knee).

Target sites for delivering carriers with tendon-related growth factor mixtures include injured (e.g. torn, ruptured) tendon.

Target sites for delivering carriers with muscle-related growth factor mixtures include sites of muscle atrophy and wasting as a result of prolonged immobilization (e.g. patients with spinal injury, burns), muscular dystrophies or amyotrophic lateral sclerosis, cancer and AIDS.

Target sites for delivering carriers with nerve-related growth factor mixtures include sites of central or peripheral nerve injury (e.g. traumatic lesion) or degeneration.

The Figures show:
- **Fig. 1**: Setup for exposing cells to hypoxic stress for upregulation of angiogenic growth factors, and time-dependent collection of produced cellular factors within an acellular matrix positioned adjacent to or wrapped around a central cell-seeded compartment.
- **Fig. 2**: A second setup for culturing cells under hypoxic stress, where the construct comprises alternating cell-seeded matrix sheet/cell-sheet layers and acellular matrix layers, which enables capturing of oxygen tension-specific angiogenic factors within the different acellular matrix layers.
- **Fig. 3**: (A) Schematic of nano-porous filter that can be optionally positioned to enclose the acellular matrix layer in order to prevent movement of cells/micro-organisms from the cell-seeded compartment into the acellular compartment. (B) System setup for exposing peripheral blood cells to hypoxia and capturing produced factors in order to deliver them into the same, blood donor, patient. The factors can be captured within a multilayer nano-/micro-porous matrix that can be applied in sheets onto wounds as a temporary/biodegradable dressing (B1), or within a nano-fiber/nano-particle network that can be mixed with a sol-gel for injection into ischaemic tissue (B2).
- **Fig. 4**: Schematic of process for generating an injectable sol-gel, spray or sheet/dressing with hypoxia-induced cellular angiogenic factors.
- **Fig.5**: Schematic of setups for application of various forms of stress stimulation on constructs comprising a central cellular compartment and an outer acellular compartment (for collection of produced factors). Table 1 provides examples of suitable cell types, tissue-specific growth factors produced, possible growth factor mixture combinations, delivery methods of factor-loaded acellular matrices, and target tissue sites for each stress stimulus and combinations of stress stimuli. that can be applied together.
- **Fig. 6**: a) Plot showing *in vitro* gelling times for neutralised collagen (bovine type I collagen) solutions (volume; 1 ml) of protein concentration 2 and 4 mg/ml, at 37 °C. Four collagen solutions were tested for each condition (n=4), *p<0.05.
b) Plot comparing gelling times for neutralized collagen solutions (volume; 1ml) of protein concentration (4mg/ml) that were either incubated at 37°C immediately after neutralization or that were stored at +5°C for 3 weeks before incubation at 37°C. Four collagen solutions were tested for each condition (n=4).
c) Plot showing the effect of freezing a neutralised collagen solution at -20°C on gelling time. Neutralized collagen solutions (volume; 1ml) of protein concentration (4mg/ml) were either incubated at 37°C directly after neutralisation or frozen at-20°C and stored for 1 hr or 3 weeks, then thawed at +20°C for 1 hr and incubated at 37°C. Four collagen solutions were tested for each condition (n=4), *p<0.05.

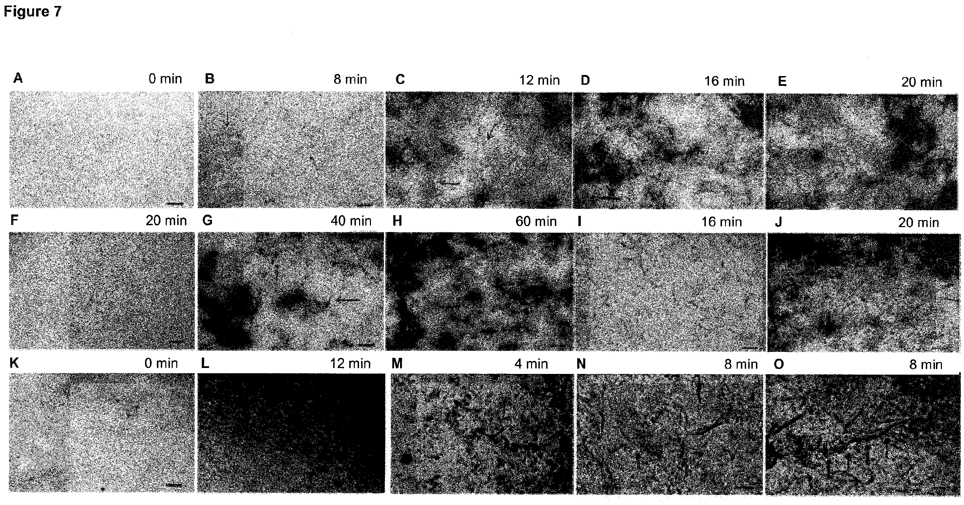

- **Fig. 7**: Time series of phase contrast micrographs of collagen solutions (same sample analysed over time for each condition) at protein concentration 4mg/ml (A-E) and 2mg/ml (F-H), that were incubated at 37°C directly after neutralisation with alkali. Collagen solutions of concentration 4mg/ml were also pre-stored for 1 hr at -20°C (I-J), and for 3 weeks at +5°C (K,L) or at -20°C (M-O), prior to incubation at 37 °C (frozen samples stored at -20°C were thawed at +20°C for 1 hr prior to incubation at 37 °C). Arrows show branched bundles of filaments (B,C,G) or larger fibre-cluster structures (N-O). Bars=100µm.

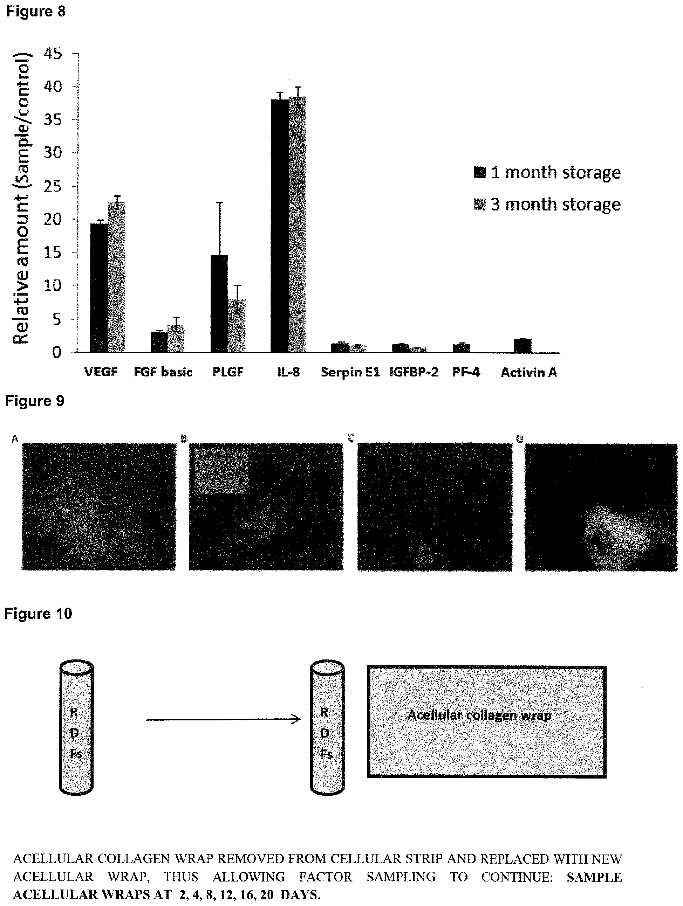

- **Fig. 8**: Plot showing the profile of angiogenic factors that were trapped in 1 and 3 month-stored(at -20°C) angiogenic matrix fractions (AFMs), and delivered by diffusion into media (DMEM), through a type I collagen sol-gel (4mg/ml). Y-axis represents the ratio of factor amount in sample medium (added to collagen gels containing AFMs) over control medium (added to empty collagen gels). 3 samples were tested for each condition. Bars indicate standard deviation of means.
- **Fig.9**: Immunofluorescent staining of VEGF (red: A,B,C) and HIF-1a (green: D), within cell-seeded (A,B,D) and acellular (C) matrix fractions obtained from fragmentation of 2 week hypoxia pre-conditioned collagen constructs (comprising a core cellular compartment and outer acellular matrix wrap), stored for 3 months at -20°C and added to a type I collagen solution (4mg/ml), which was allowed to set into gel at 37°C. DAPI (blue) nuclear staining was positive in cellular matrix fractions. Inset in image B shows corresponding field viewed under phase contrast.

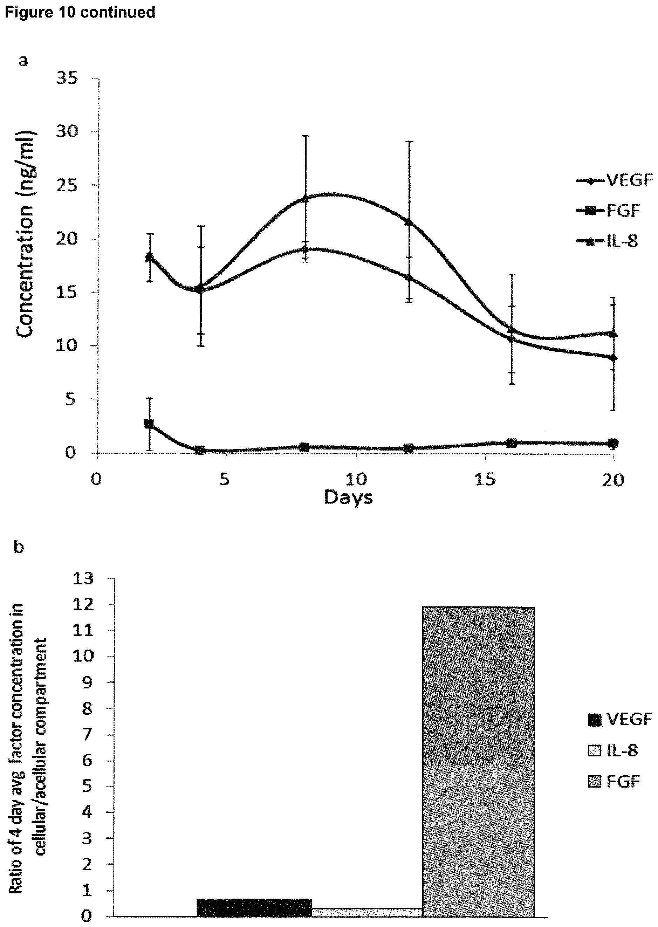

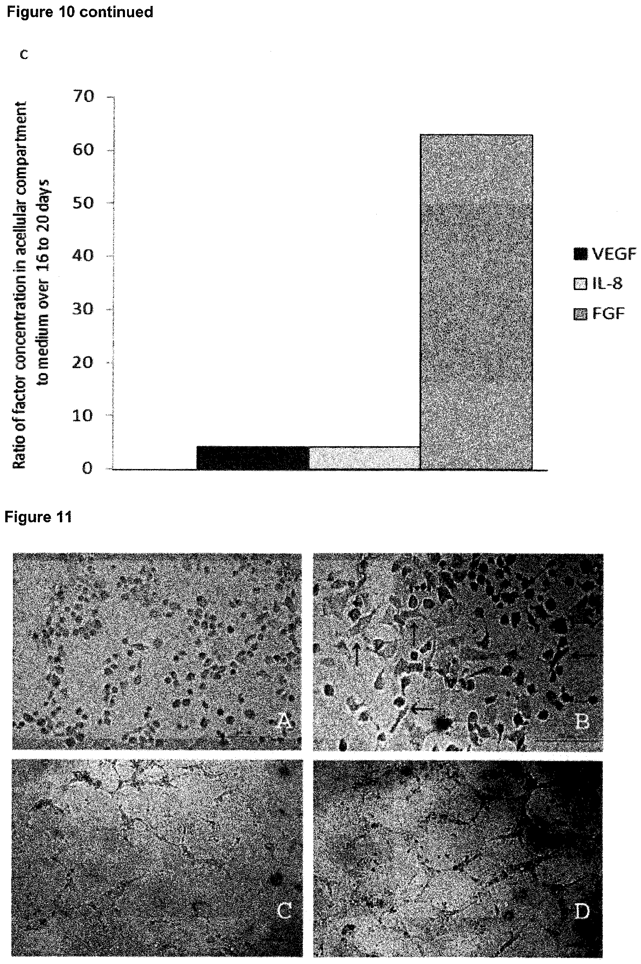

- **Fig. 10**: Analysis of the temporal profile of hypoxia-induced factor expression. Schematic shows the setup used for analysis of the temporal profile of hypoxia-induced angiogenic factors, produced by rabbit dermal fibroblasts (RDFs) cultured at 5% O₂, over 20 days. a) Plot of the temporal profile of factor concentration in the acellular matrix compartment at 2,4,8,12,16, and 20 days of *in vitro* culture at 5% O₂. Four samples were tested for each factor (n=4). Bars represent standard deviation of means. b) Plot showing the ratio of the 4 day average factor concentration in the cellular compartment to that in the acellular compartment, over the 20 day culture period (n=4). c) Plot showing the ratio of factor concentration in the acellular compartment to that in the medium over 16 to 20 days of culture (n=4).
- **Fig. 11**: Hypoxia-induced factors, delivered from 2 week culture matrix fractions into media by diffusion through type I collagen sol-gel, induce tubule formation *in vitro.* HUVECs were seeded at low density (∼10×10³ cells/cm²; A,B) and high density (40×10³ cells/cm²; C,D) on Matrigel-coated plates and cultured with factor-containing media (A,C) or control media (DMEM) (B,D) for 48hrs. Phase contrast microscopy images show elongated cells in B (arrowed) and network tube formation in D, bars=100µm.
- **Fig.12**: Schematic showing a prototype device for one-step harvesting and delivering angiogenic factor mixtures from autologous peripheral blood.
A) Macroscopic view.
B) Detailed cross-section. A desired volume of blood (e.g. 10ml) is added to the lower compartment, initially under vacuum, by aspiration through a port (1) that is designed to be compatible with standard blood-drawing equipment (e.g. BD Vacutainer ® system). The port has a double rubber-membrane for extra safety, and extends off the compartment's wall thus allowing mounting of the barrel that protects the piercing-needle (at the other end of the blood collection needle) during handling. The lower compartment contains an anticoagulant (e.g. EDTA) so that added blood remains in liquid state, ensuring uniform distribution of blood cells onto the matrix scaffold support at the bottom (2). Serum-free medium (optionally containing a hypoxia-mimicking agent, e.g. CoCl_{2,} hydralazine) is then added to the lower compartment through a second port with a double rubber-membrane (3). Depending on the volume of blood added (controlled though the scale on the wall window, shown in A), serum-free medium is added at a pre-specified volume that completely fills the lower compartment and slightly extends, though the nano-porous filter (4), into the upper compartment. The filter prevents movement of cells and pathogens into the upper compartment. The vented cap (5), communicating with a nano-porous hydrophobic membrane for gas-exchange (6), is then opened. The device is now ready for conditioning, in a 37°C 5% CO₂ incubator, under hypoxia (1-10% O₂). For space-efficiency, multiple devices can be stacked on top of each other in an incubator, by removing the device base (7) and fitting it onto the vented cap of the device below. Air flow is maintained through the openings at the side-walls of the base (8). During culture, blood cells up-regulate production of angiogenic factor proteins that diffuse into the upper compartment, through the nano-porous filter, and are trapped within a cell-free matrix carrier (9). At the end of conditioning, the carrier is removed by unscrewing the lid of the upper compartment (10).
C) Schematic showing a prototype device for one-step harvesting and delivering angiogenic factor mixtures from autologous peripheral blood. a) Prototype version for delivering factors in the form of a wound dressing. b) Prototype version for delivering factors as an injectable preparation. For full description of operational procedure see legend for part (A) of this figure. The carrier can either be a nano-porous matrix (e.g. collagen gel) protected within two fiber-based meshes (a,9), or comprise micro-particles (e.g. collagen-coated microbeads (b,9). At the end of conditioning, the wound-dressing matrix carrier is removed by unscrewing the lid of the upper compartment (a, 10). Factor-loaded microbeads are combined with a solgel delivery vehicle (e.g. neutralized cold collagen gel) that is added though a port in the upper compartment (b, 10), and mixed by agitation/shaking to obtain an injectable preparation, which is removed from the upper compartment though the same syringe that was used to add the sol-gel (syringe remains attached to port 10 during shaking).
D) Schematic showing a prototype device for one-step harvesting and delivering angiogenic factor mixtures from autologous peripheral blood. Prototype version for delivering factors as multiple injectable preparations. For operational description see legend for part (C) of this figure. By dividing the upper compartment into two or more sub-compartments, it is possible to deliver factors at different time points, which correspond to conditioning phases of different duration/length. This could be useful for example when the factors need to be delivered acutely (i.e. in an emergency situation), which could be compromising for the conditioning phase; in such cases, a short conditioning-length preparation can be delivered early on, while a second (or more) preparation (s) of more optimized conditioning (and composition) are being prepared.
E) 3D schematic of device for delivering angiogenic factors from autologous peripheral blood in the form of wound dressings.

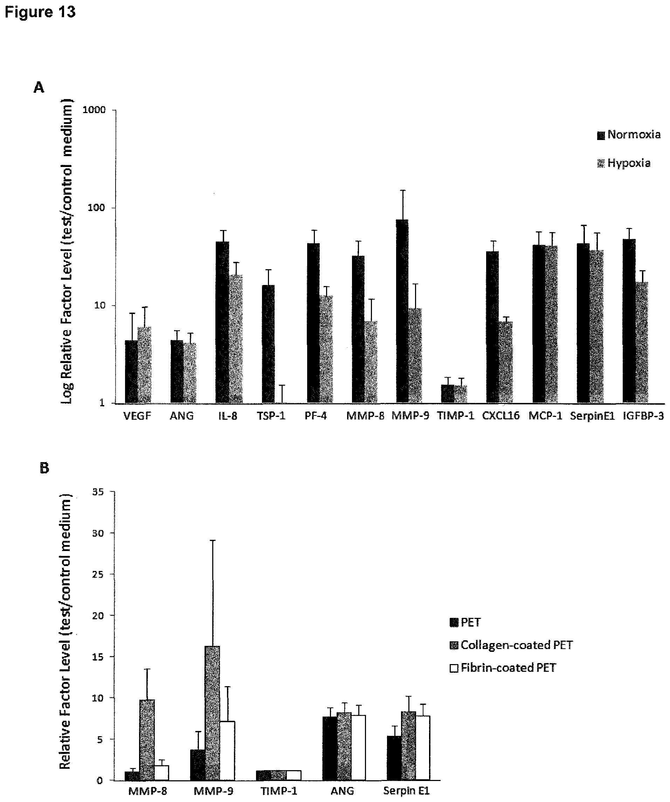

- **Fig.13**: Oxygen tension and cell-scaffold material composition influence the expression profile of PBC-generated angiogenesis-related proteins. A) Plot showing the profile of angiogenic factor proteins, as analysed by proteome profiler array, that were produced by PBCs cultured on collagen-coated PET membranes and exposed to either normoxia or hypoxia (3%02) for 10 days. B) Plot showing the profile of angiogenic factor proteins produced by PBCs cultured on PET-, collagen-coated PET or fibrin-coated PET membranes under hypoxia (3%02) for 10 days. Y-axis represents the ratio of factor level in culture supernatant over control serum-free medium. Three samples were tested for each condition. Bars indicate standard deviation of means.

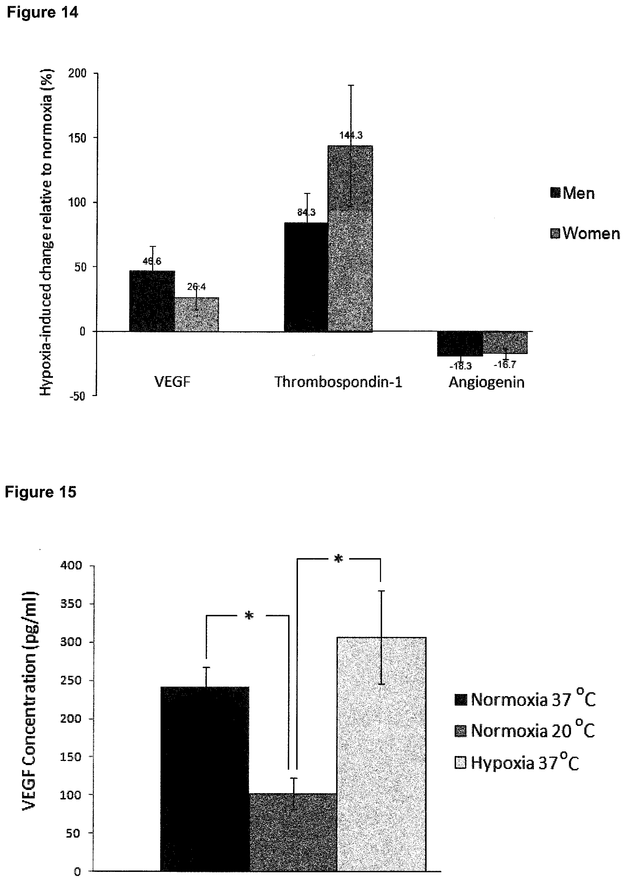

- **Fig.14**: Effect of hypoxia on VEGF, Thrombospondin-1 and Angiogenin protein expression by PBCs. Plot showing the percentage change in the level of factor protein expression when PBCs were cultured under hypoxia (3%O2) compared to when PBCs were cultured under normoxia for 7 days. Peripheral blood was obtained from 19 non-smoking healthy subjects (9 males; age=38.3±6yrs, BMI=26.3±1.3Kg/m², and 10 females; age=30.7±2.2yrs, BMI=22.5±1Kg/m²). Whole blood samples were cultured under hypoxia and normoxia on a polystyrene surface for 7 days. Quantitative analysis of factor protein levels in cell culture supernatants was carried out by ELISA, with 2 samples tested for each subject, per condition. Bars indicate standard error of the mean. Population mean hypoxic vs. normoxic supernatant concentrations were; VEGF=50.5±5.2pg/ml vs. 41.9±6.2pg/ml (p<0.05), TSP-1=226.3±13.4ng/ml vs. 118.7±7.3ng/ml (p<0.001), ANG=3192.5±108pg/ml vs. 3938±160.8pg/ml (p<0.001).
- **Fig.15**: Effect of ambient temperature on VEGF protein expression by PBCs. Plot showing the concentration of VEGF in culture supernatants, as measured by ELISA, obtained when PBCs were cultured on a collagen-coated polystyrene surface under normoxia at 37°C, normoxia at 20°C or hypoxia (3%O₂) at 37°C for 8 days. Three samples were tested for each condition. Bars indicate standard deviation of means, *p<0.05.

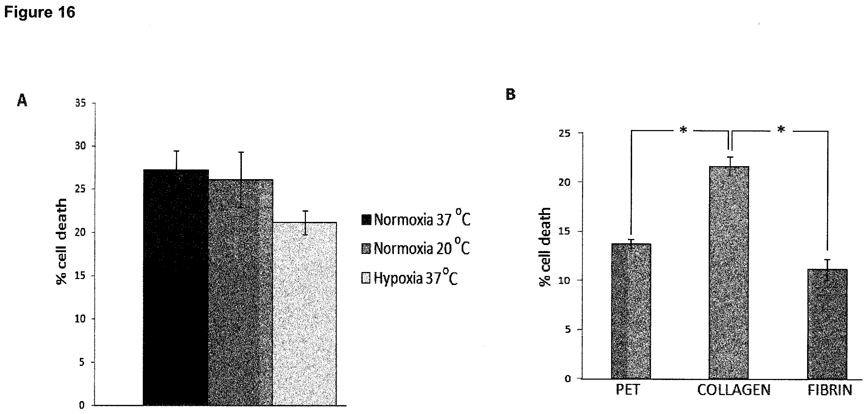

- **Fig.16**: Effect of oxygen tension, temperature and cell-scaffold material composition on total PBC viability. A) Plot showing percentage cell death relative to total cell number, as measured by LDH cytotoxicity assay, in PBCs cultured on PET-membranes under normoxia at 37°C, normoxia at 20°C or hypoxia (3%O₂) at 37°C for 8 days. B) Plot showing percentage cell death relative to total cell number in PBCs cultured on PET-, collagen-coated PET or fibrin-coated PET membranes under hypoxia (3%O2) for 10 days. Three samples were tested for each condition. Bars indicate standard deviation of means, *p<0.05.

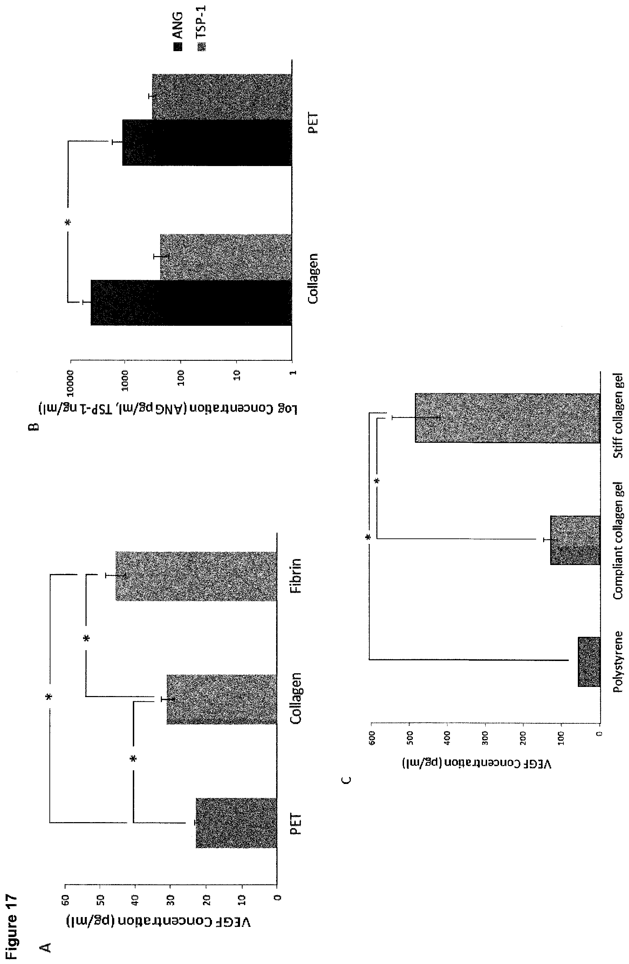

- **Fig.17**: Effect of cell-scaffold material composition and matrix stiffness on angiogenic factor expression by PBCs. A) Plot showing VEGF concentration in culture supernatants when PBCs were cultured on PET-, collagen-coated PET or fibrin-coated PET membranes under hypoxia (3%O2) for 10 days. B) Plot showing Angiogenin (ANG) and Thrombospondin-1 (TSP-1) concentration in culture supernatants when PBCs were cultured on collagen-coated PET- or naked PET-membranes under hypoxia (3%O2) for 8 days. C) Plot showing VEGF concentration in culture supernatants when PBCs were cultured on a polystyrene surface, compliant (∼50kPa) collagen gel matrix or stiff (∼2000kPa) collagen gel matrix under normoxia for 10 days. Three samples were tested for each condition. Bars indicate standard deviation of means, *p<0.05.

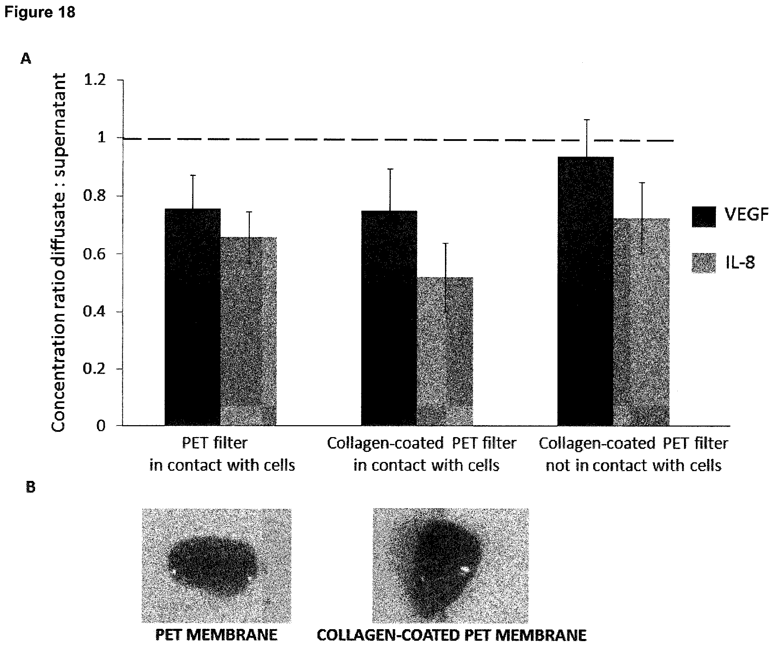

- **Fig.18**: Effect of cell-scaffold position relative to that of the filter on VEGF and IL-8 diffusion through the filter. A) Plot showing the ratio of VEGF and IL-8 concentration in the diffusate to that in the culture supernatant. PBCs were cultured on PET- or collagen-coated PET 1µm pore membranes (cells in contact with filter) or on a polystyrene surface, with a collagen-coated PET 1µm pore membrane suspended above the cells (cells not in contact with filter), under normoxia for 10 days. Media above the cell layer (supernatants) and media passing through the filter-membrane (diffusates) were then sampled and tested for VEGF and IL-8 by ELISA. Dotted line represents level of optimal diffusion, i.e. factor concentration in the diffusate equals that in the supernatant. Three samples were tested for each condition. Bars indicate standard deviation of means. B) Macroscopic images of the dense cell layer (cell-plug) that formed when PBCs were cultured (in the absence of plasma, i.e.clotting factors) on PET- or collagen-coated PET membranes for 10 days. PBC layers were lifted off the membranes and placed on a white background support.

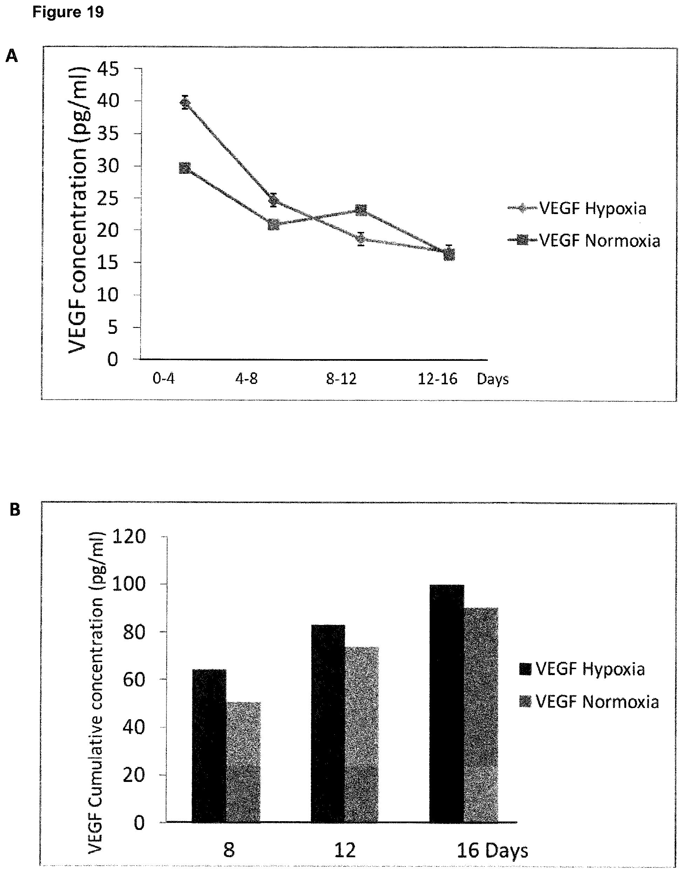

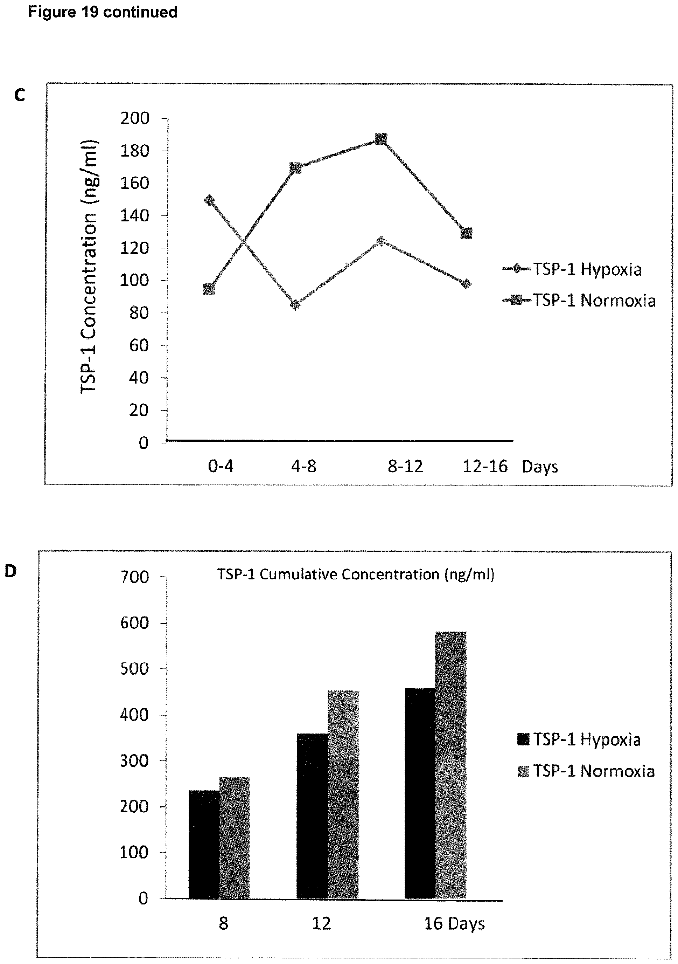

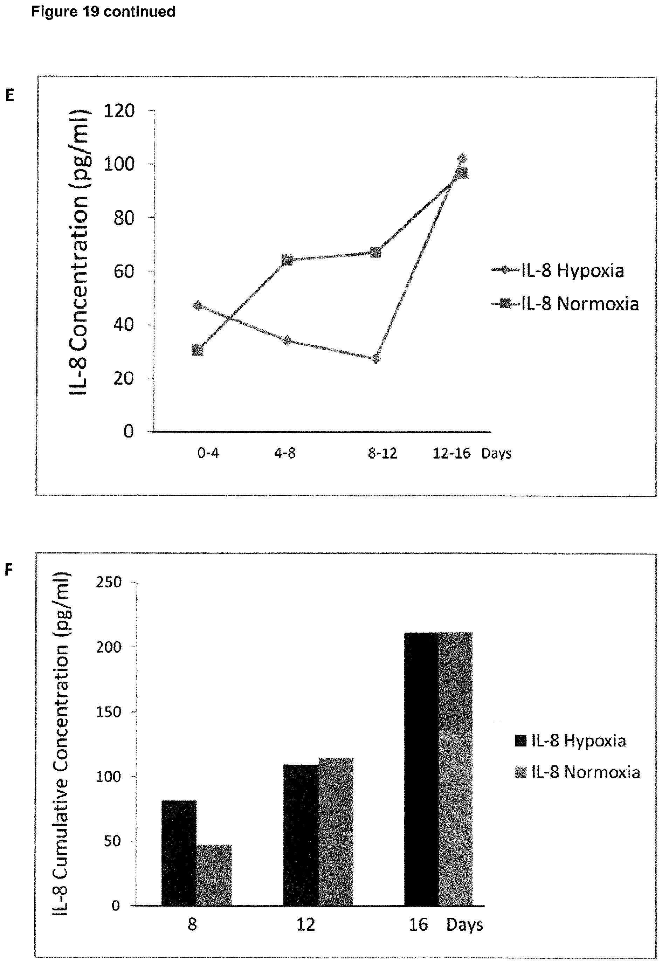

- **Fig.19**: Temporal profile of VEGF (a,b),Thrombospondin-1 (c,d) and Interleukin-8 (e,f) expression by PBCs cultured on collagen-coated PET membranes under Hypoxia (3%O₂) and Normoxia for 16 days. Plots a, c and e show factor levels in media sampled every 4 days, while plots b,d and f show cumulative factor levels for the same data. 3 samples were tested per factor per condition.

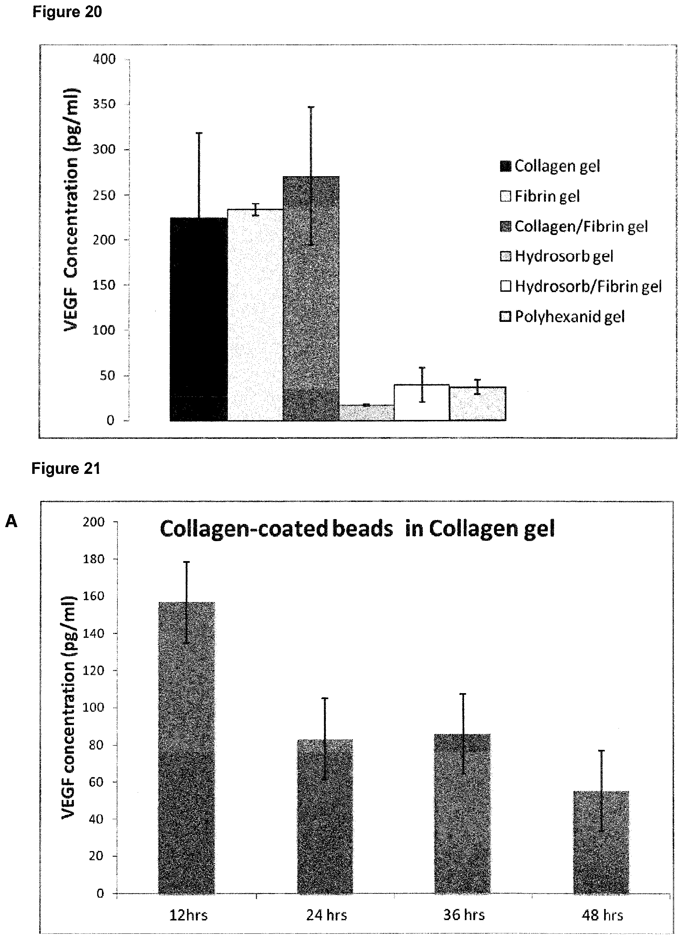

- **Fig.20**: VEGF release from gel carriers loaded with PBC-produced factors, after 12 days culture under hypoxia (3%O₂). Gel carriers (total of 3ml) tested were type I collagen gel, fibrin gel, collagen(2ml)/fibrin(1ml) gel, Hydrosorb gel and Hydrosorb(2ml)/fibrin(1ml) gel and Polyhexanid gel. Bars show standard deviation of means. 3 samples were tested per carrier type.

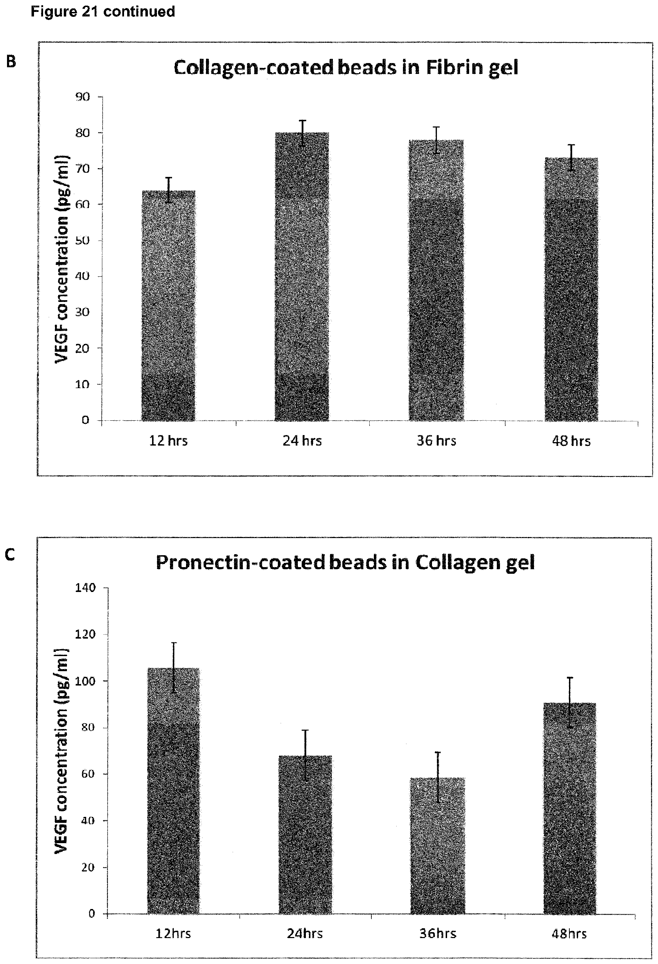

- **Fig.21**: VEGF release from microbead carriers, embedded in sol-gel, over 12, 24, 36 and 48hrs. Microbeads were incubated with PBCs under under hypoxia (3% O₂) for 12 days. After culture, collagen-coated microbeads were added to collagen gel (a) or fibrin gel (b), while pronectin-coated microbeads were added to collagen gels (c). Bars show standard deviation of means. 3 samples were tested per carrier type.

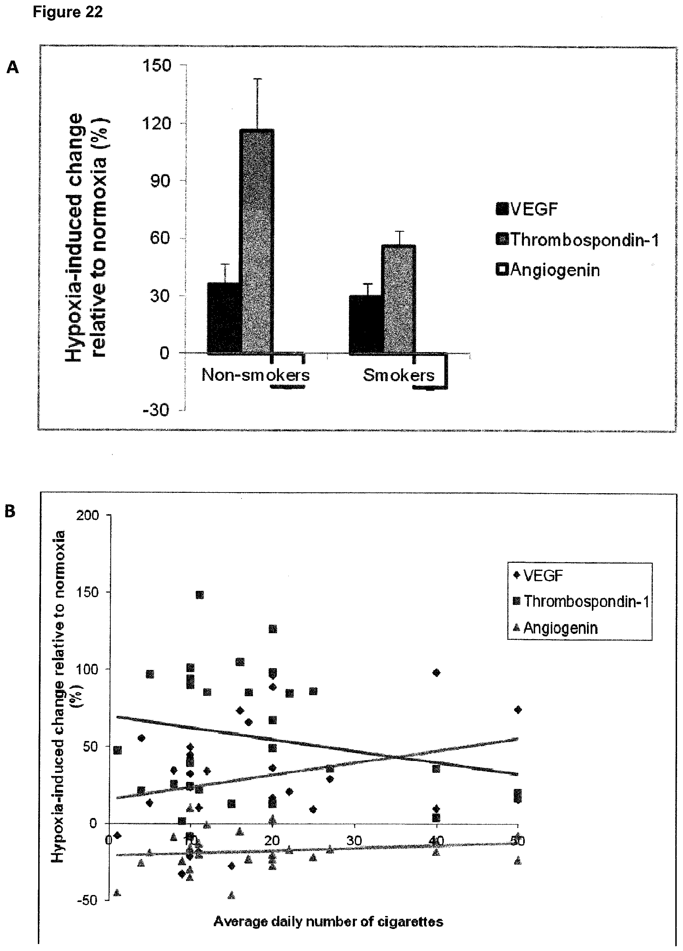

- **Fig.22**: Comparison of the angiogenic potential of PBCs derived from smokers vs. non-smokers, under 7 days hypoxic and normoxic culture. a) Plot showing the percentage hypoxia-induced change for VEGF, TSP-1 and ANG, relative to the normoxic baseline, in smokers and non-smokers. Bars show standard error of mean. 3 samples were tested per factor per condition. b) Plot showing the correlation of the percentage hypoxia-induced change for VEGF, TSP-1 and ANG, relative to the normoxic baseline, with daily cigarette consumption. Lines show linear correlation of best-fit.

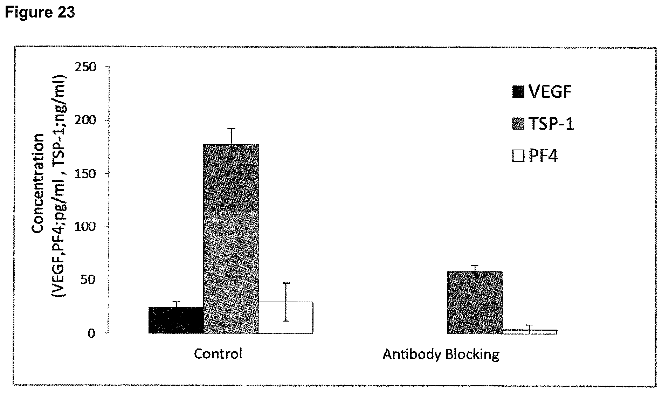

- **Fig.23**: Addition of factor-specific antibodies in PBC culture media produces a reduction in the concentration of the target factor in the carrier. Plot showing the concentration of VEGF, TSP-1 and PF4 in collagen gel carrier releasates, when carriers were incubated with PBC hypoxic cultures, where the culture medium contained an excess concentration of antibodies against VEGF, TSP-1 or PF4, or no antibodies (control). Bars show standard deviation of means. 3 samples were tested per condition.

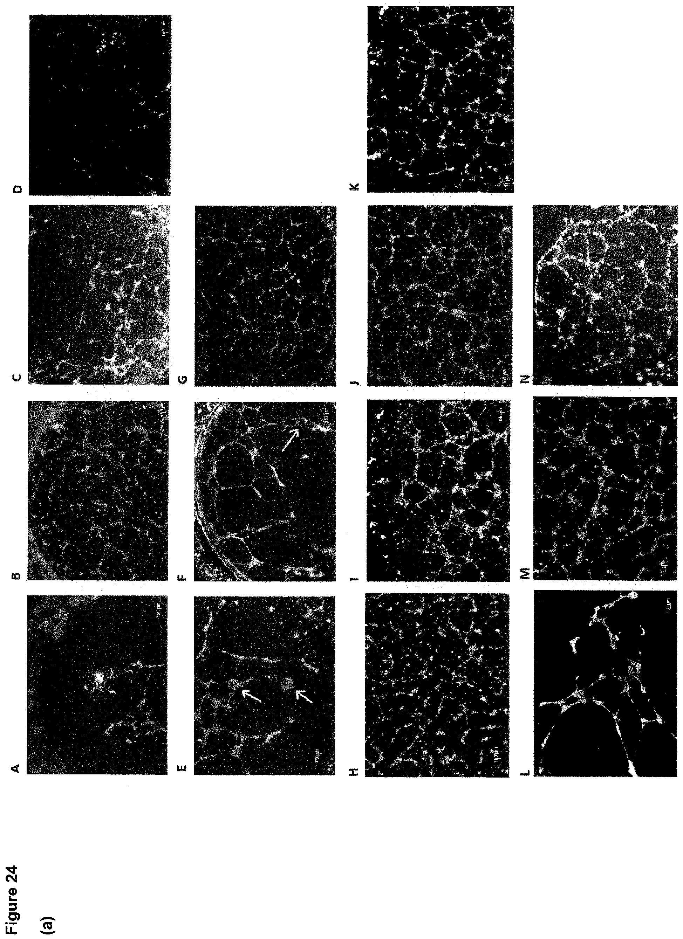

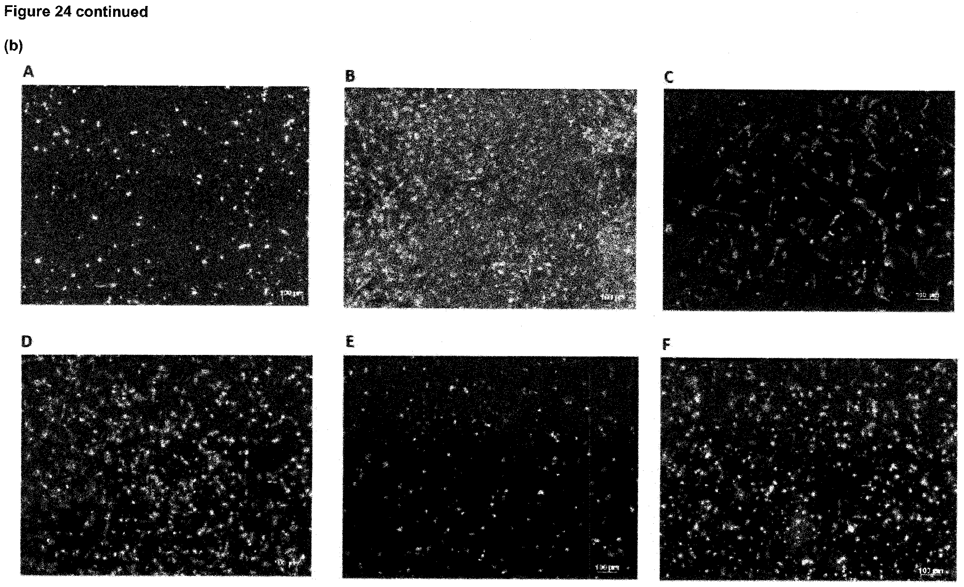

- **Fig.24**: Image panel showing the in vitro angiogenic response (tubule formation) seen when releasates from various gel and microbead carriers, loaded with PBC-derived factors, were added to HUVEC-seeded matrigel and incubated for 12hrs. In certain conditions, antibodies against specific factors were added to the PBC culture media during hypoxic culture; A:negative control medium, B:collagen gel carrier, C:collagen/fibrin carrier, D:fibrin carrier, E:collagen beads in collagen gel, F:pronectin beads in collagen gel, G: VEGF positive control, H: negative control, I: anti-PF4, J: anti-TSP-1, K:anti-VEGF, L:anti-PF4 + anti-TSP-1, M:anti-PF4 + anti-TSP-1 + anti-VEGF, N: collagen gel carrier without antibodies. Arrows in E and F show microbead carriers, in contact with endothelial cells/formed tubules. Bars=100µm. b) Image panel showing the endothelial cell invasion through a matrigel membrane induced in response to the releasates from gel carriers loaded with PBC-derived factors. A: negative control medium, B: collagen gel, C: collagen/fibrin gel, D: Fibrin gel, E:Hydrosorb gel, F: Hydrosorb/fibrin gel. Bars=100µm.

The Examples illustrate the invention:

### Example 1

In one setup (Fig. 1) cells (e.g. fibroblasts, stem cells, endothelial cells, smooth/skeletal/cardiac muscle cells, peripheral blood cells) can be seeded within a biomaterial sheet scaffold, comprising of natural polymer (e.g. collagen, fibrin) or synthetic polymer (e.g. PGA, PEG), or they can be used as a confluent cell-sheet (without exogenous matrix scaffold) (Kushida et al. 2000), which is wrapped around a mandrel support. Alternatively, (preferably autologous) split-thickness or full thickness skin graft(s) can be used as the cellular compartment. This (single-layer) tubular cell-seeded matrix sheet, cell-sheet or skin graft is cultured under physiological hypoxia (1-10% O₂, pO₂ 7.6-76 mmHg) in a controlled-oxygen incubator/gas chamber for 1 to 30 days, to upregulate production of angiogenic factors (e.g. VEGF, PLGF, FGF) by cells. Hypoxia-induced, cell produced angiogenic factors can be cuptured/trapped as they diffuse radially through an acellular matrix sheet/layer, which is positioned adjacent to (e.g. spiralled around) the core cell-seeded matrix sheet/cell-sheet. The spiral is sealed (e.g. with fibrin glue) at its two ends to prevent lateral diffusion of produced factors into culture medium. While the cellular compartment can comprise one or more cell-layers, use of a single cell-layer compartment is preferred, as it ensures that 1) produced factors diffuse directly into the acellular matrix without having first to diffuse though multiple cell-seeded layers, ensuring faster and maximal sampling of produced factors within the acellular matrix, and 2) all cells are exposed to the same O₂/nutrient levels (note; in a multilayer cell-seeded scaffold cellular O₂/nutrient consumption would produce surface-to-core O₂/nutrient gradients, therefore cells in the core would be exposed to lower O₂/nutrient levels compared to cells on the surface), which allows control of process in terms of cell viability rates and levels of factors produced. Since the concentration and ratio of trapped factors produced at a given O₂ tension will be determined by the acellular matrix density/porosity/pore size, use of a natural or biomimetic matrix (e.g. decellularised native dermis matrix, decellularised small intestinal submucosa, compressed collagen/fibrin hydrogel matrix) would ensure trapping of factors at physiological concentrations and ratios. Optionally, a nano-porous filter encloses the cell-seeded compartment, thus preventing radial movement of cells or bacteria into the acellular layer, while allowing free diffusion of factors into the acellular compartment.

Optionally, a combination of stress-stimuli can be applied simultaneously to the cell-seeded compartment within a bioreactor system. For example, mechanical loading (e.g. stretching) can be applied to cells (e.g. endothelial cells, vascular smooth muscle cells, cardiomyocytes), under hypoxic culture conditions. Since angiogenic factors (e.g. HIF1a, VEGF) are known to be regulated by both hypoxia and mechanical stimuli, such combined stress-stimulation could achieve a more physiological growth factor response, in certain cell types, in comparison to applying each stimulus independently. Furthermore, it is possible to apply a stress stimulus or a combination of stress stimuli to constructs with 2 or more different cell types in their cell-seeded compartment. For example, the cell-seeded compartment could comprise of an inner endothelial cell layer, a middle vascular smooth muscle cell layer, and an outer fibroblast layer, where the cells are simultaneously exposed to tensile loading and hypoxic stress, thus mimicking the natural stresses within a vascular wall under ischaemic conditions (Fig. 5). Other possible combinations of cell layers in the cell-seeded compartment include osteoblasts/chondrocytes (mimicking an osteo-chondral structure), neurons/glial cells (mimicking a nerve), myoblasts/tenocytes (mimicking a muscle-tendon interface).

In order to selectively load the acellular matrix with specific/preferred growth factors, it could be possible to incorporate within the filter layer(s) enclosing it, one or more types of growth factor receptors (e.g. recombinant receptors for VEGF, FGF, PLGF, IGF etc.) or antibodies against growth factors. Receptors or antibodies within the filter can selectively capture specific factors from the produced growth factor mixture, thus excluding them from the factors reaching the acellular matrix. By excluding selected factors it could be possible to selectively tune the mixture factor composition towards desired factors.

The acellular matrix (e.g. spiral) layer(s) can be removed/detached (e.g. unspiralled) from the cell-seeded compartment at different points during hypoxic culture and replaced by fresh layer(s) to enable continuous sampling of produced angiogenic factors at different time intervals (e.g. every 5 days) (Fig.1 setup 1). Time-specific angiogenic matrix fractions can be injected at suitable time intervals (matching the temporal profile of factor expression in vivo), in order to temporally reconstruct/replicate a physiological angiogenic cascade. For example, a preferred injection regime could be injecting 0-5 day, 5-10 day and 10-15 day hypoxia pre-conditioned matrix fractions at day 0, 5 and 10, respectively (i.e. the injection time interval corresponds to the time interval (5 days) between pre-conditioning groups).

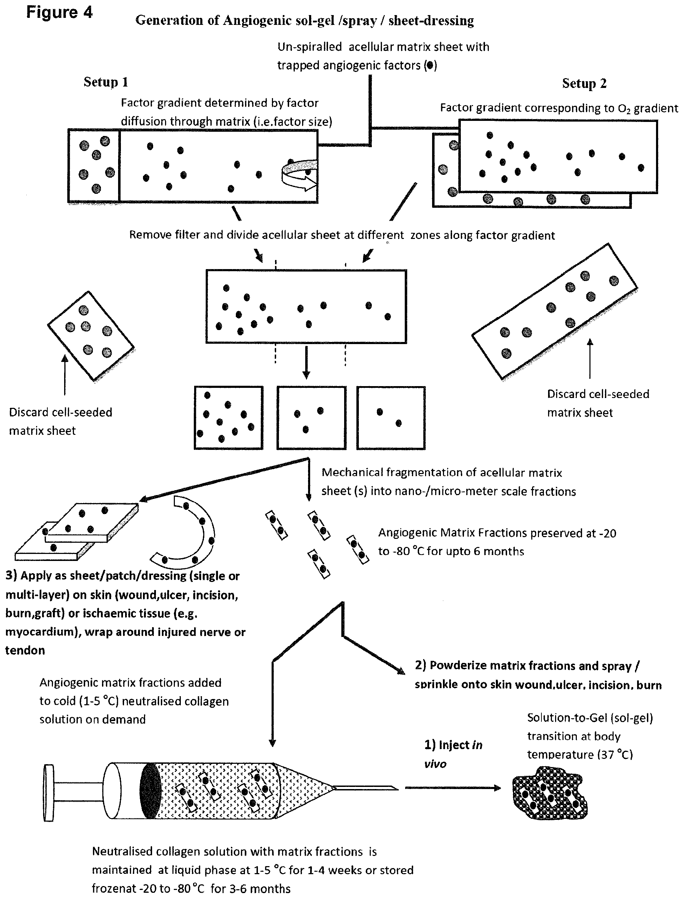

After culture, the acellular matrix (which carries the produced factors) can be optionally frozen or freeze-dried, before being mechanically fragmented into nano-/micro-scale 'angiogenic matrix fractions', which can then be preserved at low temperature (typically -20 to -80 °C) for up to 6 months (Fig. 4). On demand, 'angiogenic matrix fractions' can be incorporated/added into a low temperature (1-5 °C) thermo-responsive solution (e.g. neutralised collagen solution), which can then be stored at a liquid state at +1 to +5 °C for up to about 1 month, or stored frozen at -20 °C for longer periods (e.g. 3-6 months). The factors can be locally delivered into a tissue site by injection of the collagen solution, which at body temperature (37 °C) undergoes a solution-to-gel (sol-gel) transition to form a gel at the injection site (Fig. 4). Alternatively, angiogenic matrix fractions can be prepared in the form of a powder that can be sprayed or sprinkled onto a superficial skin area that requires vascularisation (e.g. wound, ulcer, skin graft, burn, abrasion, incision) (Fig. 4). The acellular matrix sheet loaded with angiogenic factors can also be used without fragmentation in the form of a temporary sheet/dressing/patch (single layer or multi-layer) that can be applied (e.g. sutured, simple fixation) onto a superficial skin area that requires vascularisation (e.g. wound, ulcer, skin graft, burn, abrasion, incision) or as a biodegradable patch applied during surgery onto an area of ischaemia (e.g. myocardium, skeletal muscle). Also, an acellular matrix sheet can be wrapped around lesioned nerves as a factor-delivery sheath, or can act directly as a gap-bridging guidance conduit that delivers factors, to aid nerve repair/regeneration. A similar concept can be employed in injured tendons, where a factor-loaded acellular matrix sheet can be wrapped around the tendon suture site, tendon-bone or muscle-tendon interface to aid tendon repair/healing. Natural diffusion of locally delivered factors into the tissue will result in development of spatial gradients that will directionally chemo-attract endothelial cells towards the target site (e.g. infarcted area, implant, wound etc.) to promote angiogenesis and ultimately vascularisation of the desired area.

Acellular matrix fractions with growth factors produced by different cell types in response to various forms of stress-stimulation (e.g. hypoxic stress, mechanical loading or electrical stimulation) can be delivered to a target tissue (through an injectable gel, spray or as sheet/patch) in isolation or in combination. For example, acellular matrix fractions with bone-related growth factors produced by compressive loading of osteoblasts or bone marrow stem cells can be combined with cartilage-related growth factors produced by compressive loading of chondrocytes, and delivered together through an injectable sol-gel in order to promote repair/regeneration of osteo-chondral defects (Fig. 5; Table 1). Similarly, acellular matrix fractions with angiogenic growth factors produced by exposing cells (e.g. fibroblasts, stem cells) to hypoxic stress can be co-delivered with acellular matrix fractions with angiogenic growth factors produced by exposing cells of vascular origin (e.g. microvascular endothelial cells, vascular smooth muscle cells) to stretching, through an injectable sol-gel, spray or as multi-layer matrix sheets that are directly applied (e.g. sutured, simple adherence) to a skin defect (e.g. wound, ulcer, incision, burn) or graft to aid its vascularisation and healing (Fig. 4, Fig. 5; Table1). In another example, acellular matrix fractions with angiogenic growth factors produced by exposing cells (e.g. fibroblasts, stem cells) to hypoxic stress can be co-delivered with acellular matrix fractions with neurotrophic growth factors produced by electrical stimulation of cells (e.g. neurons, glial cells, neural stem cells) though an injectable sol-gel or matrix sheet (that can be wrapped around lesioned nerves as factor-delivery sheath or guidance conduit) to aid nerve repair/regeneration and provide neuroprotection to injured areas (Fig. 5; Table1)

### Example 2

In a second setup (Fig. 2), a 3D spiral can be constructed that comprises alternating cell-seeded matrix sheet/cell-sheet layers and acellular matrix sheet layers. This could be produced by layering an acellular-matrix sheet on top of a cell-seeded matrix sheet/cell-sheet (or layering them the other way round) and spiralling both sheets together along their short (or long) axis. Optionally, a nano-porous filter encloses the acellular layer (Fig. 3), thus preventing radial movement of cells or bacteria from the cellular compartment into the acellular layer, while allowing free diffusion of produced factors. The spiral is sealed (e.g. with fibrin glue) at its two ends to prevent lateral diffusion of produced factors into medium and lateral diffusion of oxygen between layers.

Metabolic activity of the mammalian cells (which may be accompanied by cell division and increasing cell density), results in consumption of diffusible factors, such as oxygen and nutrients (e.g. glucose) and produces waste metabolites. As oxygen diffuses radially through the spiral layers, the cellular consumption of oxygen (cellular respiration) will produce a radial gradient of O₂, such that cells at different layers of the spiral will be exposed to different levels of O₂ tension (cells in the construct core will be exposed to lower O₂ than cells in surface) (Cheema et al. 2008; Cheema et al. 2009;Radisic et al. 2006). Since angiogenic factor upregulation depends on O₂ tension (higher factor production at lower O₂ tensions) (Cheema et al. 2008), cells in different layers would produce different factor levels (core cells, which are exposed to lower O₂ tension, would produce higher factor levels than surface cells). As factors produced by cells diffuse to the adjacent acellular matrix layer (s), this pattern of differential factor expression would be replicated within the acellular matrix sheet. In this way, O₂ tension-specific 'angiogenic matrix fractions' can be produced with unique angiogenic factor composition/concentration. Since cells in different tissues are exposed to different O₂ tensions (for example, while respiratory epithelium and outer skin layers are exposed to atmospheric levels of O₂ (21%; 160mmHg), physiological hypoxia (1 - 10% O₂; 7.6-76mmHg) is the most common natural environment for most mammalian tissues) (Okazaki and Maltepe 2006), such O₂ tension-specific 'angiogenic matrix fractions' could be injected in different tissue types, and/or at different time points within a tissue, to more accurately recapitulate the native angiogenic response.

### Example 3

A system is described for exposing peripheral blood cells to hypoxia and capturing produced factors in order to deliver them into the same, blood donor, patient (Fig.3B). Autologous blood, drawn from a peripheral vein, is added to a chamber that contains serum-free cell-growth medium plus glucose, and optionally an anticoagulant (e.g. EDTA) (in certain setups it might be preferred that blood clotting occurs). In preferred embodiments, blood can be centrifuged so that the plasma is removed, and all blood cell components (erythrocytes, leukocytes and platelets) or the buffy coat (leukocytes and platelets) are added to the first chamber. In cases where plasma is removed, the addition of anticoagulant is not necessary. The chamber's port is sealed air-tight after addition of blood so that cell O₂ consumption generates physiological hypoxia within the chamber. The rate of O₂ tension drop and final level of hypoxia reached within the chamber can be controlled by adjusting the volume of blood added (i.e. cell number) and/or the chamber volume (i.e. volume of residual air left within chamber after addition of blood). Hypoxia can also be artificially induced within the chamber by addition of a chemical inducer of hypoxia (e.g. cobalt chloride), but since it is difficult to prevent leakage of the chemical into chamber 2, this is better avoided in order to prevent potentially toxic effects. The chamber is incubated at 37°C, so that blood cells attach on the scaffold layer at the bottom of the chamber, and begin to secret hypoxia-induced factors. The cell-scaffold could be composed from a group of synthetic polymers (e.g. polyglycolic acid, polylactic acid, polycaprolactone) or natural proteins (e.g. fibrin), preferentially avoiding collagen-based biomaterials, as these will rapidly induce blood clotting within the cell-scaffold. Collagenous cell-scaffold can be used in cases where the plasma is removed by centrifugation. Angiogenic factors that could be produced in response to hypoxic exposure could include, but not limited to, VEGF(Burke et al. 2003;Niu et al. 2009;Panutsopulos et al. 2003), PDGF(Pang et al. 2008), bFGF(Panutsopulos et al. 2003), IL-8(Niu et al. 2009). Produced cellular factors diffuse through a nano-porous filter, positioned under the cell-scaffold, into a second chamber attached onto the blood-containing chamber. The nano-porous filter prevents migration of blood cells into the second chamber. Diffusing factors are captured by a multilayer nano-/micro-porous matrix (this can be composed of natural polymer (e.g. collagen, fibrin) or synthetic polymer) within the second chamber (Fig.3 B1). After completion of incubation, the second chamber can be detached, any contained medium/blood plasma expelled, and moved to the bedside, where it can provide a source of angiogenic factor-loaded matrix sheets that can be applied as a temporary/biodegradable dressing on wounds (e.g. burns, ulcers, traumatic wounds), ischaemic tissue (e.g. due to peripheral artery disease) or newly grafted tissue (e.g. skin graft, local/free flap) to aid angiogenesis. In a modification of this design, the matrix for capturing cell-produced factors comprises a network of nano-/micro-fibers or nano-/microparticles (Fig. 3 B2). After completion of incubation, the second chamber is detached from the first chamber, and contained medium/blood plasma is expelled before attaching the second chamber to a syringe (3^{rd} chamber) containing cold (e.g. +1 to +5°C) sol-gel (e.g. neutralised collagen). The joined chambers are shaken to mix the factor-loaded fibers/particles with the sol-gel, resulting in dissociation of the fiber-/particle-network. The solution can then be injected into a target ischaemic tissue, and gels within the body at the site of injection. Note that in addition to the vertically-orientated, two-chamber system described here, capturing of factors produced by peripheral blood cells could also be carried out using a radial configuration, i.e. central cell-scaffold and peripheral acellular compartment, as described in Example 1; in this case, autologous blood could be injected into the hollow, porous mandrel that supports the central cell-scaffold (Fig.1), so that blood cells move though the mandrel pores and attach onto the scaffold.

Since this system utilises patient autologous blood, under standard sterile conditions, it can be directly applied in the clinic, without prior regulatory body (e.g. FDA) approval. All parts of the system can be manufactured from already approved, clinically used materials while incubation can be carried out in every standard laboratory facility (use of chemical hypoxiainducer avoids the need for expensive oxygen incubator).

Another variant of a device according to the invention is shown in Figure 12. This device may be operated as described in the caption of Figure 12.

### Methods

### Collagen solution-to-gel experiments

Acid soluble type I bovine collagen solutions (2 or 4mg/ml) were neutralised with drop-wise addition of alkali (NaOH 1 M). Typically total collagen solution volume was 5ml, comprising 4ml collagen, 0.5ml 10xMEM (added as indicator) and 0.5ml NaOH. After neutralisation, solutions were left to gel within a 37 °C incubator. Solutions were either allowed to set directly after neutralisation, or stored at +5°C for 3 weeks or frozen at -20 °C for 1 h or 3 weeks, before setting at 37 °C. Neutralised collagen solutions were examined every 4 min with a phase contrast microscope to determine gelling times. Gelling time was defined as the minimum time required for observation of a homogeneous, interconnected network of fiber clusters in at least 3 visual fields (x10) within the same focal plane. Four collagen solutions were tested for each concentration (n=4), statistical significance tested with t-test or ANOVA, p<0.05.

### Quantitative analysis of hypoxia-induced angiogenic factors

Acellular compressed collagen matrix (method of collagen hydrogel plastic compression is described by Brown et al. (Brown et al. 2005)) was wrapped around a 3D collagen spiral construct seeded with 2×10⁶ Rabbit Dermal Fibroblasts, and incubated at 5% O₂ in medium (DMEM+10% FCS) for 2 weeks. Following this incubation period, acellular matrix was unwrapped, mechanically fragmented into micro-meter scale fractions by mechanical dismembranation and frozen at -20°C. Fractions were added to 500µl medium (DMEM+10% FCS; note FCS contains BSA which is important for factor stability) and stored in vials at - 20 °C for 4 weeks or 3 months. Fractions were then thawed and added into a 5ml collagen solution (4mg/ml collagen density, temperature: +5 °C) which was pipetted into 6-well plates. Collagen solutions containing matrix fractions, and control collagen solutions without any matrix fractions were allowed to set into gels within a 37°C incubator for 30min. Once gels were set, 1ml of medium (DMEM+10% FCS) was added into each well and incubated overnight, to allow diffusion of factors from collagen gels into media. Media were then sampled from each well and analysed with an Angiogenesis Proteome Profiler assay (R&D, USA), according to manufacturer's instructions. Image analysis of developed x-ray films was carried out with an Image J (NIH,USA).

### Qualitative analysis of trapped angiogenic factors within matrix fractions

Collagen matrix fractions were obtained from the cell-seeded compartment and acellular matrix wrap of 2 week hypoxia pre-conditioned 3D collagen spiral constructs (see above). Cellular and acellular collagen matrix fractions with angiogenic factors were stored for 3 months at -20°C. Fractions were then thawed and added into a 5ml collagen solution (4mg/ml collagen density) which was pipetted into 6-well plates. Collagen solutions containing matrix fractions were allowed to set into gels within a 37°C incubator for 30min. Once gels were set, they were incubated with primary antibody (rabbit anti-human VEGF or HIF1a) overnight at 4°C, followed by secondary antibody (goat anti-rabbit Alexa red or green, respectively) and DAPI (blue) for nuclear staining at 37°C for 1 h. Immunofluorescently stained samples were viewed with a fluorescent microscope (Olympus) at x10 magnification, with 4 fields viewed per sample (4 samples per condition). Fields were also viewed with a phase contrast microscope for overlap analysis.

### Temporal profile of Hypoxia-induced Factors trapped in acellular collagen fragments

A cell-seeded type I collagen gel (8 million rabbit dermal fibroblasts: RDFs) was set into a 3.3 × 2.2 mould (4ml gel volume). Following setting and plastic compression the gel was cut into 4 strips (7mm wide and 2.2 cm long each). Each strip was placed onto a compressed acellular collagen gel (originally 5 ml solution set in the same 3.3 × 2.2 cm mould - following 30 minutes setting the gel was compressed for 5 minutes). The cellular strip was placed at one end of the acellular gel, then the acellular gel was spiralled around the cellular gel. The acellular gel was spiralled 6 times, i.e. there were 6 layers from the spiral surface to its core. The composite spiral (inner cellular compartment and outer acellular compartment) was cultured in 4 ml media (10% FCS DMEM) within a controlled oxygen incubator (5% O₂) for a set period: at 2,4,8,12,16 and 20 days of culture the spiral was removed from culture, the acellular collagen wrap was unspiralled from the cellular compartment and a fresh aellular collagen gel was spiraled around the cellular strip which was then returned to culture (Fig.10a). Media were changed with every acellular wrap change (old media were stored at - 20 °C). Acellular collagen wraps with trapped factors were stored at -20 °C. At the end of the experiment all gels (acellular and cellular gels) were micro-dismembranated and resuspended in 500ul of 10% FCS DMEM media, which was stored for 4 weeks at -20°C. For ELISAs, 1ml PBS-2 %FCS was added to each 500microl solution of collagen fragments to give a solution of 1.5ml. This solution was incubated for 48 hrs at 5 °C to allow diffusion of trapped factors from the collagen fragments into the solution. ELISAs for VEGF, FGF, PLGF and IL-8 were then carried out by sampling from these solutions.

### In vitro Matrigel assay to assess biological activity of freeze-stored hypoxia-induced factors

Matrix fractions containing hypoxia-induced factors, obtained from 2 week cultures, were frozen at -20 °C for 3 months. They were then thawed and added to 5 °C neutralised type I collagen solution (4 mg/ml collagen concentration, 5 ml volume), which was pippeted into 6-well plates and allowed to set into gel at 37 °C for 30 min. Gels were incubated with 1ml media (DMEM) overnight to allow diffusion of factors into media. The biological activity of hypoxia-induced factors was assessed by the formation of capillary-like networking structures by endothelial cells seeded on Matrigel. Growth factor-depleted Matrigel (BD Biosciences, Germany) was thawed at 4 °C overnight. Using pre-cooled pipette tips and plates, the Matrigel was then distributed in 24-well plates (1ml/well) or 12-well plates (1.5ml/well) and allowed to solidify at 37 °C for at least 1 h. HUVECs (purchased from Cell Systems, Germany) were cultivated in VascuLife Basal Medium (Cell Systems, Germany) in 5% CO₂ at 37 °C, and used at passage 2. HUVECS were seeded at low density in 24-well plates (20 000 cells/well) and at high density in 12-well plates (150 000 cells/well), and left to attach for 4hrs before adding 0.5ml medium M-199 supplemented with 10 %FCS. Factor-containing media or control medium (DMEM) were added (1ml per 24-well plate, and 1 or 2ml per 12-well plate) to HUVEC-seeded Matrigel (3 samples per condition). Plates were incubated at 37 °C for 48 hrs. Tube formation was observed with phase contrast microscopy, and digital pictures were captured using an Olympus digital camera.

### Results

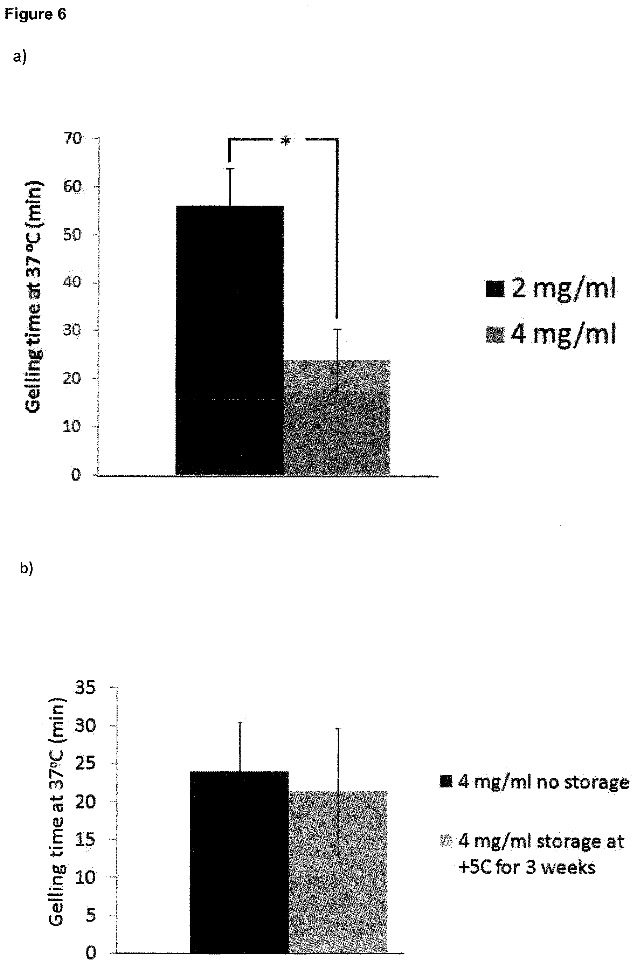

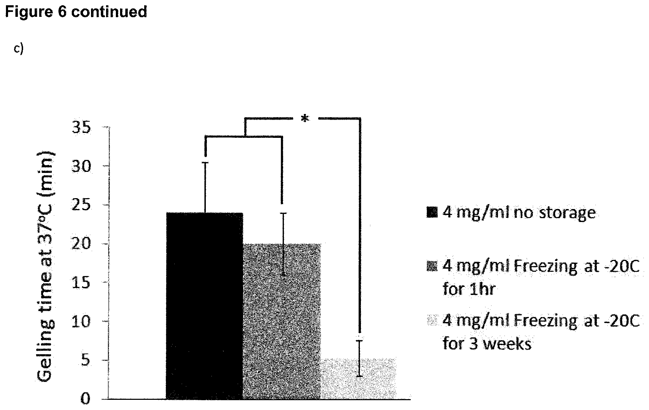

There was a direct correlation between collagen solution concentration and gelling time, with 4mg/ml solutions gelling twice as fast as 2mg/ml (Fig.6 a). Therefore collagen concentration was found to be good controller of gelling time. While there was no significant difference in terms of gelling time between collagen solutions (4mg/ml) set immediately after neutralisation or stored at +5 °C for 3 weeks or -20 °C for 1 h before setting, gelling time of solutions stored frozen at -20 °C for 3 weeks was significantly faster compared to that of solutions stored at -20 °C for 1 hr or not stored (n=4, *p<0.05) (Fig. 6b,c). Therefore, neutralised collagen solution maintains its thermo-responsive property after prolonged periods of cold or freeze storage. Since cellular factors have a comparable stability at low temperature (see below), the whole preparation (collagen solution with factor-loaded matrix fractions) could be stored for significant periods (e.g. up to 6 months) before use.

Branched structures of fibers first appeared at 8min and 40 min for solutions of concentration 4 and 2 mg/ml, respectively (Fig. 7, arrowed). For collagen solution of concentration 4mg/ml more clusters of filaments appear at various sites of polymerisation between 8 min (B) and 12 min (C), while the branched filament bundles start to intersect at 16 min (D). After this time the structure of the network does not change rapidly any more, such that no significant difference is observed between images at 16 min (D) and 20 min (E). Collagen solutions (4mg/ml) that were stored at +5 °C following neutralisation, for 3 weeks, exhibited normal gelling at 37 °C (K,L). In contrast, collagen solutions (4mg/ml) that were frozen at -20 °C following neutralisation, and stored for 3 weeks, showed accelerated gelling at 37 °C (M-O), compared to collagen solutions that were not frozen (A-E) or frozen at -20 °C for 1 hr following neutralisation (I-J). In this case, clusters of fibers (clumps) appeared at an early stage (4 min, M), while individual fibers were difficult to identify (compared with B and I, where individual fibers are visible). As gelling progressed, larger fibre-cluster structures could be seen (N,O; arrowed) that did not exhibit significant branching. This suggests that while freezing a neutralised collagen solution for a long period (weeks) does not inhibit its sol-gel property, it might have an effect on the polymerisation process at the fibrilar level.

Analysis of the angiogenic profile of 1 month or 3 month-stored (at -20 °C) matrix fractions indicated that a range of angiogenic factors were trapped within 2 week hypoxia-preconditioned matrix fractions. This indicated that a dense biomimetic matrix, such as plastic compressed collagen, has the ability to retain cellular factors (of different size) diffusing through it. Detection of these factors within media indicated successful factor release from dense collagen matrix fractions, through the collagen gel used as delivery vehicle (Fig.8). Importantly, factors were found to be stable after 3 months of storage at -20°C, with no significant difference in factor relative (sample/control) amount between sampes stored for 1 or 3 months.

HIF-1a (green fluorescence) was present in cell-seeded fractions, while VEGF (red fluorescence) was visualized within cell-seeded and acellular matrix fractions, obtained from the cellular compartment and acellular wrap, respectively, of 3D collagen constructs that were pre-conditioned under hypoxia for 2 weeks (Fig. 9).

In order to dissect out the temporal profile of angiogenic factor expression in this system, the acellular collagen compartment (peripheral wrap) containing trapped factors was removed and replaced by a fresh one at set time intervals, thus allowing continuous sampling of hypoxia-induced cellular factors over 20 days (see schematic Fig. 10). Figure 10a shows that production of factors was not linear over time. Indeed, VEGF and IL-8 expression initially decreased from day 2 to day 4, and thereafter increased, peaking at day 8. From that point on expression showed a gradual decline, falling below initial levels by day 20. FGF expression, on the other hand, showed a small initial decrease from day 2 to 4 (similar to VEGF and IL-8), but thereafter remained relatively stable. PLGF was also present within acellular matrix, reaching a concentration of 9.5±1.19 pg/ml at day 2, but levels were too low to be measurable after this time point. However, this apparent down-regulation in PLGF expression appeared to follow the same pattern as for VEGF and IL-8. There was a higher accumulation of FGF in the cellular compartment, relative to the acellular compartment, compared to VEGF and IL-8 over the 20 day culture period (Fig. 10b), which was likely the result of FGF binding to FGF receptors on fibroblasts within the cellular compartment. A similar difference (-15 fold) in FGF and VEGF/IL-8 concentration gradient was also observed between the acellular compartment and medium (16 to 20 days) (Fig. 10c). Nonetheless, retention-release ratios (ratio of factor concentration in acellular compartment to that in medium) greater than 1, for all factors tested, confirmed the ability of the nano-porous collagen matrix to efficiently trap hypoxia-induced cellular factors diffusing through it.

Since this system targets the potential of 'on-demand' delivery of hypoxia-induced angiogenic factors, the ability to store factors prior use is important. Having shown that 3-month freeze storage does not interfere with factor stability, we moved on to test the ability of 3-month freeze-stored factors to induce tubule formation in vitro, as a test of factor biological activity. Factors were delivered from 2 week hypoxic culture-matrix fractions into media (by diffusion through type I collagen sol-gel), which were then added onto Matrigel seeded with endothelial cells (HUVECs). Figure 11 shows that in low cell density cultures (~10×10³ cells/cm²), where cell-cell contact was minimal, addition of factor-containing media (VEGF concentration; 2±0.3ng/ml) resulted in endothelial cells with an elongated/sprouting morphology, within 48hrs, which is the *in vitro* counterpart of in vivo angiogenic endothelial cells. In contrast, in cultures where control media (DMEM) were added, endothelial cells maintained a cuboidal/cobblestone appearance, reminiscent of resting endothelial cells lining vessel lumens. In high cell density cultures (-40×10³ cells/cm²), capillary-like structure formation was evident at 48hrs (tubule formation was initiated by 24hrs, data not shown) when factor-containing media were used, compared with minimal tubule formation observed with control media (Fig. 11). These findings confirm that factors loaded on matrix fractions maintain their biological activity for at least 3 months of freeze storage.

### Example 4

### Rapid screening of angiogenic factors produced by PBCs under normoxia and hypoxia

Peripheral blood (10ml) was collected from a 23yr old female healthy (BMI=21Kg/m²), non-smoking subject. The buffy coat was isolated by centrifuging the blood in EDTA-Vacutainer tubes (BD, Germany) at 4000rpm / 4°C for 10min, and mixed with 12ml of serum-free (SF) media (AIM V, Invitrogen, Germany). 0.5ml of peripheral blood cell (PBC)/SF-medium mixture was added to PET-, type I collagen-coated PET- or fibrin (Tissucol Duo S 1ml Immuno, Baxter, Germany)-coated PET 1µm pore membrane cell culture inserts (BD, Germany), before adding 2ml SF medium in each insert. Inserts were then placed in 6-well plates containing 3ml SF medium per well. Cell culture was carried under hypoxia (3% O₂) or normoxia within a 37°C/5% CO₂ incubator for 10 days. Media for each condition were then sampled from wells and analysed with Angiogenesis Proteome Profiler array (R&D, USA), according to manufacturer's instructions. Utilization of the buffy coat, rather than whole blood, ensured precise control of oxygen levels in culture, as well as detection of only the factors generated by cultured PBCs, excluding any factors already present in plasma. Quantification of relative (test/control medium) factor levels was carried out by image analysis of array signals (quantification of mean spot pixel densities) from scanned x-ray film images (3 and 4 min exposures) using an imaging software (Image J, NIH, USA). Three samples were tested per condition.

### Quantification of angiogenic factor protein levels under normoxic and hypoxic culture

Peripheral blood (5ml) was collected into EDTA-Vacutainer tubes from 19 non-smoking healthy subjects (9 males; age=38.3±6yrs, BMI=26.3±1.3Kg/m², and 10 females; age=30.7±2.2yrs, BMI=22.5±1Kg/m²). All subjects provided consent as approved by the ethics committee of the Heinrich Heine University, Dusseldorf, Germany. Immediately after blood collection, 5ml of medium (DMEM supplemented with 20%FCS, 20%DMSO) was added to each tube so that the final blood-medium mixture (10ml) contained 10%FCS and 10%DMSO. Tubes were stored at -80°C during blood collection (4 days) and then allowed to thaw in a 37°C waterbath for 30min. Each 10ml blood-medium solution was then mixed with 10ml SF medium, before being aliquoted into 6-well plates (5ml/well).Wells were placed in a 37°C, 5%CO₂ incubator for 4hrs to allow blood cells to settle at the bottom of the well, then DMSO in media was further diluted by step-wise medium removal and substitution with fresh SF medium. Final medium volume per well was 4ml containing <0.5%DMSO/0.5%FCS. One set of wells was then placed in a normoxic incubator, while another in a hypoxic incubator (3% O₂), and cultured for 7 days, after which media were sampled from wells. ELISAs for VEGF, Angiogenin (ANG), and Thrombospondin-1 (TSP-1) (all from R&D, USA) were carried out on samples obtained from culture supernatants, according to manufacturer's instructions. Two samples from each subject were tested per factor for each condition (normoxia/hypoxia).

### Assessment of the effect of temperature on factor expression

The buffy coat was isolated from 10ml peripheral blood, collected from a 23yr old female healthy, non-smoking subject, and mixed with 12ml SF media. 1ml of PBC/SF-medium mixture was added to type I collagen-coated polystyrene 6-wells (BD BioCoat, Europe), before adding 2ml SF per well. Cell culture was carried out at 37°C under hypoxia (3% O₂) or normoxia, and at 20°C under normoxia for 8 days, after which media were sampled from wells. ELISA for VEGF (R&D, USA) was carried out on samples obtained from culture supernatants, according to manufacturer's instructions. Three samples were tested for each condition.

### Assessment of the effect of temperature, oxygen tension and scaffold material on cell viability

0.5ml of PBC/SF-medium mixture, prepared as described above, was added to PET-, type I collagen-coated PET-, or fibrin-coated PET 1µm pore membrane culture inserts, before adding 1.5ml SF medium per insert. Inserts were then placed in 6-well plates containing 2ml SF medium per well. Cell culture was carried under hypoxia (3% O₂) for 10 days. In another setup, 1ml of PBC/SF-medium mixture was added to PET 1µm pore membrane culture inserts, before adding 1ml SF medium per insert, and placing inserts in 6-well plates containing 2ml SF medium per well. Cell culture was carried out at 37°C under hypoxia (3% O₂) or normoxia, and at 20°C under normoxia for 8 days. At the end of the culture period, media (cell-free) were harvested from wells and centrifuged for 5min at 300 × g. Triton-X-100 was added to media in inserts to a final concentration of 1 % and allowed to react for 2hrs at 37°C for complete cell lysis (max LDH release). Lactate Dehydrogenase assay reaction mixture was prepared according to manufacturer's (Roche, Germany) instructions and mixed with 100µl of media from wells or inserts in 96-well plates. Plates were incubated at 25°C for 30 min in the dark, before measuring absorbance at a wavelength of 492nm. SF-medium was tested as background control, and values were subtracted from all data points. Percentage cytotoxicity was calculated from the ratio of well/insert values. Three samples were tested for each condition.

### Characterization of cell-scaffold material properties

### Effect of scaffold material composition on factor expression

0.5ml of PBC/SF-medium mixture, prepared as described above, was added to PET-, type I collagen-coated PET-, or fibrin-coated PET 1µm pore membrane culture inserts, before adding 1.5ml SF medium per insert. Inserts were then placed in 6-well plates containing 2ml SF medium per well. Cell culture was carried out under hypoxia (3% O₂) for 8 or 10 days. Media for each condition were then sampled from wells and analysed by ELISA for VEGF, ANG and TSP-1 (R&D, USA) according to manufacturer's instructions. Three samples were tested per factor per condition.

### Effect of scaffold matrix stiffness on factor expression

1ml of PBC/SF-medium mixture, prepared as described above, was added to 6-well plates as follows: Group I; PBC suspension added to wells with a polystyrene surface, Group II; PBC suspension added to wells containing 5ml type I bovine collagen gels (c: 4mg/ml, composition of 80 % collagen (Biochrom, Germany), 10% 0,5M NaOH and 10% 10xMEM with phenol red as pH-indicator; prepared by drop-wise neutralisation of acid-soluble collagen solution with alkali), Group III; PBC suspension added to wells layered by a sheet of plastic compressed type I bovine collagen. Compression of collagen gels was carried out on a blotting paper support under fixed mechanical loading for 5min, and resulted in an increase in gel Young's modulus from ~50kPa to ~2000kPa (Hadjipanayi, E., Mudera, V., and Brown, R. A., Close dependence of fibroblast proliferation on collagen scaffold matrix stiffness, J.Tissue Eng Regen.Med. 3 (2009) 77-84). After adding cells, 4ml SF medium were added per well. Cell culture was carried out under normoxia within a 37°C/5% CO₂ incubator for 10 days, after which media were sampled from wells and tested for VEGF by ELISA. Three samples were tested per condition.

### Assessment of the optimal spatial organization of device elements

1ml of PBC/SF-medium mixture, prepared as described above, was added to 6-well plates or culture insert as follows: Group I; PBC suspension added to 6-well plates, after which a culture insert with a type I collagen-coated PET 1µm pore membrane (serving as filter) was placed in each plate (filter not in contact with cells), Group II; PBC suspension added to type I collagen-coated PET 1µm pore membrane culture inserts, which were then placed in 6-well plates (filter in contact with cells). Group III; PBC suspension added to PET 1µm pore membrane culture inserts, which were then placed in 6-well plates (filter in contact with cells). After adding cells, 2ml SF-medium was added per well and per insert, respectively. Cell culture was carried under normoxia within a 37°C/5% CO₂ incubator for 10 days, after which media above the cell layer (supernatant) and media passing through the filter-membrane (diffusate) were sampled and tested for VEGF and IL-8 by ELISA (R&D, USA) according to manufacturer's instructions. Three samples were tested per factor per condition.

### Statistical Analysis

For each experimental condition n ≥ 3 was used. Data is expressed as mean ± standard deviation or mean ± standard error, as noted. Statistical analysis was carried out using Student's independent t-test where a maximum of 2 groups was compared or oneway ANOVA accompanied with multiple comparison tests for analysis of more than 2 groups, using SPSS 14 software. The probability of a type one error was set to 5% (α = 0.05), unless noted otherwise.

### Results

### Regulation of factor expression by oxygen tension

Oxygen tension was the first environmental factor investigated as a means of controlling angiogenic factor production by peripheral blood cells (PBCs). Proteome profiler array was first used to rapidly screen the expression profile of angiogenesis-related proteins released in culture supernatants when PBCs were seeded on type I collagen-coated PET membranes and cultured under normoxia or hypoxia (3%O₂) for 10 days. As shown in figure 1a a range of factor proteins were present in the media of both normoxic and hypoxic cultures, as compared with control serum-free media, including pro-angiogenic (VEGF,Angiogenin, IL-8), anti-angiogenic (Thrombospondin-1, Platelet Factor-4) and matrix remodeling (MMP-8, MMP-9, TIMP-1) factors.

VEGF, Thrombospondin-1 (TSP-1) and Angiogenin (ANG) were the three factors selected for quantitative analysis by ELISA. Peripheral blood was collected from 19 healthy, non-smoking subjects and cultured for 7 days under normoxia or hypoxia. Figure 2 shows that VEGF and TSP-1 expression were significantly up-regulated in hypoxic cultures relative to normoxic cultures (hypoxic vs. normoxic population mean supernatant concentration values for VEGF and TSP-1 were 50.5±5.2pg/ml vs. 41.9±6.2pg/ml (p<0.05) and 226.3±13.4ng/ml vs. 118.7±7.3ng/ml (p<0.001 ), respectively), while ANG expression was down-regulated under hypoxia (hypoxic vs. normoxic ANG supernatant concentration was 3192.5±108pg/ml vs. 3938±160.8pg/ml (p<0.001)). For all factors tested, no significant difference was found in factor expression between male and female subjects.

### Regulation of factor expression by temperature

Ambient temperature was the second environmental factor tested. In particular, we investigated the effect of culturing PBCs at room temperature (RT; 20°C) on VEGF expression. After 8 days culture, VEGF protein concentration in RT normoxic culture supernatants was significantly lower (~50% reduction, p<0.05) when compared to media obtained from normoxic, as well as hypoxic cultures at 37°C (15). Analysis of cell viability at 8 days by LDH assay showed that neither culturing PBCs under hypoxia, nor lowering the culture temperature to 20°C had a significant cytotoxic effect (Fig.16A).

### Effect of cell-scaffold material properties on factor expression

The two parameters tested with regards to the cell-scaffold were material composition and mechanical properties, in this case matrix stiffness. As when examining the effect of oxygen tension on factor expression, we first screened the profile of angiogenesis-related proteins produced by PBCs cultured on three different scaffolds under hypoxia; naked PET membrane, type I collagen-coated PET membrane and fibrin-coated PET membrane. In this setup utilization of the same underlying substrate (i.e. PET membrane), ensured that matrix stiffness felt by cells was comparable in all three scaffolds tested (Buxboim, A., Rajagopal, K., Brown, A. E., and Discher, D. E., How deeply cells feel: methods for thin gels, J Phys.Condens.Matter 22 (2010) 194116). As shown in Figure 13B, a range of factor proteins could be detected in 10 day culture supernatants using this assay. PBCs cultured on collagen-coated PET membrane appeared to produce the highest level of the matrix remodeling proteins MMP-8 and MMP-9. Interestingly, this higher level of expression was observed in the background of greater total cell death on collagen-coated PET membrane compared to naked PET membrane and fibrin-coated PET membrane (p<0.05) (Fig.16B). In contrast, quantitative analysis of VEGF expression using ELISA showed that the highest level was produced by PBCs cultured on fibrin-coated PET membrane, followed by collagen-coated PET membrane and naked PET membrane (p<0.05) (Fig. 17A). Similarly, ANG expression was significantly higher when PBCs were cultured on collagen-coated PET membranes than on naked PET membranes (p<0.05) (Fig. 17B). However, no significant difference in TSP-1 expression was observed between the two scaffold supports (Fig. 17B).

In order to test the effect of matrix stiffness on factor expression, we seeded PBCs on compliant and stiff collagen hydrogel matrices (Young's moduli of ~50kPa and ~2000kPa, respectively). Importantly, the increase in matrix stiffness was achieved here by irreversible removal of interstitial fluid from the matrix through plastic compression, rather than by chemical modification, thus ensuring that material composition remained the same in both conditions (Hadjipanayi, E., Mudera, V., and Brown, R. A., Close dependence of fibroblast proliferation on collagen scaffold matrix stiffness, J.Tissue Eng Regen.Med. 3 (2009) 77-84). Figure 17C shows that VEGF protein expression was significantly higher (~4-fold increase, p<0.05) in stiff versus compliant matrices. According to our previous results comparing collagen-coated PET vs. naked PET membranes (Fig. 17A), seeding PBCs on collagen gel matrices yielded higher VEGF levels than seeding them on a polystyrene surface, although this difference was only significant for stiff matrices (p<0.05) (Fig. 17C).

### Correlation of cell-scaffold/filter spatial organization with factor diffusion through filter

We last investigated the effect of cell-scaffold position, relative to that of the filter, on the ability of produced factors to diffuse through the filter. Two possible spatial organizations were tested; in the first, cell-scaffold and filter were combined, by culturing PBCs on PET- or collagen-coated PET 1µm pore filter-membrane, i.e. cells were in contact with the filter. In the second, cell-scaffold and filter were dissociated, by culturing PBCs on a polystyrene surface, and suspending a collagen-coated PET 1µm pore filter-membrane above the cells, i.e. cells were not in contact with the filter. Cultures were carried out under normoxia for 10 days, at which point media above the cell layer (supernatants) and media passing through the filter-membrane (diffusate) were then sampled and tested for VEGF and IL-8 by ELISA. Figure 6a shows that for both VEGF (~19-42Da) and IL-8 (~8-9kDa), protein concentration in the diffusate was less than that in the supernatant, when PBCs were in contact with the filter (diffusate : supernatant concentration ratio < 1). No significant difference in factor diffusion was observed between PET- and collagen-coated PET membranes, for either factor tested, indicating that the effect was purely driven by pore size. Culturing PBCs on a surface not in contact with the filter appeared to alleviate the problem of limited diffusion, at least for VEGF (diffusate : supernatant VEGF concentration ratio ≥ 1) (Fig. 18A). Macroscopically, PBCs that were cultured on PET- or coliagen-coated PET membranes appeared to form a dense layer that could be lifted off the membrane, even in the absence of clotting factors (only the buffy coat was used for this experiment) (Fig. 18B). Thus, the restricted diffusion of factors observed here was likely due to (partial) pore-blocking of the membrane by the cell plug, formed once PBCs had settled onto the scaffold support.

### Example 5

### Materials and Methods

### Analysis of the temporal profile of hypoxia-induced factor expression

The buffy coat was isolated from 10ml peripheral blood collected from a 23yr old female healthy (BMI=21), non-smoker subject and mixed with 12ml of serum-free (SF) media and 1ml blood plasma. 0.5ml of PBC suspension was added to cell culture inserts (type I collagen-coated 1µm pore membrane), before adding 5ml SF medium in each insert. Inserts were then placed in 6-well plates containing 5ml SF medium per well. Cell culture was carried under hypoxia (3% O₂) or normoxia within a 37°C/5% CO₂ incubator for 20 days. Media from wells were sampled every 4, 8, 12, 16 and 20 days and replaced by fresh media allowing culture to continue (media in inserts not changed). This setup ensured no disturbance of the cell layer during sampling. ELISAs for VEGF, IL-8, Thrombospondin-1 (all from R&D, USA) were carried out on samples obtained from well media, according to manufacturer's instructions. Four samples were tested for each factor, per time point (n=4).

### Test of VEGF release from gel carriers and anaiogenic potential of releasates by in vitro assay

1 ml of PBC/SF-medium mixture was prepared as above, mixed with 1ml SF-medium and added to type I collagen-coated 6-well plates. Carriers were added to culture inserts (1µm pore PET membrane) as follows: Gel carriers (total of 3ml) tested were Polyhexanid gel, type I collagen gel, fibrin gel, collagen(2ml)/fibrin(1ml) gel, Hydrosorb gel and Hydrosorb(2ml)/fibrin(1ml) gel. 1 ml of SF-medium was added per insert. Culture was carried under hypoxia (3% O₂) for 12 days, after which gel carriers were removed from inserts, added to 1.5ml SF-media, centrifuged and incubated overnight over a rocking platform to allow diffusion of factors from carriers to media. Media were then sampled and tested for VEGF by ELISA. Three samples were tested per carrier (n=3).
The angiogenic potential of releasates was tested in an in vitro angiogenesis assay (Ibidi, Germany), by testing the ability of releasates to induce tubule formation in endothelial cells (HUVECs) seeded on Matrigel. The ability of releasates to induce endothelial cell invasion though a Matrigel membrane was tested using the BD Fluoroblock cell culture system (BD, USA).

### Analysis of factor release kinetics from bead microcarriers embedded in sol-gel

The buffy coat was isolated from 10ml peripheral blood collected from a 23yr old female healthy (BMI=21), non-smoker subject and mixed with 12ml of serum-free (SF) media and 1ml blood plasma. 1ml of PBC/SF-medium mixture was added to type I collagen-coated 6-well plates and complemented with 1ml SF-medium. Bead microcarriers were added in excess (1.5g per insert) to culture inserts (1µm pore PET membrane). Bead microcarriers tested were polystyrene beads (125-212 µm) coated with denatured porcine-skin collagen and polystyrene beads (125-212 µm) coated with pronectin F (Sigma, Germany). 2 ml SF-medium was added per insert. Culture was carried under hypoxia (3% O₂) for 12 days, after which bead carriers were removed from inserts, added to 2ml type I collagen gel or 2ml fibrin gel. Gels containing beads were incubated overnight in 2 ml SF-media on a rocking platform overnight to allow diffusion of factors from carriers to media. Media (200µl) were then sampled at 12, 24, 36 and 48hrs and tested for VEGF by ELISA. Three samples were tested per gel/carrier combination (n=3).

The angiogenic potential of releasates was tested in an in vitro angiogenesis assay (Ibidi, Germany), by testing the ability of releasates to induce tubule formation in endothelial cells (HUVECs) seeded on Matrigel.

### Comparison of the angiogenic Potential of PBCs derived from smokers vs non-smokers

Peripheral blood (5ml) was collected into Vacutainer tubes from 19 non-smoking subjects (9 male; 38.3±6yrs, BMP=26.3±1.3, and 10 female;30.7±2.2, BMI=22.5±1) and 29 smoking subjects (11 male; 39.6±4.6yrs, BMI=27.5±1.3, and 18 female;36.9±3.2, BMI=23.6±1). All subjects provided consent as approved by the ethical committee of Dusseldorf Univerisy Hospital. Immediately after blood collection, 5ml of medium (DMEM supplemented with 20%FCS, 20%DMSO) was added to each tube so that the final blood-medium mixture (10ml) contained 10%FCS and 10%DMSO. Tubes were stored at -80°C during blood collection (4 days) and then allowed to thaw in a 37 °C waterbath for 30min. Each 10ml blood-medium solution was then mixed with 10ml serum-free medium, before being aliquoted into 4 6-well plates (5ml/well). Wells were placed in a 37 °C, 5%CO₂ incubator for 4hrs to allow blood cells to attach, then DMSO in media was further diluted by step-wise medium removal and substitution with fresh SF medium. Final medium volume per well was 4ml containing <0.5%DMSO. One set of wells was then placed in a normoxic incubator, while another into a hypoxic incubator (3% O₂), and cultured for 7 days, after which media were sampled from wells. ELISAs for VEGF, Angiogenin, and Thrombospondin-1 (all from R&D, USA) were carried out on samples obtained from well media, according to manufacturer's instructions. Two samples from each patient were tested for each condition (normoxia/hypoxia).

### Engineering PBC-derived factor protein mixtures using antibodies against specific factors

The buffy coat was isolated from 10ml peripheral blood collected from a 23yr old female healthy (BMI=21), non-smoker subject and mixed with 12ml of serum-free (SF) media and 1ml blood plasma. 1ml of PBC/SF-medium mixture was added to type I collagen-coated 6-well plates and complemented with 1ml SF-medium containing an excess concentration of antibodies or control SF medium without antibodies. Antibodies used were mouse anti-human VEGF (Invitrogen, Germany), mouse anti-human TSP-1(lnvitrogen, Germany) and rabbit anti-human PF4 (Abcam,USA). 3ml collagen gel carriers were added to culture inserts (1µm pore PET membrane) and supplemented with 1ml SF medium per insert. Culture was carried under hypoxia (3% O₂) for 4 days. After culture, collagen gels were removed from inserts and added to 1.5ml SF media and incubated overnight over a rocking platform to allow diffusion of factors from carriers to media. Media from gels incubated with antibodies or without any antibodies were analysed for VEGF, TSP-1 and PF-4 by ELISA. The angiogenic potential of releasates was tested in an in vitro angiogenesis assay (Ibidi, Germany), by testing the ability of releasates to induce tubule formation in endothelial cells (HUVECs) seeded on Matrigel. The ability of releasates to induce endothelial cell invasion though a Matrigel membrane was tested using the BD Fluoroblock cell culture system (BD, USA).

### Results

Analysis of the temporal profile of factor expression by PBCs revealed that the rate of factor expression was not constant (Fig. 19). VEGF showed a gradual down-regulation over time, under both normoxia and hypoxia (Fig. 19A). This was likely due to cellular habituation to a given oxygen tension. Quantification of cumulative VEGF levels showed that PBCs cultured under hypoxia produced about 50% more VEGF compared to PBCs cultured under normoxia, over 8 days (Fig. 19B). This difference in cumulative factor levels was reduced over time. Expression of TSP-1, an angiogenesis-inhibiting factor, was interesting in that while PBCs under hypoxia initially produced more of the factor compared to PBCs under normoxia in the first 4 days, this pattern was reversed over time (Fig. 19C). Indeed, normoxic PBCs produced at least 2-fold greater levels of TSP-1 compared to hypoxic PBCs between 4 and 8 days of culture (Fig. 19C). Thus, TSP-1 expression showed a down-regulation over time, under hypoxia. In contrast to VEGF expression, expression of IL-8 showed an upregulation over time, under both hypoxia and normoxia (Fig. 19E, F). However, there seemed to be a lag phase in the hypoxic response, which showed a plateau/small reduction in expression over the first 12 days (Fig. 19E). These findings confirm the temporal complexity of PBC angiogenic factor expression, and justify the development of a device capable of delivering factor mixtures at different time points of the conditioning phase (see prototype version for multiple injectable preparations).

Analysis of VEGF release from gel carriers, loaded with PBC-derived factors under hypoxic culture, showed that collagen gel and fibrin gel were better carriers than both hydrosorb gel and polyhexanid gel (Fig. 20). We hypothesise that the nano-porous matrix structure of collagen and fibrin gels facilitated this increased factor retention. Addition of fibrin to collagen and hydrosorb gels appeared to increase VEGF retention, though not significantly. This was likely due to direct binding of VEGF to fibrin.

Analysis of VEGF release from microbead carriers, loaded with PBC-derived factors under hypoxic culture, showed that both collagen-coated and pronectin-coated microbead carriers were able to retain PBC-produced VEGF (Fig. 21A, C). While adding beads (collagen-coated or pronectin-coated beads) to collagen gels appeared to produce an initial 12hr burst-release into media (Fig. 21A, C), adding beads to fibrin gels resulted in a more gradual release, which showed a 30% increase over time (Fig. 21B 12hrs vs.24hrs.) This effect was likely due to binding/sequestration of microbead-released VEGF by fibrin gels.

Comparison of the angiogenic potential of PBCs derived from smokers vs. non-smokers revealed that smoking did not reduce the ability of PBCs to produce angiogenesis-related factor proteins, under hypoxic stimulation. Indeed, there was no significant difference in the relative hypoxic/normoxic levels of VEGF, TSP-1 and ANG between smokers and non-smokers (Fig. 22A). Furthermore, smoking a greater number of cigarettes per day did not appear to inhibit VEGF or ANG production by PBCs. On the contrary, a weak positive correlation was observed between VEGF/ANG levels and daily cigarette consumption (Fig. 22B).

In another experiment, the composition of factors that were loaded onto the carrier was influenced by adding an excess concentration of antibodies (i.e. greater than the expected concentration of the targeted factor) against specific factors in the PBC culture media during the hypoxic conditioning phase. As shown in Figure 23, addition of factor-specific antibodies in culture media produced a reduction of the factor concentration in the carrier. This was true for all factors tested, with VEGF not being detectable in the releasates of carriers incubated with anti-VEGF antibodies, and PF4 and TSP-1 showing a 3 fold reduction compared with antibody-free control cultures (Fig. 23).

Analysis of the angiogenic potential of releasates from various gel carriers using in vitro standard endothelial cell-seeded Matrigel assay showed that while collagen gel releasates strongly induced tubule formation compared to control media, addition of fibrin to the collagen gel carriers, reduced the response (Fig. 24(a)). Also, pure fibrin gel carriers did not produce any significant tubule formation. We hypothesize that this was likely, at least partly, due to VEGF-binding by the fibrin in the carrier. Collagen and pronectin microbead carriers also induced a strong response, confirming their utility as a carrier for injectable preparations. Interestingly, we could observe direct contact between the microbeads and the formed tubules (Fig. 24(a)E and F arrows), suggesting that the role of microbeads, in addition to being a carrier one, also could involve traction-based guidance of endotheial cells towards the factor delivery site. Collagen gel carrier releasates could also strongly induce endothelial cell invasion through a Matrigel membrane compared to the weak cell invasion seen with control media (Fig. 24(b)). In contrast to their weak angiogenic response, fibrin gel carrier releasates also induced a strong invasion response. The effectiveness of fibrin here was confirmed with the increase in the weak response seen with hydrosorb gel carriers, when these were mixed with fibrin (Fig. 24(b)).

In carrier releasates where the factor composition was influenced by adding an excess concentration of antibodies against specific factors in the PBC culture media during the hypoxic conditioning phase, we observed strong tubule formation with the releasates of anti-PF4 and anti-TSP1 -incubated carriers (Fig. 24(a)). This was indeed expected, due to the dis-inhibitory effect of partial blockade of these anti-angiogenic factors (PF4 and TSP-1). Also, combination of both antibodies appeared to produce less, but thicker tubules (Fig. 24(a)L). What was perhaps more interesting was that the angiogenic response was not abolished when VEGF was blocked. This finding confirms the multi-factorial nature of the angiogenic response induced by PBC-derived hypoxic mixtures.

The above findings indicate that gel carriers loaded with hypoxia-induced PBC-derived factor mixtures can stimulate an angiogenic response, as well as direct endothelial cell growth/migration towards the site of localized delivery of the factors, i.e. the target tissue site (e.g. wound, ulcer etc).

### References

Adamo ML, Farrar RP (2006) Resistance training, and IGF involvement in the maintenance of muscle mass during the aging process. Ageing Res Rev 5:310-331
Aguirre JI, Plotkin LI, Stewart SA, Weinstein RS, Parfitt AM, Manolagas SC, Bellido T (2006) Osteocyte apoptosis is induced by weightlessness in mice and precedes osteoclast recruitment and bone loss. J Bone Miner Res 21:605-615
Al-Majed AA, Brushart TM, Gordon T (2000) Electrical stimulation accelerates and increases expression of BDNF and trkB mRNA in regenerating rat femoral motoneurons. Eur J Neurosci 12:4381-4390
Alrashdan MS, Park JC, Sung MA, Yoo SB, Jahng JW, Lee TH, Kim SJ, Lee JH (2010) Thirty minutes of low intensity electrical stimulation promotes nerve regeneration after sciatic nerve crush injury in a rat model. Acta Neurol Belg 110:168-179
Alzghoul MB, Gerrard D, Watkins BA, Hannon K (2004) Ectopic expression of IGF-I and Shh by skeletal muscle inhibits disuse-mediated skeletal muscle atrophy and bone osteopenia in vivo. FASEB J 18:221-223
Androjna C, Gatica JE, Belovich JM, Derwin KA (2008) Oxygen diffusion through natural extracellular matrices: Implications for estimating "Critical thickness" values in tendon tissue engineering. Tissue Engineering Part A 14:559-569
Ara J, Fekete S, Frank M, Golden JA, Pleasure D, Valencia I (2011) Hypoxic-preconditioning induces neuroprotection against hypoxia-ischemia in newborn piglet brain. Neurobiol Dis 43:473-485
Badylak SF, Freytes DO, Gilbert TW (2009) Extracellular matrix as a biological scaffold material: Structure and function. Acta Biomater 5:1-13
Barkefors I, Le Jan S, Jakobsson L, Hejll E, Carlson G, Johansson H, Jarvius J, Park JW, Jeon NL, Kreuger J (2008) Endothelial cell migration in stable gradients of vascular endothelial growth factor a and fibroblast growth factor 2 - Effects on chemotaxis and chemokinesis. Journal of Biological Chemistry 283:13905-13912
Bhang SH, Jeon O, Choi CY, Kwon YH, Kim BS (2007) Controlled release of nerve growth factor from fibrin gel. J Biomed Mater Res A 80:998-1002
Bian L, Zhai DY, Tous E, Rai R, Mauck RL, Burdick JA (2011) Enhanced MSC chondrogenesis following delivery of TGF-beta3 from alginate microspheres within hyaluronic acid hydrogels in vitro and in vivo. Biomaterials 32:6425-6434
Bonner JF, Blesch A, Neuhuber B, Fischer I (2010) Promoting directional axon growth from neural progenitors grafted into the injured spinal cord. J Neurosci Res 88:1182-1192
Brown RA, Wiseman M, Chuo CB, Cheema U, Nazhat SN (2005) Ultrarapid engineering of biomimetic materials and tissues: Fabrication of nano- and microstructures by plastic compression. Advanced Functional Materials 15:1762-1770
Burdick JA, Ward M, Liang E, Young MJ, Langer R (2006) Stimulation of neurite outgrowth by neurotrophins delivered from degradable hydrogels. Biomaterials 27:452-459
Burke B, Giannoudis A, Corke KP, Gill D, Wells M, Ziegler-Heitbrock L, Lewis CE (2003) Hypoxia-induced gene expression in human macrophages: implications for ischemic tissues and hypoxia-regulated gene therapy. Am J Pathol 163:1233-1243
Canalis E (2010) Update in new anabolic therapies for osteoporosis. J Clin Endocrinol Metab 95:1496-1504
Cao L, Mooney DJ (2007) Spatiotemporal control over growth factor signaling for therapeutic neovascularization. Adv Drug Deliv Rev 59:1340-1350
Carmeliet P (2000) Mechanisms of angiogenesis and arteriogenesis. Nat Med 6:389-395
Cassano M, Quattrocelli M, Crippa S, Perini I, Ronzoni F, Sampaolesi M (2009) Cellular mechanisms and local progenitor activation to regulate skeletal muscle mass. J Muscle Res Cell Motil 30:243-253
Chang H, Shyu KG, Wang BW, Kuan P (2003) Regulation of hypoxia-inducible factor-1alpha by cyclical mechanical stretch in rat vascular smooth muscle cells. Clin Sci (Lond) 105:447-456
Cheema U, Chuo CB, Sarathchandra P, Nazhat SN, Brown RA. Engineering functional collagen scaffolds: cyclical loading increases material strength and fibril aggregation. Advanced Functional Materials 17, 2426-2431. 2007. Ref Type: Journal (Full)
Cheema U, Brown R, Mudera V, Yang SY, McGrouther G, Goldspink G (2005) Mechanical signals and IGF-I gene splicing in vitro in relation to development of skeletal muscle. J Cell Physiol 202:67-75
Cheema U, Brown RA, Alp B, MacRobert AJ (2008) Spatially defined oxygen gradients and vascular endothelial growth factor expression in an engineered 3D cell model. Cellular and Molecular Life Sciences 65:177-186
Cheema U, Hadjipanayi E, Tammi N, Alp B, Mudera V, Brown RA (2009) Identification of key factors in deep 02 cell perfusion for vascular tissue engineering. Int J Artif Organs 32:318-328
Davis-Lopez de Carrizosa MA, Morado-Diaz CJ, Tena JJ, itez-Temino B, Pecero ML, Morcuende SR, de la Cruz RR, Pastor AM (2009) Complementary actions of BDNF and neurotrophin-3 on the firing patterns and synaptic composition of motoneurons. J Neurosci 29:575-587
Deng D, Liu W, Xu F, Yang Y, Zhou G, Zhang WJ, Cui L, Cao Y (2009) Engineering human neo-tendon tissue in vitro with human dermal fibroblasts under static mechanical strain. Biomaterials 30:6724-6730
Di SS, Yang Z, Wyler von BM, Voelzmann J, Diehm N, Baumgartner I, Kalka C (2009) Novel cell-free strategy for therapeutic angiogenesis: in vitro generated conditioned medium can replace progenitor cell transplantation. PLoS One 4:e5643
Dubey N, Letourneau PC, Tranquillo RT (1999) Guided neurite elongation and schwann cell invasion into magnetically aligned collagen in simulated peripheral nerve regeneration. Exp Neurol 158:338-350
Egermann M, Baltzer AW, Adamaszek S, Evans C, Robbins P, Schneider E, Lill CA (2006) Direct adenoviral transfer of bone morphogenetic protein-2 cDNA enhances fracture healing in osteoporotic sheep. Hum Gene Ther 17:507-517
Egger M, Schgoer W, Beer AG, Jeschke J, Leierer J, Theurl M, Frauscher S, Tepper OM, Niederwanger A, Ritsch A, Kearney M, Wanschitz J, Gurtner GC, Fischer-Colbrie R, Weiss G, Piza-Katzer H, Losordo DW, Patsch JR, Schratzberger P, Kirchmair R (2007) Hypoxia up-regulates the angiogenic cytokine secretoneurin via an HIF-1alpha-and basic FGF-dependent pathway in muscle cells. FASEB J 21:2906-2917
Favier FB, Benoit H, Freyssenet D (2008) Cellular and molecular events controlling skeletal muscle mass in response to altered use. Pflugers Arch 456:587-600
Fisher JP, Jo S, Mikos AG, Reddi AH (2004) Thermoreversible hydrogel scaffolds for articular cartilage engineering. J Biomed Mater Res A 71:268-274
Fortier LA, Barker JU, Strauss EJ, McCarrel TM, Cole BJ (2011) The Role of Growth Factors in Cartilage Repair. Clin Orthop Relat Res
Freeman JW, Silver FH (2005) The effects of prestrain and collagen fibril alignment on in vitro mineralization of self-assembled collagen fibers. Connect Tissue Res 46:107-115
Garrett IR (2007) Anabolic agents and the bone morphogenetic protein pathway. Curr Top Dev Biol 78:127-171
Germani A, Di CA, Mangoni A, Straino S, Giacinti C, Turrini P, Biglioli P, Capogrossi MC (2003) Vascular endothelial growth factor modulates skeletal myoblast function. Am J Pathol 163:1417-1428
Goldspink G (1999) Changes in muscle mass and phenotype and the expression of autocrine and systemic growth factors by muscle in response to stretch and overload. J Anat 194 (Pt 3):323-334
Goldspink G (2005) Mechanical signals, IGF-I gene splicing, and muscle adaptation. Physiology (Bethesda) 20:232-238
Gordon T (2009) The role of neurotrophic factors in nerve regeneration. Neurosurg Focus 26:E3
Gordon T (2010) The physiology of neural injury and regeneration: The role of neurotrophic factors. J Commun Disord 43:265-273
Graham S, Leonidou A, Lester M, Heliotis M, Mantalaris A, Tsiridis E (2009) Investigating the role of PDGF as a potential drug therapy in bone formation and fracture healing. Expert Opin Investig Drugs 18:1633-1654
Groothuis A, Duda GN, Wilson CJ, Thompson MS, Hunter MR, Simon P, Bail HJ, van Scherpenzeel KM, Kasper G (2010) Mechanical stimulation of the pro-angiogenic capacity of human fracture haematoma: involvement of VEGF mechano-regulation. Bone 47:438-444
Gurkan UA, Krueger A, Akkus O (2011) Ossifying bone marrow explant culture as a three-dimensional mechanoresponsive in vitro model of osteogenesis. Tissue Eng Part A 17:417-428
Hadjipanayi E, Ananta M, Binkowski M, Streeter I, Lu Z, Cui ZF, Brown RA, Mudera V (2010a) Mechanisms of structure generation during plastic compression of nanofibrillar collagen hydrogel scaffolds: towards engineering of collagen. J Tissue Eng Regen Med
Hadjipanayi E, Brown RA, Mudera V (2009a) interface integration of layered collagen scaffolds with defined matrix stiffness: implications for sheet-based tissue engineering. J Tissue Eng Regen Med 3:230-241
Hadjipanayi E, Brown RA, Mudera V, Deng D, Liu W, Cheema U (2010b) Controlling physiological angiogenesis by hypoxia-induced signaling. J Control Release 146:309-317
Hadjipanayi E, Cheema U, Mudera V, Deng D, Liu W, Brown RA (2011) First implantable device for hypoxia-mediated angiogenic induction. J Control Release 153:217-224
Hadjipanayi E, Mudera V, Brown RA (2009b) Close dependence of fibroblast proliferation on collagen scaffold matrix stiffness. J Tissue Eng Regen Med 3:77-84
He C, Kim SW, Lee DS (2008) In situ gelling stimuli-sensitive block copolymer hydrogels for drug delivery. J Control Release 127:189-207
Hu X, Yu SP, Fraser JL, Lu Z, Ogle ME, Wang JA, Wei L (2008) Transplantation of hypoxia-preconditioned mesenchymal stem cells improves infarcted heart function via enhanced survival of implanted cells and angiogenesis. J Thorac Cardiovasc Surg 135:799-808
Huang CY, Hagar KL, Frost LE, Sun Y, Cheung HS (2004) Effects of cyclic compressive loading on chondrogenesis of rabbit bone-marrow derived mesenchymal stem cells. Stem Cells 22:313-323
Huang J, Ye Z, Hu X, Lu L, Luo Z (2010) Electrical stimulation induces calcium-dependent release of NGF from cultured Schwann cells. Glia 58:622-631
Huang L, Zhuang X, Hu J, Lang L, Zhang P, Wang Y, Chen X, Wei Y, Jing X (2008a) Synthesis of biodegradable and electroactive multiblock polylactide and aniline pentamer copolymer for tissue engineering applications. Biomacromolecules 9:850-858
ishii I, Mizuta H, Sei A, Hirose J, Kudo S, Hiraki Y (2007) Healing of full-thickness defects of the articular cartilage in rabbits using fibroblast growth factor-2 and a fibrin sealant. J Bone Joint Surg Br 89:693-700
Issack PS, DiCesare PE (2003) Recent advances toward the clinical application of bone morphogenetic proteins in bone and cartilage repair. Am J Orthop (Belle Mead NJ) 32:429-436
Iwanuma O, Abe S, Hiroki E, Kado S, Sakiyama K, Usami A, Ide Y (2008) Effects of mechanical stretching on caspase and IGF-1 expression during the proliferation process of myoblasts. Zoolog Sci 25:242-247
Jain A, Kim YT, McKeon RJ, Bellamkonda RV (2006) In situ gelling hydrogels for conformal repair of spinal cord defects, and local delivery of BDNF after spinal cord injury. Biomaterials 27:497-504
Kido S, Kuriwaka-Kido R, Imamura T, Ito Y, Inoue D, Matsumoto T (2009) Mechanical stress induces Interleukin-11 expression to stimulate osteoblast differentiation. Bone 45:1125-1132
Klouda L, Mikos AG (2008) Thermoresponsive hydrogels in biomedical applications. Eur J Pharm Biopharm 68:34-45
Komatsu DE, Hadjiargyrou M (2004) Activation of the transcription factor HIF-1 and its target genes, VEGF, HO-1, iNOS, during fracture repair. Bone 34:680-688
Komobuchi H, Hato N, Teraoka M, Wakisaka H, Takahashi H, Gyo K, Tabata Y, Yamamoto M (2010) Basic fibroblast growth factor combined with biodegradable hydrogel promotes healing of facial nerve after compression injury: an experimental study. Acta Otolaryngol 130:173-178
Kubo M, Li TS, Kamota T, Ohshima M, Qin SL, Hamano K (2009) Increased expression of CXCR4 and integrin alphaM in hypoxia-preconditioned cells contributes to improved cell retention and angiogenic potency. J Cell Physiol 220:508-514
Kushida A, Yamato M, Konno C, Kikuchi A, Sakurai Y, Okano T (2000) Temperature-responsive culture dishes allow nonenzymatic harvest of differentiated Madin-Darby canine kidney (MDCK) cell sheets. J Biomed Mater Res 51:216-223
L'Heureux N, Paquet S, Labbe R, Germain L, Auger FA (1998) A completely biological tissue-engineered human blood vessel. Faseb Journal 12:47-56
Levy AP (1999) A cellular paradigm for the failure to increase vascular endothelial growth factor in chronically hypoxic states. Coron Artery Dis 10:427-430
Li M, Liu X, Liu X, Ge B (2010a) Calcium phosphate cement with BMP-2-loaded gelatin microspheres enhances bone healing in osteoporosis: a pilot study. Clin Orthop Relat Res 468:1978-1985
Li Z, Yao SJ, Alini M, Stoddart MJ (2010b) Chondrogenesis of human bone marrow mesenchymal stem cells in fibrin-polyurethane composites is modulated by frequency and amplitude of dynamic compression and shear stress. Tissue Eng Part A 16:575-584
Lopiz-Morales Y, Abarrategi A, Ramos V, Moreno-Vicente C, Lopez-Duran L, Lopez-Lacomba JL, Marco F (2010) In vivo comparison of the effects of rhBMP-2 and rhBMP-4 in osteochondral tissue regeneration. Eur Cell Mater 20:367-378
Luo F, Wariaro D, Lundberg G, Blegen H, Wahlberg E (2002) Vascular growth factor expression in a rat model of severe limb ischemia. J Surg Res 108:258-267
Lyras DN, Kazakos K, Verettas D, Chronopoulos E, Folaranmi S, Agrogiannis G (2010) Effect of combined administration of transforming growth factor-b1 and insulin-like growth factor I on the mechanical properties of a patellar tendon defect model in rabbits. Acta Orthop Belg 76:380-386
Malemud CJ (2004) Cytokines as therapeutic targets for osteoarthritis. BioDrugs 18:23-35
Manning CN, Kim HM, Sakiyama-Elbert S, Galatz LM, Havlioglu N, Thomopoulos S (2011) Sustained delivery of transforming growth factor beta three enhances tendon-to-bone healing in a rat model. J Orthop Res 29:1099-1105
Mierisch CM, Cohen SB, Jordan LC, Robertson PG, Balian G, Diduch DR (2002) Transforming growth factor-beta in calcium alginate beads for the treatment of articular cartilage defects in the rabbit. Arthroscopy 18:892-900
Milkiewicz M, Doyle JL, Fudalewski T, Ispanovic E, Aghasi M, Haas TL (2007) HIF-1alpha and HIF-2alpha play a central role in stretch-induced but not shear-stress-induced angiogenesis in rat skeletal muscle. J Physiol 583:753-766
Namiki A, Brogi E, Kearney M, Kim EA, Wu TG, Couffinhal T, Varticovski L, Isner JM (1995) Hypoxia induces vascular endothelial growth factor in cultured human endothelial cells. Journal of Biological Chemistry 270:31189-31195
Neidlinger-Wilke C, Stalla I, Claes L, Brand R, Hoellen I, Rubenacker S, Arand M, Kinzl L (1995) Human osteoblasts from younger normal and osteoporotic donors show differences in proliferation and TGF beta-release in response to cyclic strain. J Biomech 28:1411-1418
Nguyen MK, Lee DS (2010) Injectable biodegradable hydrogels. Macromol Biosci 10:563-579
Niu X, Nouraie M, Campbell A, Rana S, Minniti CP, Sable C, Darbari D, Dham N, Reading NS, Prchal JT, Kato GJ, Gladwin MT, Castro OL, Gordeuk VR (2009) Angiogenic and inflammatory markers of cardiopulmonary changes in children and adolescents with sickle cell disease. PLoS One 4:e7956
Okazaki KM, Maltepe E (2006) Oxygen, epigenetics and stem cell fate. Regenerative Medicine 1:71-83
Orth P, Kaul G, Cucchiarini M, Zurakowski D, Menger MD, Kohn D, Madry H (2011) Transplanted articular chondrocytes co-overexpressing IGF-I and FGF-2 stimulate cartilage repair in vivo. Knee Surg Sports Traumatol Arthrosc
Ozduman K, Ozkan A, Yildirim O, Pamir MN, Gunel M, Kilic T (2010) Temporal expression of angiogenesis-related genes in developing neonatal rodent retina: a novel in vivo model to study cerebral vascular development. Neurosurgery 66:538-543
Pajusola K, Kunnapuu J, Vuorikoski S, Soronen J, Andre H, Pereira T, Korpisalo P, Yla-Herttuala S, Poellinger L, Alitalo K (2005) Stabilized HIF-1alpha is superior to VEGF for angiogenesis in skeletal muscle via adeno-associated virus gene transfer. FASEB J 19:1365-1367
Pang C, Gao Z, Yin J, Zhang J, Jia W, Ye J (2008) Macrophage infiltration into adipose tissue may promote angiogenesis for adipose tissue remodeling in obesity. Am J Physiol Endocrinol Metab 295:E313-E322
Panutsopulos D, Zafiropoulos A, Krambovitis E, Kochiadakis GE, Igoumenidis NE, Spandidos DA (2003) Peripheral monocytes from diabetic patients with coronary artery disease display increased bFGF and VEGF mRNA expression. J Transl Med 1:6
Papachroni KK, Karatzas DN, Papavassiliou KA, Basdra EK, Papavassiliou AG (2009) Mechanotransduction in osteoblast regulation and bone disease. Trends Mol Med 15:208-216
Patel MJ, Chang KH, Sykes MC, Talish R, Rubin C, Jo H (2009) Low magnitude and high frequency mechanical loading prevents decreased bone formation responses of 2T3 preosteoblasts. J Cell Biochem 106:306-316
Petersen W, Varoga D, Zantop T, Hassenpflug J, Mentlein R, Pufe T (2004) Cyclic strain influences the expression of the vascular endothelial growth factor (VEGF) and the hypoxia inducible factor 1 alpha (HIF-1alpha) in tendon fibroblasts. J Orthop Res 22:847-853
Plotkin LI, Mathov I, Aguirre JI, Parfitt AM, Manolagas SC, Bellido T (2005) Mechanical stimulation prevents osteocyte apoptosis: requirement of integrins, Src kinases, and ERKs. Am J Physiol Cell Physiol 289:C633-C643
Potta T, Chun C, Song SC (2010) Injectable, dual cross-linkable polyphosphazene blend hydrogels. Biomaterials 31:8107-8120
Quinn LS, Anderson BG, Drivdahl RH, Alvarez B, Argiles JM (2002) Overexpression of interleukin-15 induces skeletal muscle hypertrophy in vitro: implications for treatment of muscle wasting disorders. Exp Cell Res 280:55-63
Radisic M, Malda J, Epping E, Geng W, Langer R, Vunjak-Novakovic G (2006) Oxygen gradients correlate with cell density and cell viability in engineered cardiac tissue. Biotechnol Bioeng 93:332-343
Raghunath M, Wong YS, Farooq M, Ge R (2009) Pharmacologically induced angiogenesis in transgenic zebrafish. Biochemical and Biophysical Research Communications 378:766-771
Rath B, Nam J, Knobloch TJ, Lannutti JJ, Agarwal S (2008) Compressive forces induce osteogenic gene expression in calvarial osteoblasts. J Biomech 41:1095-1103
Ray PS, Estrada-Hernandez T, Sasaki H, Zhu L, Maulik N (2000) Early effects of hypoxia/reoxygenation on VEGF, ang-1, ang-2 and their receptors in the rat myocardium: implications for myocardial angiogenesis. Mol Cell Biochem 213:145-153
Rosenstein JM, Krum JM, Ruhrberg C (2010) VEGF in the nervous system. Organogenesis 6:107-114
Rubin C, Xu G, Judex S (2001) The anabolic activity of bone tissue, suppressed by disuse, is normalized by brief exposure to extremely low-magnitude mechanical stimuli. FASEB J 15:2225-2229
Seil JT, Webster TJ (2010) Electrically active nanomaterials as improved neural tissue regeneration scaffolds. Wiley Interdiscip Rev Nanomed Nanobiotechnol 2:635-647
Semenza GL (2007) Regulation of tissue perfusion in mammals by hypoxia-inducible factor 1. Exp Physiol 92:988-991
Silva EA, Mooney DJ (2007) Spatiotemporal control of vascular endothelial growth factor delivery from injectable hydrogels enhances angiogenesis. J Thromb Haemost 5:590-598
Simic P, Culej JB, Orlic I, Grgurevic L, Draca N, Spaventi R, Vukicevic S (2006) Systemically administered bone morphogenetic protein-6 restores bone in aged ovariectomized rats by increasing bone formation and suppressing bone resorption. J Biol Chem 281:25509-25521
Skutek M, van GM, Zeichen J, Brauer N, Bosch U (2001) Cyclic mechanical stretching modulates secretion pattern of growth factors in human tendon fibroblasts. Eur J Appl Physiol 86:48-52
Smith LA, Liu X, Ma PX (2008) Tissue Engineering with Nano-Fibrous Scaffolds. Soft Matter 4:2144-2149
Tagami Y, Kurimoto T, Miyoshi T, Morimoto T, Sawai H, Mimura O (2009) Axonal regeneration induced by repetitive electrical stimulation of crushed optic nerve in adult rats. Jpn J Ophthalmol 53:257-266
Takeuchi Y, Watanabe S, Ishii G, Takeda S, Nakayama K, Fukumoto S, Kaneta Y, Inoue D, Matsumoto T, Harigaya K, Fujita T (2002) Interleukin-11 as a stimulatory factor for bone formation prevents bone loss with advancing age in mice. J Biol Chem 277:49011-49018
Taylor SJ, McDonald JW, III, Sakiyama-Elbert SE (2004) Controlled release of neurotrophin-3 from fibrin gels for spinal cord injury. J Control Release 98:281-294
Thomopoulos S, Das R, Sakiyama-Elbert S, Silva MJ, Charlton N, Gelberman RH (2010) bFGF and PDGF-BB for tendon repair: controlled release and biologic activity by tendon fibroblasts in vitro. Ann Biomed Eng 38:225-234
Tidball JG (2005) Mechanical signal transduction in skeletal muscle growth and adaptation. J Appl Physiol 98:1900-1908
Tomanek RJ, Zheng W, Yue X (2004) Growth factor activation in myocardial vascularization: therapeutic implications. Mol Cell Biochem 264:3-11
Torry RJ, Tomanek RJ, Zheng W, Miller SJ, Labarrere CA, Torry DS (2009) Hypoxia increases placenta growth factor expression in human myocardium and cultured neonatal rat cardiomyocytes. J Heart Lung Transplant 28:183-190
Uggen C, Dines J, McGarry M, Grande D, Lee T, Limpisvasti O (2010) The effect of recombinant human platelet-derived growth factor BB-coated sutures on rotator cuff healing in a sheep model. Arthroscopy 26:1456-1462
Wan L, Xia R, Ding W (2010) Short-term low-frequency electrical stimulation enhanced remyelination of injured peripheral nerves by inducing the promyelination effect of brain-derived neurotrophic factor on Schwann cell polarization. J Neurosci Res 88:2578-2587
Wang Y, Wan C, Gilbert SR, Clemens TL (2007) Oxygen sensing and osteogenesis. Ann N Y Acad Sci 1117:1-11
Wenjin W, Wenchao L, Hao Z, Feng L, Yan W, Wodong S, Xianqun F, Wenlong D (2011) Electrical stimulation promotes BDNF expression in spinal cord neurons through Ca(2+)-and Erk-dependent signaling pathways. Cell Mol Neurobiol 31:459-467
Wu G, Ju L, Jin T, Chen L, Shao L, Wang Y, Liu B (2010) Local delivery of recombinant human bone morphogenetic protein-2 increases axonal regeneration and the expression of tau protein after facial nerve injury. J Int Med Res 38:1682-1688
Yang G, Crawford RC, Wang JH (2004) Proliferation and collagen production of human patellar tendon fibroblasts in response to cyclic uniaxial stretching in serum-free conditions. J Biomech 37:1543-1550
Zdanowicz MM, Teichberg S (2003) Effects of insulin-like growth factor-1/binding protein-3 complex on muscle atrophy in rats. Exp Biol Med (Maywood ) 228:891-897

## Claims

1. An in vitro or ex vivo method of preparing a cell-free composition, said method comprising or consisting of
(a) subjecting cells to stress; and
(b) collecting factors produced by said cells when subjected to said stress, thereby obtaining said cell-free composition;
wherein
said cells are comprised in or form at least one first carrier and said collecting is effected by means of a second carrier which second carrier is cell-free and concomitantly present with and spatially distinct from the first carrier(s) and is a scaffold composed of natural or synthetic polymer or comprises inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium; and
said collecting is effected using a device comprising or consisting of
(i) said first carrier(s) which first carrier(s) comprise(s) said cells or is/are suitable to hold said cells;
(ii) said second carrier which is cell-free; and
(iii) means of subjecting said cells in said first carrier to stress;
wherein first carrier(s) and second carrier are positioned such that factors secreted by said cells when subjected to stress are collected in said second carrier,
wherein at least one filter is positioned between said first and second carrier which prevents any cells and pathogens present in (any of) said first carrier(s) from entering into said second carrier.

2. The method of claim 1, wherein the subjecting to stress is effected by exposing said cells to
(i) hypoxia;
(ii) mechanical stress; and/or
(iii) electric stimulation;
and
wherein said cells are autologous, allogeneic or xenogeneic
(iv) fibroblasts; bone-marrow derived stromal cells; skeletal, smooth or cardiac muscle cells; epithelial cells; adult stem cells; blood cells; or adipose tissue-derived stromal cells;
(v) myoblasts; osteocytes; osteoblasts; osteoclasts; chondrocytes; skeletal, smooth or cardiac muscle cells; dermal, tendon or cardiac fibroblasts; endothelial cells; or adult stem cells, or
(vi) nerve cells; glial cells; skeletal, smooth or cardiac muscle cells; fibroblasts; osteocytes; osteoblasts; osteoclasts; chondrocytes; or adult stem cells including neural stem cells;
or wherein step (a) or step (b) are repeated two or more times.

3. The method of claim 1 or 2, wherein all produced factors are collected;
or
wherein said factors are peptidic or proteinaceous factors.

4. The method of any one of claims 2 or 3, wherein step (b) is repeated two or more times, and two or more cell-free compositions are obtained.

5. The method of claim 4, wherein step (a) is performed once and step (b) is performed two or more times at defined points in time.

6. A device comprising or consisting of
(a) one or more first carrier(s) which first carrier(s) comprise(s) cells or is/are suitable to hold cells;
(b) a second carrier which is cell-free and is a scaffold composed of natural or synthetic polymer or comprises inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium; and
(c) means of subjecting said cells in said first carrier to stress;
wherein first carrier(s) and second carrier are positioned such that factors secreted by said cells when subjected to stress are collected in said second carrier;
wherein at least one filter is positioned between said first and second carrier which prevent any cells and pathogens present in (any of) said first carrier(s) from entering into said second carrier.

7. The device according to claim 6, wherein
(a) said filter is a nano-porous filter;
(b) said first carrier(s) or second carrier or at least one of said first carrier(s) or second carrier comprises at least one agent binding at least one of the factors produced by said cells when subjected to stress;
(c) in case of more than one first carrier each of said first carriers comprises or is suitable to hold different cells; or
(d) said carriers are multi-layered or planar, cylindrical or spherical; or said second carrier is a thread or suture.

8. The device according to claim 6 or 7, wherein
(a) one or both carriers comprise one or more of
(i) proteins;
(ii) polysaccharides;
(iii) glycosaminoglycans;
(iv) synthetic polymers selected from polyglycolic acid, polylactic co-glycolic acid, polylactic acid polycaprolactone, polystyrene and polyethylene terephthalate;
(v) de-cellularised native tissue; and
(vi) inorganic material selected from calcium phosphate ceramics, alumina ceramics, zirconia ceramics, nitride ceramics, carbon nanotubes and metals selected from or alloys comprising titanium, tantalum, iron, magnesium, cobalt and chromium;
(b) one or both carriers are selected from gel; sponge; mesh; microparticles; nanoparticles; microfibers; nanofibers; preferably electrospun microfibers or nanofibers; and nanotubes;
(c) said first carrier or at least one of said first carrier(s) furthermore comprises an electric conductor;
(d) said first carrier or at least one of said first carriers is/are selected from cell sheet(s), split-thickness and full-thickness skin graft(s); and/or
(e) said second carrier is selected from cream, emollient and ointment.

9. A device according to any one of claims 6 to 8, said device comprising
(a) a first chamber comprising
(i) at least one inlet;
(ii) a first carrier which is capable of adsorbing, absorbing or trapping blood cells; and
(iii) at least one filter which prevent any cells or pathogens from entering into a second chamber while permitting entry of peptidic or proteinaceous factors into said second chamber;
(b) said second chamber comprising a second carrier which second carrier is cell-free, said second carrier being selected from
(i) a nano- or microporous matrix capable of specifically binding, adsorbing, absorbing or trapping said peptidic or proteinaceous factors; and
(ii) a nano- or microfiber mesh or nano- or microparticle network which mesh or network can be rendered injectable by mechanical agitation; and
(c) in case (b)(ii), said second chamber has at least one port for a syringe, a needle and/or a third chamber connected to said second chamber.

10. The device of claim 9, wherein said first or second chamber has at least one inlet which is sealable.

11. The method of any one of claims 1 to 5, wherein said collecting is effected by using the device according to any one of claims 6 to 10.

12. The method of any one of claims 1 to 5 or 11, said method comprising the step of fragmenting at least one factor-loaded second carrier.

13. One or more cell-free compositions obtained by the method of any one of claims 1 to 5 or 11 for use in a method of treating or preventing tissue damage, wherein said composition comprises or consists of at least one second carrier as defined in claim 1.

14. The one or more cell-free composition(s) for use according to claim 13, wherein said tissue damage is related to
conditions selected from wounds, including wounds caused by radiation, steroid wounds and venous stasis wounds; ulcers, in particular decubitus ulcers and diabetic ulcers; incisions; lacerations; abrasions; burns; grafts, in particular skin grafts, local flaps and free flaps; and ischemic tissues such as post-wounding or post-grafting ischemic tissues;
or disorders selected from vascular occlusive disease; peripheral artery disease; atherosclerosis; myointimal hyperplasia; thromboangiitis obliterans; thrombotic disorders; mesenteric ischemia; limb ischemia; peripheral artery stenosis; vasculitis; infarctions including cerebral and myocardial infarctions; diabetes; traumatic injuries; bone fractures; osteoporosis; arthritis including osteoarthritis; rheumatism; cartilage rupture or detachment; torn or ruptured tendons; spinal injuries; muscular dystrophies; amyotrophic lateral sclerosis; cancer; AIDS; and central or peripheral nerve injury or degeneration.

15. The cell-free compositions for use of claim 13 or 14, wherein said compositions are (to be) topically administered at defined points in time which defined points in time match the defined points in time according to claim 5.

## Patentansprüche

1. In vitro- oder ex vivo-Verfahren zum Herstellen einer zellfreien Zusammensetzung, wobei das Verfahren die folgenden Schritte umfasst oder daraus besteht:
(a) die Zellen werden Stress unterworfen; und
(b) Faktoren, die von den Zellen hergestellt werden, wenn sie Stress unterworfen sind, werden gesammelt, wodurch die zellfreie Zusammensetzung erhalten wird;
wobei
die Zellen von mindestens einem ersten Träger umfasst sind oder diesen bilden, und das Sammeln mittels eines zweiten Trägers durchgeführt wird, wobei der zweite Träger zellfrei, gleichzeitig mit dem/den ersten Träger(n) anwesend und räumlich davon getrennt und ferner ein Gerüst ist, das aus natürlichem oder synthetischem Polymer besteht oder anorganisches Material umfasst, das ausgewählt ist aus Kalziumphosphatkeramik, Aluminiumoxidkeramik, Zirkonoxidkeramik, Nitridkeramik, Kohlenstoff-Nanoröhren und Metallen ausgewählt aus oder Legierungen umfassend Titan, Tantal, Eisen, Magnesium, Kobalt und Chrom; und
das Sammeln unter Verwendung einer Vorrichtung durchgeführt wird, die das Folgende umfasst
(i) den/die ersten Träger, wobei der/die erste(n) Träger die Zellen umfassen oder geeignet ist/sind, die Zellen zu halten;
(ii) den zweiten Träger, der zellfrei ist; und
(iii) Mittel, die Zellen im ersten Träger Stress zu unterwerfen;
oder daraus besteht
wobei erste(r) Träger und zweiter Träger so positioniert sind, dass von den Zellen ausgeschiedene Faktoren, wenn diese Stress unterworfen sind, im zweiten Träger gesammelt werden,
wobei mindestens ein Filter zwischen dem ersten und zweiten Träger positioniert ist, der Zellen und Pathogene im/in den ersten Träger(n) am Eindringen in den zweiten Träger hindert.

2. Verfahren nach Anspruch 1, wobei die Zellen
(i) Hypoxie;
(ii) mechanischem Stress; und/oder
(iii) elektrischer Stimulation
ausgesetzt und dadurch Stress unterworfen werden,
und
wobei die Zellen autologe, allogene oder xenogene
(iv) Fibroblasten; Knochenmark-abgeleitete Stroma-Zellen; Skelett-, glatte oder Herzmuskelzellen; Epithelzellen; adulte Stammzellen; Blutzellen; oder Fettgewebe-abgeleitete Stroma-Zellen;
(v) Myoblasten; Osteozyten; Osteoblasten; Osteoklasten; Chondrozyten; Skelett-, glatte oder Herzmuskelzellen; Haut-, Sehnen- oder Herz-Fibroblasten; Endothelzellen; oder adulte Stammzellen, oder
(vi) Nervenzellen; Gliazellen; Skelett-, glatte oder Herzmuskelzellen; Fibroblasten; Osteozyten; Osteoblasten; Osteoklasten; Chondrozyten; oder adulte Stammzellen einschließlich neuralen Stammzellen sind;
oder wobei Schritt (a) oder Schritt (b) zwei oder mehrmals wiederholt werden.

3. Verfahren nach Anspruch 1 oder 2, wobei alle hergestellten Faktoren gesammelt werden; oder
wobei die Faktoren peptidische oder Protein-Faktoren sind.

4. Verfahren nach Anspruch 2 oder 3, wobei der Schritt (b) zwei oder mehrmals wiederholt wird, und zwei oder mehr zellfreie Zusammensetzungen erhalten werden.

5. Verfahren nach Anspruch 4, wobei Schritt (a) einmal durchgeführt wird und Schritt (b) zwei- oder mehrmals zu definierten Zeitpunkten durchgeführt wird.

6. Vorrichtung umfassend
(a) einen oder mehrere erste Träger, der/die Zellen umfasst/umfassen oder geeignet ist/sind, Zellen zu halten;
(b) einen zweiten Träger, der zellfrei und ein Gerüst ist, das aus natürlichem oder synthetischem Polymer besteht oder anorganisches Material umfasst, das ausgewählt ist aus Kalziumphosphatkeramik, Aluminiumoxidkeramik, Zirkonoxidkeramik, Nitridkeramik, Kohlenstoff-Nanoröhren und Metallen ausgewählt aus oder Legierungen umfassend Titan, Tantal, Eisen, Magnesium, Kobalt und Chrom; und
(c) Mittel, um die Zellen im ersten Träger Stress zu unterwerfen;
oder daraus bestehend
wobei erste(r) Träger und zweiter Träger so positioniert sind, dass von den Zellen ausgeschiedene Faktoren, wenn diese Stress unterworfen sind, im zweiten Träger gesammelt werden,
wobei mindestens ein Filter zwischen dem ersten und zweiten Träger positioniert ist, der Zellen und Pathogene im/in den ersten Träger(n) am Eindringen in den zweiten Träger hindert.

7. Vorrichtung nach Anspruch 6, wobei
(a) der Filter ein nanoporöser Filter ist;
(b) der/die erste(n) Träger oder der zweite Träger oder mindestens einer der ersten oder zweiten Träger mindestens ein Agens umfasst, das mindestens einen der Faktoren bindet, der von den Zellen hergestellt wird, wenn diese Stress unterworfen werden;
(c) für den Fall, dass mehr als ein erster Träger vorliegt, jeder der ersten Träger unterschiedliche Zellen umfasst oder geeignet ist, diese zu halten; oder
(d) die Träger mehrschichtig oder planar, zylindrisch oder sphärisch sind; oder der zweite Träger ein Faden oder eine Naht ist.

8. Vorrichtung nach Anspruch 6 oder 7, wobei
(a) ein oder beide Träger eine oder mehrere der folgenden Optionen
(i) Proteine;
(ii) Polysaccharide;
(iii) Glycosaminoglykane;
(iv) synthetische Polymere ausgewählt aus Polyglykolsäure, Polymilch-Co-Glycolsäure, Polymilchsäure-Polykaprolakton, Polystyrol und Polyethylen-Terephtalat;
(v) von Zellen befreites natives Gewebe; und
(vi) anorganisches Material ausgewählt aus Kalziumphosphatkeramik, Aluminiumoxidkeramik, Zirkonoxidkeramik, Nitridkeramik, Kohlenstoff-Nanoröhren und Metallen ausgewählt aus oder Legierungen umfassend Titan, Tantal, Eisen, Magnesium, Kobalt und Chrom
umfassen;
(b) einer oder beide Träger ausgewählt sind aus Gel; Schwamm; Gewebe; Mikropartikeln; Nanopartikeln; Mikrofasern; Nanofasern; vorzugsweise elektrogesponnenen Mikrofasern oder Nanofasern; und Nanoröhren;
(c) der erste Träger oder mindestens einer der ersten Träger ferner einen elektrischen Leiter umfasst;
(d) der erste Träger oder mindestens einer der ersten Träger ausgewählt ist aus Zelllagen, Spalthaut-Transplantat und Hauttransplantat voller Dicke; und/oder
(e) der zweite Träger ausgewählt ist aus Creme, Weichmacher und Salbe.

9. Vorrichtung nach einem der Ansprüche 6 bis 8, wobei die Vorrichtung umfasst
(a) eine erste Kammer umfassend
(i) einen ersten Einlass;
(ii) einen ersten Träger, der in der Lage ist, Blutzellen zu adsorbieren, zu absorbieren oder einzufangen; und
(iii) mindestens einen Filter, der Zellen oder Pathogene am Eintreten in eine zweite Kammer hindert und gleichzeitig den Eintritt peptidischer Faktoren oder von Protein-Faktoren in die zweite Kammer zulässt;
(b) die zweite Kammer, umfassend einen zweiten Träger, wobei der zweite Träger zellfrei und ausgewählt ist aus
(i) einer nano- oder mikroporösen Matrix, die in der Lage ist, die peptidischen oder Protein-Faktoren spezifisch zu binden, zu adsorbieren, zu absorbieren oder einzufangen; und
(ii) einem Nano- oder Mikrofasergewebe oder einem Nano- oder Mikropartikelnetzwerk, wobei das Gewebe oder Netzwerk durch mechanische Bewegung injizierbar gemacht werden kann; und
(c) im Fall (b)(ii), die zweite Kammer mindestens einen Eingang für eine Spritze, eine Nadel und/oder eine dritte Kammer, die mit der zweiten Kammer verbunden ist hat.

10. Vorrichtung nach Anspruch 9, wobei erste oder zweite Kammer mindestens einen Einlass hat, der verschließbar ist.

11. Verfahren nach einem der Ansprüche 1 bis 5, wobei das Sammeln unter Verwendung der Vorrichtung nach einem der Ansprüche 6 bis 10 durchgeführt wird.

12. Verfahren nach einem der Ansprüche 1 bis 5 oder 11, wobei das Verfahren den Schritt des Fragmentierens mindestens eines Faktor-beladenen zweiten Trägers umfasst.

13. Eine oder mehrere zellfreie Zusammensetzungen, erhalten mit dem Verfahren nach einem der Ansprüche 1 bis 5 oder 11, für die Verwendung in einem Verfahren zur Behandlung oder Vorbeugung von Gewebeschäden, wobei die Zusammensetzung mindestens einen zweiten Träger wie in Anspruch 1 definiert umfasst oder daraus besteht.

14. Eine oder mehrere zellfreie Zusammensetzungen für die Verwendung nach Anspruch 13, wobei der Gewebeschaden in Zusammenhang steht mit Zuständen ausgewählt aus Wunden, einschließlich durch Strahlung verursachter Wunden, Steroidwunden und venösen Stauwunden; Geschwüren, insbesondere Dekubitusgeschwüren und diabetischen Geschwüren; Schnitten; Platzwunden, Schürfwunden; Verbrennungen; Transplantaten, insbesondere Hauttransplantaten, lokalen Lappen und freien Lappen; und ischämischen Geweben, wie nach Wunden oder nach Transplantationen vorliegenden ischämischen Geweben; oder Störungen ausgewählt aus Gefäßverschlusskrankheit, peripherer Arterienerkrankung, Atherosklerose; myointimaler Hyperplasie; Thromboangütis obliterans; thrombotischen Störungen; mesenterialer Ischämie; Ischämie eines Körpergliedes; periphere Arterienstenose, Gefäßentzündung; Infarkten einschließlich Hirn- und Myokardinfarkt; Diabetes; traumatischen Verletzungen; Knochenbrüchen; Osteoporose; Arthritis einschließlich Osteoarthritis; Rheumatismus; Knorpelriss oder -ablösung; gezerrten oder gerissenen Sehnen; spinalen Verletzungen; muskulären Dystrophien; amyotropher Lateralsklerose; Krebs; AIDS; und zentraler oder peripherer Nervenverletzung oder -degeneration.

15. Zellfreie Zusammensetzungen für die Verwendung nach Anspruch 13 oder 14, wobei die Zusammensetzungen topisch zu verabreichen sind und zwar zu definierten Zeitpunkten, die den definierten Zeitpunkten gemäß Anspruch 5 entsprechen.

## Revendications

1. Méthode de préparation *in vitro* ou *ex vivo* d'une composition exempte de cellules, ladite méthode comprenant ou consistant à
(a) soumettre les cellules à un stress; et
(b) collecter les facteurs produits par lesdites cellules quand elles sont soumises audit stress, ce qui permet d'obtenir ladite composition exempte de cellules;
dans laquelle
lesdites cellules sont incluses dans ou bien forment au moins un premier support et ladite collecte est effectuée au moyen d'un second support, ledit second support est exempt de cellules et présent concomitamment avec et spatialement distinct le(s) premier(s) support(s), et est une structure support composée de polymère naturel ou synthétique ou comprend une matière inorganique choisie parmi les céramiques de phosphate de calcium, les céramiques d'alumine, les céramiques de zircone, les céramiques de nitrure, les nanotubes de carbone et les métaux sélectionnés parmi ou les alliages comprenant du titane, du tantale, du fer, du magnésium, du cobalt et du chrome; et
ladite collecte est effectuée en utilisant un dispositif comprenant ou constitué
(i) dudit ou desdits premiers supports, ledit ou lesdits premiers supports comprenant lesdites cellules ou étant appropriés à supporter lesdites cellules;
(ii) dudit second support qui est exempt de cellules; et
(iii) de moyens pour soumettre lesdites cellules dans ledit premier support à un stress;
dans laquelle le ou les premiers supports et le second support sont placés de sorte que les facteurs sécrétés par lesdites cellules quand elles sont soumises au stress soient collectés dans ledit second support,
dans laquelle au moins un filtre est placé entre ledit premier et ledit second support, ce qui évite à toute cellule et tout pathogène présent dans (l'un quelconque) desdits premiers supports d'entrer dans ledit ou lesdits seconds supports.

2. Méthode selon la revendication 1, dans laquelle la soumission au stress est effectuée en exposant lesdites cellules à
(i) une hypoxie;
(ii) une contrainte mécanique; et/ou
(iv) une stimulation électrique;
et
dans laquelle lesdites cellules sont des
(iv) fibroblastes; cellules de stroma de moelle osseuse; cellules du muscle squelettique, lisse ou cardiaque; cellules épithéliales; cellules souches adultes; cellules sanguines; ou cellules de stroma de tissu adipeux;
(v) myoblastes; ostéocytes; ostéoblastes; ostéoclastes; chondrocytes; cellules du muscle squelettique, lisse ou cardiaque; fibroblastes dermiques, ligamentaires ou cardiaques ; cellules endothéliales; ou cellules souches adultes, ou
(vi) cellules nerveuses; cellules gliales; cellules du muscle squelettique, lisse ou cardiaque; fibroblastes; ostéocytes; ostéoblastes; ostéoclastes; chondrocytes; ou cellules souches adultes y compris cellules souches neuronales,
autologues, allogènes ou xénogènes;
ou dans laquelle l'étape (a) ou l'étape (b) est répétée deux fois ou plus.

3. Méthode selon la revendication 1 ou 2, dans laquelle tous les facteurs produits sont collectés; ou
dans laquelle lesdits facteurs sont des facteurs peptidiques ou protéiniques.

4. Méthode selon l'une quelconque des revendications 2 ou 3, dans laquelle l'étape (b) est répétée deux fois ou plus, et deux compositions exemptes de cellules ou plus sont obtenues.

5. Méthode selon la revendication 4, dans laquelle l'étape (a) est réalisée une fois et l'étape (b) est réalisée deux fois ou plus à des moments définis.

6. Dispositif comprenant ou consistant en
(a) un ou plusieurs premiers supports, ledit ou lesdits premiers supports comprenant des cellules ou étant appropriés à supporter des cellules;
(b) un second support qui est exempt de cellules et est une structure support composée de polymère naturel ou synthétique ou comprend une matière inorganique choisie parmi les céramiques de phosphate de calcium, les céramiques d'alumine, les céramiques de zircone, les céramiques de nitrure, les nanotubes de carbone et les métaux sélectionnés parmi ou les alliages comprenant du titane, du tantale, du fer, du magnésium, du cobalt et du chrome; et
(c) de moyens pour soumettre lesdites cellules dans ledit premier support à un stress;
dans lequel le ou les premiers supports et le second support sont placés de sorte que les facteurs sécrétés par lesdites cellules quand elles sont soumises au stress soient collectés dans ledit second support,
dans lequel au moins un filtre est placé entre ledit premier et ledit second support, ce qui évite à toute cellule et tout pathogène présent dans (l'un quelconque) desdits premiers supports d'entrer dans ledit second support.

7. Dispositif selon la revendication 6, dans lequel
(a) ledit filtre est un filtre nanoporeux;
(b) ledit ou lesdits premiers supports ou seconds supports ou au moins l'un desdits premiers supports ou seconds supports comprennent au moins un agent liant au moins un des facteurs produits par lesdites cellules quand elles sont soumises à un stress;
(c) s'il y a plus d'un premier support, chacun desdits premiers supports comprend ou est approprié à supporter les différentes cellules; ou
(d) lesdits supports sont multicouches ou planes, cylindriques ou sphériques; ou ledit second support est un fil ou une suture.

8. Dispositif selon la revendication 6 ou 7, dans lequel
(a) un support ou les deux supports comprennent un ou plusieurs éléments parmi:
(i) des protéines;
(ii) des polysaccharides;
(iii) des glycosaminoglycanes;
(iv) des polymères synthétiques choisis parmi l'acide polyglycolique, l'acide polylactique co-glycolique, la polycaprolactone - acide polylactique, le téréphtalate de polystyrène et de polyéthylène;
(v) du tissu natif décellularisé; et
(vi) une matière inorganique choisie parmi les céramiques de phosphate de calcium, les céramiques d'alumine, les céramiques de zircone, les céramiques de nitrure, les nanotubes de carbone et les métaux sélectionnés parmi ou les alliages comprenant du titane, du tantale, du fer, du magnésium, du cobalt et du chrome;
(b) un ou les deux supports sont choisis parmi un gel, une éponge, une grille, des microparticules, des nanoparticules, des microfibres, des nanofibres, de préférence des microfibres ou des nanofibres électrofilées, et des nanotubes;
(c) ledit premier support ou au moins un desdits premiers supports comprend en outre un conducteur électrique;
(d) ledit premier support ou au moins un desdits premiers supports est choisi parmi le ou les tapis cellulaires, un ou des greffons de peau d'épaisseur pleine et d'épaisseur divisée; et/ou
(e) ledit second support est choisi parmi une crème, un émollient et une pommade.

9. Dispositif selon l'une quelconque des revendications 6 à 8, ledit dispositif comprenant
(a) une première chambre comprenant
(i) au moins un orifice d'entrée;
(ii) un premier support qui est capable d'adsorber, d'absorber ou de piéger les cellules sanguines; et
(iii) au moins un filtre qui interdit à toute cellule ou tout pathogène d'entrer dans la seconde chambre tout en permettant l'entrée des facteurs peptidiques ou protéiniques dans ladite seconde chambre;
(b) ladite seconde chambre comprenant un second support, ledit second support étant exempt de cellules, ledit second support étant choisi parmi
(i) une matrice nanoporeuse ou microporeuse apte à lier spécifiquement, adsorber ou piéger lesdits facteurs peptidiques ou protéiniques; et
(ii) un maillage de nanofibres ou microfibres ou un réseau de nanoparticules ou microparticules, ledit maillage ou réseau pouvant devenir injectable par agitation mécanique; et
(c) dans le cas du (b)(ii), ladite seconde chambre possède au moins un port pour une seringue, une aiguille et/ou une troisième chambre reliée à ladite seconde chambre.

10. Dispositif selon la revendication 9, dans lequel ladite première ou ladite seconde chambre a au moins un orifice d'entrée qui peut être scellé.

11. Méthode selon l'une quelconque des revendications 1 à 5, dans laquelle ladite collecte est effectuée en utilisant le dispositif selon l'une quelconque des revendications 6 à 10.

12. Méthode selon l'une quelconque des revendications 1 à 5 ou 11, ladite méthode comprenant l'étape de fragmentation d'au moins un second support chargé en facteurs.

13. Composition(s) exempte(s) de cellules, obtenue(s) par la méthode selon l'une quelconque des revendications 1 à 5 ou 11 pour l'utilisation dans une méthode de traitement ou de prévention de la lésion des tissus, où ladite composition comprend ou est constitué d'au moins un second support tel que défini selon la revendication 1.

14. Composition(s) exempte(s) de cellules pour l'utilisation selon la revendication 13, dans laquelle ladite lésion des tissus est associée à:
des conditions choisies parmi les plaies, y compris les plaies provoquées par le rayonnement, les plaies dues à des stéroïdes et les plaies dues à la stase veineuse; les ulcères, en particulier les ulcères du décubitus et les ulcères diabétiques; les incisions; les lacérations; les abrasions; les brûlures; les greffes, en particulier les greffes de peau, les lambeaux locaux et les lambeaux libres; et les tissus ischémiques comme les tissus ischémiques après une plaie ou après une greffe;
ou des troubles choisis parmi la vasculopathie oblitérante; l'artériopathie périphérique; l'athérosclérose; l'hyperplasie myo-intimale; la thromboangéite oblitérante; les thromboses; l'ischémie mésentérique; l'ischémie des membres; l'artériosténose périphérique; la vascularite; les infarctus y compris les infarctus cérébraux et myocardiaques; le diabète; les lésions traumatiques; les fractures osseuses; l'ostéoporose; l'arthrite y compris l'ostéoarthrite; les rhumatismes; la rupture ou le détachement de cartilage; l'entorse ou la rupture des ligaments; les lésions médullaires; les dystrophies musculaires; la sclérose latérale amyotrophique; le cancer; le SIDA; et la lésion ou la dégénérescence des nerfs centraux ou périphériques.

15. Compositions exemptes de cellules pour l'utilisation selon la revendication 13 ou 14, lesdites compositions étant (devant être) administrées topiquement à des moments définis, lesdits moments définis correspondant aux moments définis selon la revendication 5.
